(19)

**Europäisches Patentamt**
**European Patent Office**
**Office européen des brevets**

(11) **EP 4 065 023 B1**

(12) **EUROPEAN PATENT SPECIFICATION**

(45) Date of publication and mention
of the grant of the patent:
**17.12.2025 Bulletin 2025/51**

(21) Application number: **20828173.3**

(22) Date of filing: **20.11.2020**

(51) International Patent Classification (IPC):
*A61B 34/10* (2016.01)    *A61F 2/30* (2006.01)
*A61F 2/40* (2006.01)    *G06T 7/00* (2017.01)
*G06T 7/149* (2017.01)    *G16H 30/40* (2018.01)
*G16H 50/50* (2018.01)    *A61B 34/00* (2016.01)
*A61B 90/00* (2016.01)

(52) Cooperative Patent Classification (CPC):
**A61B 34/10; A61F 2/30942; A61F 2/4014;
G06T 7/0012; G06T 7/149; G16H 30/40;
G16H 50/50;** A61B 34/25; A61B 2034/105;
A61B 2034/108; A61B 2090/365; A61F 2002/30943;
A61F 2002/30948; G06T 2207/10072;
G06T 2207/30008;                    (Cont.)

(86) International application number:
**PCT/US2020/061608**

(87) International publication number:
**WO 2021/108270 (03.06.2021 Gazette 2021/22)**

(54) **PRE-OPERATIVE PLANNING AND INTRA OPERATIVE GUIDANCE FOR ORTHOPEDIC
SURGICAL PROCEDURES IN CASES OF BONE FRAGMENTATION**

PRÄOPERATIVE PLANUNG UND INTRAOPERATIVE FÜHRUNG FÜR ORTHOPÄDISCHE
CHIRURGISCHE EINGRIFFE BEI KNOCHENFRAGMENTIERUNG

PLANIFICATION PRÉOPÉRATOIRE ET GUIDAGE PEROPÉRATOIRE POUR DES PROCÉDURES
CHIRURGICALES ORTHOPÉDIQUES DANS DES CAS DE FRAGMENTATION OSSEUSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.11.2019 US 201962940810 P
08.07.2020 US 202063049497 P**

(43) Date of publication of application:
**05.10.2022 Bulletin 2022/40**

(73) Proprietor: **Howmedica Osteonics Corp.
Mahwah, NJ 07430 (US)**

(72) Inventors:
• **CHAOUI, Jean
Plouzané, 29280 (FR)**
• **POLTARETSKYI, Sergii
Plouzané, 29280 (FR)**

(74) Representative: **Zimmermann & Partner
Patentanwälte mbB
Postfach 330 920
80069 München (DE)**

(56) References cited:
**EP-A1- 3 421 001        WO-A1-2014/145267
WO-A1-2019/047099    US-A1- 2016 157 751**

• **JIMÉNEZ-DELGADO JUAN J ET AL: "Computer
assisted preoperative planning of bone fracture
reduction: Simulation techniques and new
trends", MEDICAL IMAGE ANALYSIS, vol. 30, 13
January 2016 (2016-01-13), pages 30 - 45,
XP029458416, ISSN: 1361-8415, DOI: 10.1016/
J.MEDIA.2015.12.005**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

(52) Cooperative Patent Classification (CPC): (Cont.)
    Y02A 90/10

**Description**

This Application claims the benefit of:

U.S. Provisional Patent Application 62/9-40,810, filed 26 November 2019, and
U.S. Provisional Patent Application 63/049,497, filed 8 July 2020.

**BACKGROUND**

Surgical joint repair procedures involve repair and/or replacement of a damaged or diseased joint. A surgical joint repair procedure, such as joint arthroplasty as an example, may involve replacing the damaged joint with a prosthetic that is implanted into the patient's bone. Proper selection or design of a prosthetic that is appropriately sized and shaped and proper positioning of that prosthetic are important to ensure an optimal surgical outcome. A surgeon may analyze damaged bone to assist with prosthetic selection, design and/or positioning, as well as surgical steps to prepare bone or tissue to receive or interact with a prosthetic.

International Patent Application WO 2014/145267 Al discloses patient-adapted articular repair systems, including implants, instruments, and surgical plans, and methods of making and using such systems. In particular, methods of selecting and/or designing patient-adapted surgical repair systems using parameterized models and/or multibody simulations are disclosed.

**SUMMARY**

For fractured joints, fracture patterns can have wide variations and be difficult to classify and analyze. This disclosure describes devices, systems, and methods for preoperative planning and intra operative guidance for orthopedic surgical procedures for fractured joints that suffer from bone fragmentation.

The present invention is defined by the features of the independent claims. Preferred embodiments are given in the dependent claims.

**BRIEF DESCRIPTION OF DRAWINGS**

FIG. 1 is a block diagram illustrating an example computing device that may be used to implement the techniques of this disclosure.

FIG. 2 shows an example workflow in accordance with the techniques of this disclosure.

FIGS. 3A-3C show Hessian eigenvector decomposition for tubular-, blob- and sheet-like structures.

FIGS. 4A-4D show examples of different locations of a displaced broken humeral head.

FIGS. 5A-5D show an example of the automatic detection of a broken humeral head and the associated segmentation zone for the examples of FIGS. 4A-4D.

FIGS. 6A-6F show examples of identified broken fragment regions of interest.

FIGS. 7A-7C show examples of a detected humeral head.

FIGS. 8A-8C show an example of cleaned humeral head points.

FIGS. 9A-9C show an example for computing start and end limit points.

FIGS. 10A-10D shows an example of diaphysis points computing.

FIG. 11 illustrates detection of the most medial and lateral elbow points.

FIGS. 12A and 12B show elbow landmarks.

FIGS. 13A-13C illustrate trochlea and capitulum landmark detection.

FIGS. 14A and 14B show an example of humerus bicipital groove detection.

FIGS. 15A-15D illustrate an example of humerus landmark detection.

FIGS. 16A and 16B show examples of upper humerus zones for correspondence establishment.

FIGS. 17A-17D show examples of landmarks for rigid and non-rigid registrations

FIGS. 18A-18H shows different variations for the mean shape of a humerus.

FIGS. 19A and 19B show the humeral length impact on the retroversion and inclination variations.

FIGS. 20A-20C illustrate different processes that cause broken fragment fusion.

FIGS. 21A-21F illustrate a step segmentation algorithm applied to a broken proximal humerus.

FIGS. 22A and 22B show 3D images of a proximal humerus fracture with vertices that belong to fracture lines.

FIGS. 23A and 23B show a 2D contour for most significant vertices detection performed using a discrete curve evolution (DCE) algorithm.

FIGS. 24A and 24B shows two types of contour topology.

FIGS. 25A and 25B illustrate steps associated with a segmentation algorithm for step segmentation.

FIGS. 26A-26D show a process for diaphysis detection and characterization.

FIGS. 27A-27H represent examples of initial alignment of a broken diaphysis with a humerus statistical shape model (SSM).

FIGS. 28A-28C show examples of broken humerus shape estimation based on diaphysis shape.

FIG. 29 is a flowchart illustrating an example method of operation of a computing device in accordance with one or more example techniques described in this disclosure.

FIGS. 30A-30D show examples of 3D meshes, which corresponds to a portion of a diaphysis.

FIGS. 31A-31C show examples of 3D meshes for a predicted humerus with a sphere identifying a region for the humeral head.

FIGS. 32A-32C show an allowed region of a humeral head for placing humeral head fragments.

FIGS. 33A and 33B show examples of translation and rotation of fragments.

FIGS. 34A and 34 are flowcharts illustrating an example method of operation of a computing device in accordance with one or more example techniques described in this disclosure.

FIG. 35 is a flowchart illustrating an example method of operation of a computing device in accordance with one or more example techniques described in this disclosure.

## DETAILED DESCRIPTION

A patient may suffer from a disease (e.g., aliment) or injury that causes damage to the patient anatomy. For shoulders, as an example of patient disease or injury, a patient may suffer from primary glenoid humeral osteoarthritis (PGHOA), rotator cuff tear arthropathy (RCTA), instability, massive rotator cuff tear (MRCT), rheumatoid arthritis (RA), post-traumatic arthritis (PTA), osteoarthritis (OA), or acute fracture, as a few examples. According to some estimates, humerus fractures are the fourth most common type of fracture in the world (after hip, spine and radius fractures) occurring in osteoporotic bone, accounting for approximately 8% of all fractures in the world.

Proximal humerus fracture patterns can have wide variations and be difficult to classify. In order to help a surgeon choose a treatment for a particular patient, several classification systems have been proposed. In 1970, Charles Neer described a four-segment classification system as he believed that existing systems did not differentiate between injuries of varied severity and did not group like fractures. The classification systems at that time were based on the mechanism of injury or level of the fracture line but did not consider many surgically important aspects or pathologic features of injury such as tuberosity displacement. Neer's classification system was based on an observation, made much earlier by Codman, that all proximal humerus fractures were composed of four major segments: the lesser tuberosity, greater tuberosity, articular surface, and humeral shaft. The four segment classification system defines proximal humerus fractures by the number of displaced segments or parts, with additional categories for articular fractures and dislocations. The potential segments involved are the greater tuberosity, lesser tuberosity, articular surface, and humeral diaphysis. A segment is defined as displaced if there is greater than 1 cm separation or 45-degree angulation. Separate categories were added for dislocations because dislocations represent more severe injuries and are more likely to have avascular necrosis and heterotopic ossification develop.

AO classification is, along with the Neer classification, a frequently used techniques for classifying fractures. The AO classification divides proximal humeral fractures into three groups, A, B and C, each with subgroups, and places more emphasis on the blood supply to the articular surface. The assumption is that if either the lesser or greater tuberosity remains attached to the articular segment, then blood supply is probably adequate to avoid avascular necrosis. The risk of avascular necrosis increases from type A (very low) to type C (high risk) and thus determines the treatment.

Due to the combination of complex anatomy and complex classification system, both of the described systems suffer from poor inter- and intra-observer reproducibility. Despite this known drawback, both of these systems are currently used for proximal humerus fracture surgery planning. It has been suggested that some of the observed variability may be attributable to difficulty interpreting the patterns of complex three-dimensional (3-D) fractures on two-dimensional plain radiographs.

To address the disease or injury, a surgeon may perform a surgical procedure such as Reversed Arthroplasty (RA), Augmented Reverse Arthroplasty (RA), Standard Total Shoulder Arthroplasty (TA), Augmented Total Shoulder Arthroplasty (TA), or Hemispherical shoulder surgery, as a few examples. There may be benefits for the surgeon to determine, prior to the surgery, characteristics (e.g., size, shape, and/or location) of the patient anatomy. For instance, determining the characteristics of the patient anatomy may aid in prosthetic selection, design and/or positioning, as well as planning of surgical steps to prepare a surface of the damaged bone to receive or interact with a prosthetic. With advance planning, the surgeon can determine, prior to surgery, rather than during surgery, steps to prepare bone or tissue, tools that will be needed, sizes and shapes of the tools, the sizes and shapes or other characteristics of one or more protheses that will be implanted and the like.

Pre-morbid characterization refers to characterizing the patient anatomy as that anatomy existed prior to the patient suffering disease or injury. However, pre-morbid characterization of the anatomy is generally not available because the patient may not consult with a doctor or surgeon until after suffering the disease or injury.

Pre-morbid anatomy, also called native anatomy, refers to the anatomy prior to the onset of a disease or the occurrence of an injury. Even after disease or injury, there may be portions of the anatomy that are healthy and portions of the anatomy that are not healthy (e.g., diseased or damaged). The diseased or damaged portions of the anatomy are referred to as pathological anatomy, and the healthy portions of the anatomy are referred to as non-pathological anatomy.

As will be described in more detail below, according to techniques of this disclosure, a computing device may be configured to receive image data (e.g., CT-Scans) of an injured joint of a patient. The computing device may also receive other information such as a patient gender or age. Based on this data, the computing device may be configured to detect and classify a fracture pattern of the injured joint. Based on this classification, the computing device may be configured to recommend a treatment procedure (e.g., no operation, minimally invasive surgery (such as a nail), or installation of an implant). In cases where an implant installation is chosen by a surgeon, the computing device may also be configured to propose an implant type, size, orientation, positioning, etc. The computing device may also be configured to provide guidance, such as visual guidance, to a surgeon as to how to move broken fragments in order to perform a desirable reduction.

This disclosure also describes techniques for configuring a computing device to register a fracture together in image data. That is, the computing device may be configured to perform a fully or partially automatic process to manipulate the fragments to restore the proximal humerus shape, and thus provide guidance to a surgeon on how to manipulate the fragments during a surgery. As will be explained in more detail below, according to techniques of this disclosure, a computing device may be configured to locate, in the image data, a diaphysis of a fractured humerus and determine an estimated shape for the humerus, prior to the fracture, based on the image of the diaphysis. In a fracture event, it is possible that all fragments of a humerus, including the diaphysis, have moved relative to where those pieces would be located in a non-fractured humerus. Thus, because the diaphysis may have moved as a result of the fracture, the diaphysis may not be usable as a reference point for determining locations of other fragments of the humerus. According to techniques of this disclosure, the computing device may use a portion of the scapula, such as the glenoid, as a reference for determining the location of a reassembled humerus after fracture. In some implementations, the computing device may first determine a location for the diaphysis based on the location of the glenoid. Other fragments of the fractured humerus may then be moved using the diaphysis as a reference location. Typically, the glenoid does not move in a fracture event, making the glenoid usable as a reference location for positioning the humerus.

Image data, in this context, includes both display images and raw image data associated with anatomy scans. In this context, raw image data refers to image data that is used to determine display images. Raw image data may have a different resolution, bit-depth, or dynamic range than a display image and may additionally be in a different color space or color model. In general, raw image data includes data that is captured as part of the imaging, including data used by a display or visualization device to display the images and in some examples, even data that is not directly used by a display or visualization device to display images. For example, raw image data, in the context of this disclosure, may include data not traditionally even thought of as being image data. As one example, in CT image data, pixels are associated with a relative radiodensity value corresponding to a mean attenuation, as measured in Hounsfield units (HUs) using the Hounsfield scale. These HU values are an example of raw image data. A display device converts the HU values into gray scale for display. Unless otherwise stated, it should be assumed that techniques of this disclosure that are described as being performed on image data can be performed using either display images or raw images.

Additionally, it should be understood that image data in this disclosure refers to both 2D and 3D image data. For simplicity, certain techniques will be described as being performed on pixels, which represent a location within a 2D model, but unless stated otherwise, it should be assumed those techniques can also be performed on voxels, which represent a location within a 3D model. Similarly, unless stated otherwise, it should be assumed that techniques described as being performed on voxels may also be performed on pixel s.

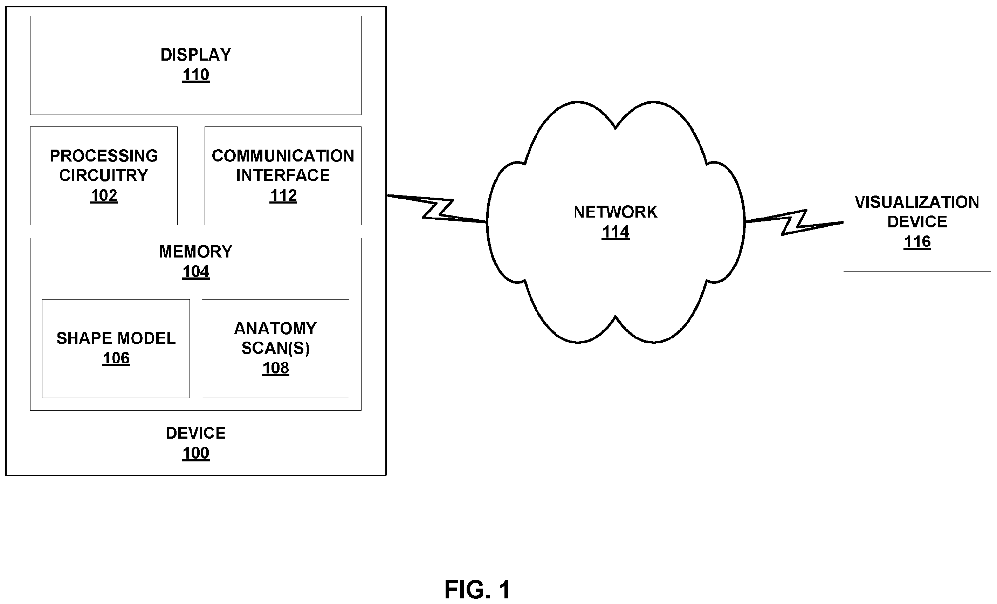

FIG. 1 is a block diagram illustrating an example computing device that may be used to implement the techniques of this disclosure. FIG. 1 illustrates device 100, which is an example of a computing device configured to perform one or more example techniques described in this disclosure.

Device 100 may include various types of computing devices, such as server computers, personal computers, smartphones, laptop computers, and other types of computing devices. Device 100 includes processing circuitry 102, memory 104, and display 110. Display 110 is optional, such as in examples where device 100 is a server computer.

Examples of processing circuitry 102 include one or more microprocessors, digital signal processors (DSPs), application specific integrated circuits (ASICs), field programmable gate arrays (FPGAs), discrete logic, software, hardware, firmware or any combinations thereof. In general, processing circuitry 102 may be implemented as fixed-function circuits, programmable circuits, or a combination thereof. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. In some examples, the one or more of the units may be distinct circuit blocks (fixed-function or programmable), and in some examples, the one or more units may be integrated circuits.

Processing circuitry 102 may include arithmetic logic units (ALUs), elementary function units (EFUs), digital circuits, analog circuits, and/or programmable cores, formed from programmable circuits. In examples where the operations of processing circuitry 102 are performed using software executed by the programmable circuits, memory 104 may store the object code of the software that processing circuitry 102 receives and executes, or another memory within processing circuitry 102 (not shown) may store such instructions. Examples of the software include software designed for surgical planning.

Memory 104 may be formed by any of a variety of memory devices, such as dynamic random access memory (DRAM), including synchronous DRAM (SDRAM), magnetoresistive RAM (MRAM), resistive RAM (RRAM), or other types of memory devices. Examples of display 110 include a liquid crystal display (LCD), a plasma display, an organic light emitting diode (OLED) display, or another type of display device.

Device 100 may include communication interface 112 that allows device 100 to output data and instructions to and receive data and instructions from visualization device 116 via network 114. For example, after determining a pre-morbid characteristic of the anatomical object, using techniques described in this disclosure, communication interface 112 may output information of the pre-morbid characteristic to visualization device 116 via network 114. A surgeon may then view a graphical representation of the pre-morbid anatomical object with visualization device 116 (e.g., possibly with the pre-morbid anatomical object overlaid on top of image of the injured or diseased anatomical object).

Communication interface 112 may be hardware circuitry that enables device 100 to communicate (e.g., wirelessly or using wires) to other computing systems and devices, such as visualization device 116. Network 114 may include various types of communication networks including one or more wide-area networks, such as the Internet, local area networks, and so on. In some examples, network 114 may include wired and/or wireless communication links.

Visualization device 116 may utilize various visualization techniques to display image content to a surgeon. Visualization device 116 may be a mixed reality (MR) visualization device, virtual reality (VR) visualization device, holographic projector, or other device for presenting extended reality (XR) visualizations. In some examples, visualization device 116 may be a Microsoft HOLOLENS™ headset, available from Microsoft Corporation, of Redmond, Washington, USA, or a similar device, such as, for example, a similar MR visualization device that includes waveguides. The HOLOLENS™ device can be used to present 3D virtual objects via holographic lenses, or waveguides, while permitting a user to view actual objects in a real-world scene, i.e., in a real-world environment, through the holographic lenses.

Visualization device 1116 may utilize visualization tools that are available to utilize patient image data to generate three-dimensional models of bone contours to facilitate preoperative planning for joint repairs and replacements. These tools allow surgeons to design and/or select surgical guides and implant components that closely match the patient's anatomy. These tools can improve surgical outcomes by customizing a surgical plan for each patient. An example of such a visualization tool for shoulder repairs is the BLUEPRINT™ system available from Wright Medical Technology, Inc. The BLUEPRINT™ system provides the surgeon with two-dimensional planar views of the bone repair region as well as a three-dimensional virtual model of the repair region. The surgeon can use the BLUEPRINT™ system to select, design or modify appropriate implant components, determine how best to position and orient the implant components and how to shape the surface of the bone to receive the components, and design, select or modify surgical guide tool(s) or instruments to carry out the surgical plan. The information generated by the BLUEPRINT™ system is compiled in a preoperative surgical plan for the patient that is stored in a database at an appropriate location (e.g., on a server in a wide area network, a local area network, or a global network) where the plan can be accessed by the surgeon or other care provider, including before and during the actual surgery.

As illustrated, memory 104 stores data representative of a shape model 106 and data representative of anatomy scans 108. Anatomy scans 108 are examples of computed tomography (CT) scans of a patient, e.g., as represented by CT scan image data.

A CT scan combines series of X-ray images taken from different angles and uses computer processing to create cross-sectional images, or slices, of the bones, blood vessels, and soft tissues inside a body. A CT Scan has many uses, but is particularly well-suited to quickly examine people who may have internal injuries, such as fractured bones, from car accidents or other types of trauma. A CT-Scan can be used to visualize nearly all parts of the body and is used to diagnose disease or injury as well as to plan medical, surgical or radiation treatment.

An output of a CT-Scan is a volumetric image composed of elementary cubes (i.e. voxels), where each voxel is associated with a numerical value that represents an X-Ray attenuation measured at the corresponding 3-D position. This value is typically transformed into the Hounsfeld unit (HU) scale. The HU scale is a linear transformation of the original linear attenuation coefficient measurement into one in which the radiodensity of distilled water at standard pressure and temperature (STP) is defined as zero HU, while the radiodensity of air at STP is defined as -1000 HU. High HU values of 400 and above correspond to materials with high density such as bone, while low HU values correspond to low-density materials such as fat (e.g., -100 to -50 HU), muscle (+10 to +40 HU), blood (+30 to +45 HU) etc. For the purpose of computer visualization the HU values are mapped to gray scale intensities.

Anatomy scans 108 may be sufficient to reconstruct a three-dimensional (3D) representation of the anatomy of the patient, such as the scapula and glenoid as examples of patient anatomy (e.g., by automated segmentation of the CT image data to yield segmented anatomical objects). One example way of automated segmentation is described in U.S. Patent No. 8,971,606. There may be various other ways in which to perform automated segmentation, and the techniques are not limited to automated segmentation using techniques described in U.S. Patent No. 8,971,606. As one example, segmentation of the CT image data to yield segmented objects includes comparisons of voxel intensity in the image data to determine bony anatomy and comparisons to estimated sizes of bony anatomy to determine a segmented object. Moreover, the example techniques may be performed with non-automated segmentation techniques, where a medical professional evaluates the CT image data to segment anatomical objects, or some combination of automation and user input for segmenting anatomical objects.

In one or more examples, anatomy scans 108 may be scans of anatomy that is pathological due to injury or disease. The patient may have an injured shoulder requiring surgery, and for the surgery or possibly as part of the diagnosis, the surgeon may have requested anatomy scans 108 to plan the surgery. A computing device (like device 100 or some other device) may generate segmentations of the patient anatomy so that the surgeon can view anatomical objects and the size, shape, and interconnection of the objects with other anatomy of the patient anatomy needing surgery. In trauma cases, not all fracture types necessarily require an operation. For example, 1-part or 2-part fractures usually do not require surgery, and shoulder immobilization is typically enough. For 2-part or 3-part fractures, a full surgery or minimally invasive intervention may be sufficient for treatment. As described in more detail elsewhere in this disclosure, device 100 may be configured to perform a fracture classification during a diagnostics phase. In cases where a surgeon decides to do a surgery, then device 100 may also be configured to plan the surgical intervention

In one or more examples, processing circuitry 102 may utilize image data of scans 108 to compare (e.g., size, shape, orientation, etc.) against a statistical shape model (SSM) as a way to determine characteristics of the patient anatomy prior to the patient suffering the injury or disease. In some examples, processing circuitry 102 may compare 3D point data of non-damaged points of anatomical objects of patient anatomy in the image data of scans 108 to points in SSM.

For instance, scans 108 provide the surgeon with a view of the current characteristics of the damaged or diseased anatomy. To reconstruct the anatomy (i.e., to represent a pre-morbid state), the surgeon may find it beneficial to have a model indicating the characteristics of the anatomy prior to the injury or disease. For instance, the model may be a predictor of the anatomy of the patient prior to damage or disease. However, it may be likely the patient did not consult with the surgeon until after the injury occurred or disease progressed, and therefore, a model of the patient anatomy prior to the injury or disease (e.g., pre-morbid or native anatomy) may not be available. Using the SSM as a way to model the pre-injured anatomy allows the surgeon to determine characteristics of the pre-morbid patient anatomy.

SSMs are a compact tool to represent shape variations among a population of samples (database). For example, a clinician or researcher may generate a database of image scans, such as CT image data, of different people representative of the population as a whole who do not suffer from damaged or diseased glenoid, humerus, or neighboring bones. The clinician or researcher may determine a mean shape for the patient anatomy from the shapes in the database. In FIG. 1, memory 104 stores information indicative of shape model 106. As one example, shape model 106 represents a mean shape for the anatomical object (e.g., glenoid, humeral head, etc.) of patient anatomy from the shapes in the database. Other examples of shape model 106 are possible, such as a mode or median of the shapes in the database. A weighted average is another possible example of shape model 106, Shape model 106 may be a surface or volume of points (e.g., a graphical surface or a volume of points that define a 3 D model), and the information indicative of mean shape may be coordinate values for vertices of primitives, such as triangles, that interconnect to form the surface. Various techniques of this disclosure are described with respect to shape modes that use triangles, but it should be understood that shape models may also use primitives other than triangles. Furthermore, it should be understood that other types of modeling, such as basis spline (BSpline)-based model, including non-uniform rational basis spline (NURBS) models, may also be used.

With shape model 106, processing circuitry 102 may represent anatomy shape variations by adding points or values of shape model 106 to a covariance matrix. For example, the SSM can be interpreted as a linear equation:

$$s_i = s' + \Sigma_i \, b_i \sqrt{\lambda_i} \times v_i.$$

In the above equation, s' is shape model 106 (e.g., point cloud of mean shape as one example, where point cloud defines coordinates of points within shape model 106, such as vertices of primitives that form shape model 106). In the equation, $\lambda_i$ is the eigenvalues and $v_i$ is the eigenvectors of the covariance matrix respectively (also called *modes of variations*). The covariance matrix represents the variance in a dataset. The element in the i, j position is the co-variance between the i-th and j-th elements of a dataset array.

SSM stands for constructing the covariance matrix of the database then performing "singular value decomposition" which extracts a matrix of principal vectors (also called eigenvectors) and another diagonal matrix of positive values (called eigenvalues). Eigenvectors ($v_i$ in the equation) are new coordinate system bases of the database. Eigenvalues ($\lambda_i$ in the equation) represent the variance around the eigenvectors ($v_i$). Together eigenvectors and eigenvalues may reflect the amount of variation around the corresponding axis.

This mathematical equation allows processing circuitry 102 to create an infinite number of instances of $s_j$ (e.g., different variations of the shape model) by changing the weights $b_i$ of the covariance matrix. For instance, to generate a new shape model, processing circuitry may determine a value of $b_i$, and determine a new value of $s_i$. In the above example, $\lambda_i$ and $v_i$ and s' are all known based on the manner in which s' was generated (e.g., based on the manner in which shape model 106 was generated). By selecting different values of $b_i$, processing circuitry 102 may determine different instances of a shape model (e.g., different $s_i$ which are different variations of the shape model 106).

The shape model(s) may represent what an anatomical object should look like for a patient. As described in more detail, processing circuitry 102 may fit points (e.g., in the 3D cloud of points) of the shape model of the anatomical object with anatomical points represented in the image data of scans 108, as anatomical points of the anatomical object that are not impacted or minimally impacted by the injury or disease (e.g., non-pathological points). Based on the fitting, processing circuitry 102 may determine a pre-morbid characterization of the anatomical object.

As an example, assume that the surgeon would like to determine the pre-morbid characteristics of the glenoid cavity for shoulder surgery. There is a correlation between the shape of the glenoid cavity and the surrounding bony zone, such as the medical glenoid vault, the acromion, and the coracoid. Shape model 106 may be the mean shape of the scapula that includes the glenoid cavity. Assume that in the patient the glenoid cavity is pathological (e.g., diseased or damaged).

In accordance with example techniques described in this disclosure, processing circuitry 102 may determine the instance of "s" (e.g., shape model of scapula with glenoid cavity) that best matches the non-pathological anatomy of the anatomical object of the patient (e.g., non-pathological portions of the scapula in scans 108). The glenoid cavity, in the instance of "s," that best matches the non-pathological anatomy (referred to as s* and represented by a point cloud similar to the shape model) may be indicative of the pre-morbid characterization of the pathological glenoid cavity.

Processing circuitry 102 may determine the instance of s* (e.g., shape model of scapula with glenoid cavity that best matches the non-pathological portions of the patient's scapula). The non-pathological portions may be portions of the anatomical object with minimal to no impact from the disease or injury, where the anatomical object and its surrounding anatomy are taken from the segmentation performed by scans 108 to segment out the anatomical object.

In some examples, to determine pre-morbid characterization of a particular anatomical object, anatomy beyond the anatomical object may be needed to align the shape model. For instance, to determine pre-morbid characterization of the glenoid, anatomy of the scapula may be needed to align the shape model. Then, the glenoid of the shape model represents the pre-morbid characterization of the patient's glenoid. Hence, the shape model may not be limited to modeling just the anatomical object of interest (e.g., glenoid) but may include additional anatomical objects.

Processing circuitry 102 may perform the following example operations: (1) initially align the segmented anatomical object from image data of scans 108 to a coordinate system of shape model 106 to generate an initial aligned shape; (2) compensate for errors in the initial alignment due to under and over segmentation, where under- and over-segmentation are due to imperfections in generating scans 108, to generate an aligned shape (also called intermediate aligned shape); and (3) perform iterative operations that includes iterative closest point (ICP) operations and elastic registration to determine the instance of s* (i.e., the instance of s' that most closely matches the patient anatomy as identified by the segmentation object). Example techniques to perform these operations are described in more detail below.

Accordingly, there may be a few technical problems with generating the pre-morbid characterization of the patient anatomy. One issue may be that due to under- or over-segmentation the image data needed from scans 108 is not available or distorted creating a challenge to align the segmented object to shape model 106. Another issue may be that once there is alignment to shape model 106, registration of shape model 106 to the segmented object may be poor, resulting in poor pre-morbid characterization.

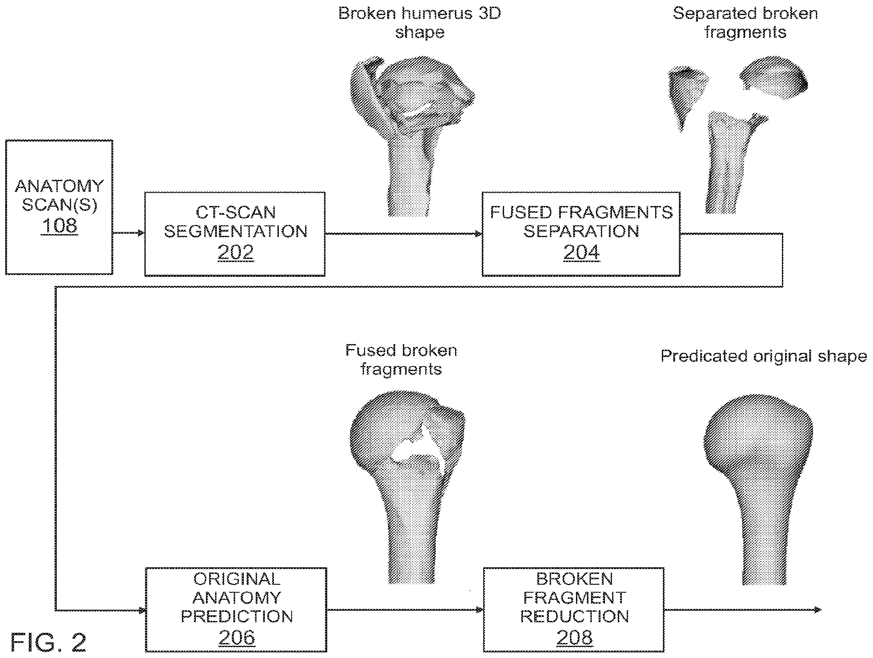

When encountering a fracture case, processing circuitry 102 may implement different processing techniques than for non-fracture cases. FIG. 2 shows an example workflow of processing circuitry 102 for fracture cases. In the example of FIG. 2, and as will be explained in greater detail below, processing circuitry 102 performs CT-scan segmentation

(202) on anatomy scan(s) 108, performs fused fragments separation (204), performs originally anatomy prediction (206), and performs broken fragments reduction (208). Processing circuitry 102 may also perform, if needed, predicted shape adjustment based on the reduced fragments.

The human body has two types of bone tissue: cortical and cancellous (also known as spongy) bone. Cortical bone has a sturdy calcified matrix typically with very few spaces and forms a protective shell around the spongy bone tissue. Cortical bone provides the rigidity to the bones. Cortical bone may also be referred to as compact bone, as cortical bone looks to have been "compacted" together. Due to this compactness, the cortical bone appears very bright in a CT image. Higher cortical thickness is found on a diaphysis where the cortical bone encloses the medullary cavity consisting of bone marrow. Towards the joints, the thickness of cortical shell decreases, and the medullary cavity is replaced by cancellous bone. In total, spotic, bone makes up about 20% of the typical adult human skeleton. Cancellous bone is a highly vascular and porous structure with the main functions to absorb, damp and transfer loads acting at the joints. In addition, cancellous bone contains most of the body's red bone marrow that produces blood cells.

The interior of the humeral head is formed by cancellous bone thatis surrounded by a thin cortical layer, such thatin some regions the cortical shell is barely visible. Due to these facts, the contrast to the surrounding tissue is much lower compared to the diaphysis zone. This is particularly true for the elderly or patients suffering from osteoporosis whose bone mineral density is decreased.

Processing circuitry 102 may be configured to rely on a 3D model of a cortical shell such that each voxel belonging to cortical bone can be identified in the CT-Scan. This procedure is referred to as segmentation and can be divided into two parts: diaphysis segmentation and broken head segmentation. Thresholding may be used for diaphysis segmentation, using the high contrast between the bone shaft and soft tissue.

By contrast, humeral head segmentation is a very challenging task due to the following problems. First, the humeral head produces weak bone boundaries. As has been previously stated, the cortical shell is very thin at the level of humeral head resulting in similar intensity as the surrounding tissue on CT images. Second, the humeral head produces varying density. The density of long bones varies substantially along the longitudinal axis. As a consequence, intensity histograms of bone and tissue do frequently overlap. Therefore, a segmentation only relying on a global estimation of the intensity is not accurate. Third, fusion between broken fragments and scapula makes segmentation difficult. A fracture near a joint can include multiple thin-walled bone fragments which are in close proximity or direct contact. The segmentation of such data can be extremely difficult, in particular when a broken fragment is fused with a scapula.

A number of techniques for segmenting 3D medical images currently exist. These techniques may generally be divided into two groups - (1) low-level algorithms and (2) high-level algorithms. Processing circuitry 102 may be configured to perform low level or high level segmentation algorithms. Low-level algorithms directly operate on the voxels of the given intensity images. Such algorithms are usually not sufficiently flexible to solve all problems encountered during bone segmentation. High-level algorithms include additional knowledge, such as shape or appearance in a mathematical model to determine the segmentation.

Thresholding is a fast low-level segmentation techniques that is widely used for bone segmentation. Thresholding alone may perform poorly if the object and background intensities do not vary substantially. Therefore, thresholding alone may be insufficient when segmenting bone parts near joints with similar intensity as soft tissue (e.g., the proximal humerus).

Another low-level segmentation technique is region growing. One common region-based segmentation algorithm is seeded region growing, where starting from an initially defined seed, neighboring voxels are incrementally added to the region based on a user-defined similarity measure, e.g. the intensity between neighboring voxels. The main drawback of region-based algorithms for bone segmentation is the tendency to leak into tissue due to weak boundaries and merging bones that are close together.

Another low-level segmentation technique is edge detection based on, for example, first-order or second order derivatives of the image intensity function. Such techniques produce excellent results on data sets with good contrast between the bone shaft and soft tissue, but the performance may be poorer at joint boundaries (e.g., the humeral head) resulting in discontinuities.

Another low-level segmentation technique is referred to as a watershed algorithm. The basic idea is to interpret the image values as a height-map that is "flooded" with water from different sources, which can be local minima in the image or user-defined markers. For each source, a catchment basin (i.e. labeled region) is constructed which grows during successive flooding. Watersheds, representing the segmented region boundaries, are built for adjacent basins to avoid merging. While the watershed transform is effective in separating regions, a combination with a different method such as thresholding is required to achieve a bone segmentation. Therefore, watershed transform shares the same problems as thresholding.

Various high-level segmentation techniques utilize statistical models of shape and appearance. For the segmentation of anatomy, a statistical shape model may be fit to provided data by deforming the mean shape with respect to the trained modes of variations. Therefore, the deformation is constrained to statistically valid directions resulting in a robust segmentation. While statistical models have proven to be a powerful tool for the segmentation of healthy bones, these methods are not particularly suited for traumatological cases due to the huge pathological variability caused by broken fragments displacement.

Various high-level segmentations techniques utilize energy minimization in conjunction with deformable models. A 3D parametric deformable model may be represented by a parameterized surface of the segmented object. The model is deformed according to external image forces and internal forces, resulting in corresponding terms in the overall energy function. Internal energy enforces the smoothness of the surface while the surface is pulled towards the segmentation target by external image forces. These models have several limitations. The performance of the algorithms is poor in case of elongated structures with low intensity as well as for highly textured bone parts. Additionally, the quality of the initialization of a parametric model is crucial for the segmentation result, where insufficient initialization can result in slow convergence or the attraction of the model to incorrect features.

Various high-level segmentations techniques utilize energy minimization in conjunction with deformable graph cuts. One such technique provides interactive segmentation of n-dimensional images through energy minimization via graph cuts. Interactive approaches based on the image intensity were proposed for bone segmentation, where the algorithms generate intensity distributions of the object and background from user-defined strokes. Such techniques may be very time-consuming and require strokes to be accurately placed on critical areas where the cortical shell is thin and less pronounced. Another important issue is the optimal parameter selection. Usually each image has a different set of optimal parameters and significant user interaction may be required if parameter settings are far from optimal.

Processing circuitry 102 may be configured to implement one or more of the segmentation techniques introduced above. In view of the shortcomings of those techniques, however, processing circuitry 102 may also be configured to perform image enhancement with local structure analysis. For example, processing circuitry 102 may be configured to use first- and second-order intensity derivatives to detect local structures in images. In order to handle the segmentation of cortical bone layer with varying thickness, processing circuitry 102 may be configured to use an enhancement filter, such as vesselness enhancement filter.

The enhancement filter may enable processing circuitry 102 to segment images with low contrast between object and background regions. Moreover, the enhancement filter is also efficient on noisy images. Processing circuitry 102, in applying the enhancement filter, performs a local structure analysis around each voxel in the dataset and computes a measure that is able to distinguish cortical voxels from other voxels (air, spongy bone, noise etc.). Processing circuitry 102 may be configured to search for geometrical tubular structures. Since vessels appear in different sizes and may be measured with a measurement scale "s"' which varies within a specific range. By analyzing the second order derivative information (Hessian) of a Gaussian kernel at scale "s," processing circuitry 102 can measure the contrast between the regions inside and outside the range (-s, s) in the direction of the derivative (gradient).

As part of the eigenvalue decomposition, processing circuitry 102 may extract three orthonormal directions (eigenvectors) and with magnitudes (eigenvalues) which are invariant up to a scaling factor when mapped by the Hessian matrix. The eigenvalues can be sorted in a decreased order: $|\lambda 1| < |\lambda 2| < |\lambda 3|$. A voxel is classified as tubular structure if $\lambda 1$ being small (ideally zero) and $\lambda 2$ and $\lambda 3$ have high magnitudes and same sign (the sign is an indicator of brightness/darkness). Thus, an ideal tubular structure voxel is characterized by:

$$|\lambda 1| \approx 0 \tag{2.1}$$

$$|\lambda 1| \quad |\lambda 2| \tag{2.2}$$

$$\lambda 2 \approx \lambda 3 \tag{2.3}$$

Processing circuitry 102 may calculate a dissimilarity measure based on two geometric ratios. The first ratio accounts for the deviation from a blob-like structure but cannot distinguish between a tube- and a plate-like pattern:

$$R_b = \frac{|\lambda 1|}{\sqrt{|\lambda 2||\lambda 3|}} \tag{2.4}$$

The second ratio accounts for the aspect ratio of the two largest second order derivatives. This ratio is essential to distinguish plate-like and tube-like structures since only the latter case is zero:

$$R_a = \frac{|\lambda 2|}{|\lambda 3|} \tag{2.5}$$

To cope with the noise presented in the acquired images, processing circuitry 102 may use a Frobenius matrix normalization as follows:

$$S = \sqrt{\lambda 1^2 + \lambda 2^2 + \lambda 3^2} \tag{2.6}$$

This measure may be expected to be low in the background where no structure is present, and the eigenvalues may be expected to be small for the lack of contrast. In regions with high contrast compared to the background, the norm may become larger due to at least one of the eigenvalues being large. The final combination of a vesselness function components is defined as follows:

$$V(s) = \begin{cases} 0 & if\ \lambda 2 > 0\ or\ \lambda 3 > 0 \\ (1 - exp(-\frac{R_a^2}{2\alpha^2}))exp(-\frac{R_b^2}{2\beta^2})(1 - exp(-\frac{S^2}{2c^2})) \end{cases} \tag{2.7}$$

where $\alpha$, $\beta$ and c are thresholds which control the sensitivity of the filter to the measures Ra, Rb and S. The criteria are combined using their product to ensure that the response of the filter is maximal only if all three criteria are fulfilled. Processing circuitry 102 analyzes the vesselness measure in Equation (2.7) at different scales s. The response of the filter may be maximum at a scale that approximately matches the size of the vessel to detect.

$$V_{max} = \max_{s_{min} \leq s \leq s_{max}} (V(s)) \tag{2.8}$$

where smin and smax are the maximum and minimum scales at which relevant structures are expected to be found.

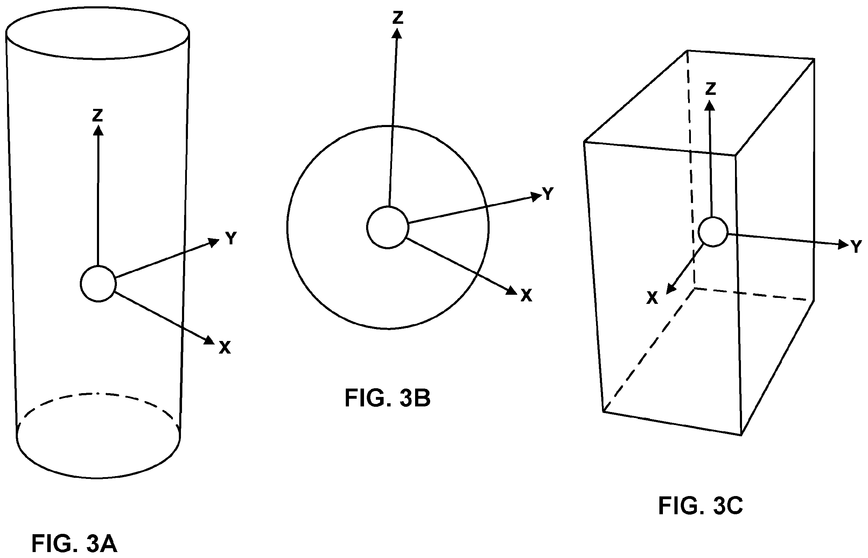

FIGS. 3A-3C show Hessian eigenvector decomposition for tubular-, blob- and sheet-like structures. FIG. 3A shows eigenvectors of a central voxel of a tubular structure. FIG. 3B shows eigenvectors of a central voxel of a blob structure. FIG. 3C shows eigenvectors of a central voxel of a sheet structure.

Processing circuitry 102 may implement a bone enhancement filter, particularly for scenarios, in the segmentation of the bone structure, where the cortical bone appears as a sheet-like structure. Processing circuitry 102 may be configured to use the geometric ratios Rb, Ra and S described above or may use the slightly modified Rb ratio shown below.

$$R_b = \frac{|\, 2|\lambda_3| - |\lambda_2| - |\lambda_1|\,|}{|\lambda_3|}$$

$$(2.9)$$

This measure is equal to 2 for a sheet-like structure, to 1 for a tube-like structure, and becomes zero for blob structures.

In order to expedite processing, processing circuitry 102 may define zones where the Hessian segmentation is applied. Specifically, processing circuitry 102 may perform the following four steps, which will be described in more detail below : (1) humerus and scapula automatic classification(no Hessian computing at this stage), (2) automatic identification of broken head zone; (3) automatic identification of the other broken fragments zone; (4) estimation of the optimal Hessian parameters.

For humerus and scapula automatic classification, processing circuitry 102 may be configured to perform image down-sampling via an automatic algorithm that optimizes the image dimensions to reduce the processing time. Processing circuitry 102 may also be configured to perform coarse segmentation by applying thresholding to a down-sampled dataset where all voxels with HUS above 200 are set to 1 and the rest of the voxels are set to zero. Processing circuitry 102 may be configured to perform an initial classification that starts with a classification based on morphpmetric features of binary forms detected after thresholding. Then, each form is labeled as scapula, humerus, or other. Processing circuitry 102 may be configured to perform diaphysis axis computation using gravity centers of the diaphysis forms on each slice to best fit an axis. Processing circuitry 102 may be configured to perform accurate scapula detection. It may be important to have an accurate scapula segmentation for further virtual surgical planning. During this stage, certain techniques are used to enhance scapula segmentation and classification.

For automatic identification of a broken head zone, processing circuitry 102 may be configured to implement an automatic proximal head segmentation algorithm based on a bone enhancement filter. As the humeral head is not attached to any muscle, the displacement of the humeral head after a fracture is not necessarily limited to specific location or direction. Depending on an accident type, applied forces and pre-hospital immobilization, the humeral head may be found in different locations, some close to the original location and some far from the original location.

In order to define a humeral head segmentation zone, processing circuitry 102 may be configured to execute a fully automatic algorithm that is able to identify the broken head center and its radius on the CT-Scan exam image. To do so, processing circuitry 102 computes gradient vectors for all voxels with HU values superior to 0. Processing circuitry 102 then performs a non-maximum suppression, where a voxel that has a neighbor along the gradient vector with a larger magnitude is set to zero. Then, for each noti-zero voxel, processing circuitry 102 constructs a gradient line that is centered at the voxel center and is prolongated for 35mm into two directions: along the gradient vector and in opposite direction. The value of 35mm represents a statistically maximal humeral head radius, but other values may also be used. Processing circuitry 102 then computes the intersections between each gradient line and image voxels; if a line intersects a voxel, a counter is incremented by one. A voxel with the maximum number of intersections can be used to identify a center of a humeral head. Processing circuitry 102 may also determine gradient lines that intersect the humeral head center and their origins (voxel centers) to compute a humeral head radius. The algorithm may be invariant to translation and rotation, and thus able to detect a broken humeral head at any location on a CT image.

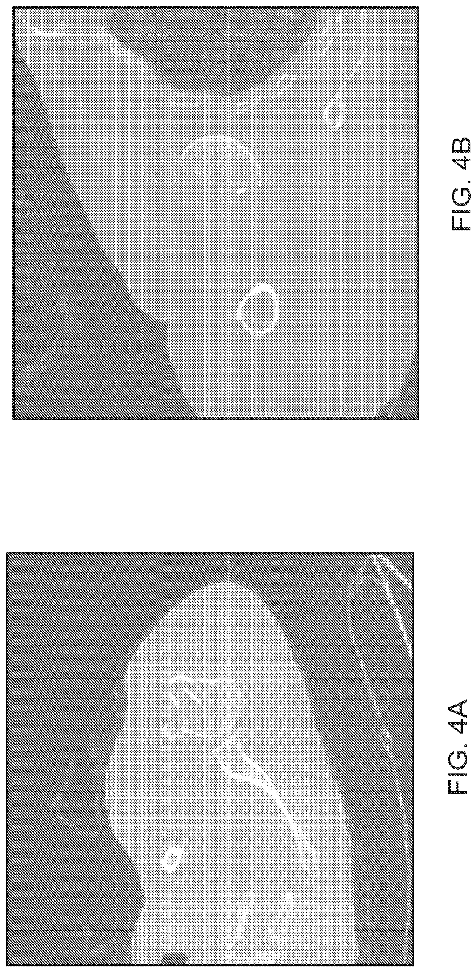

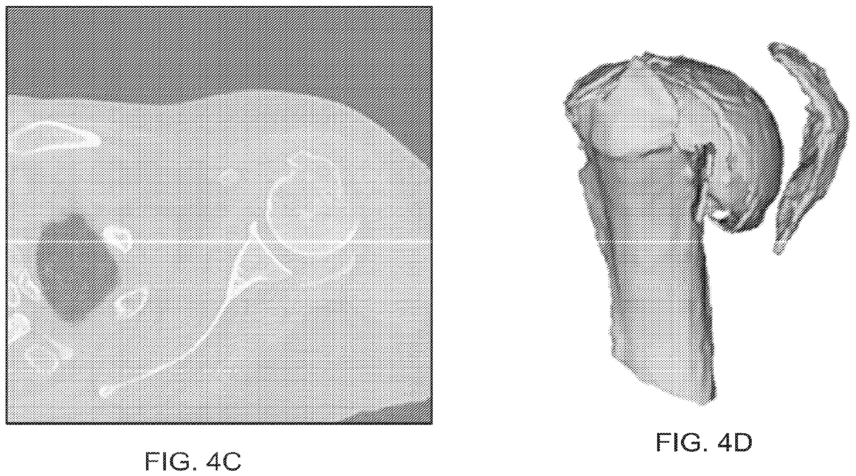

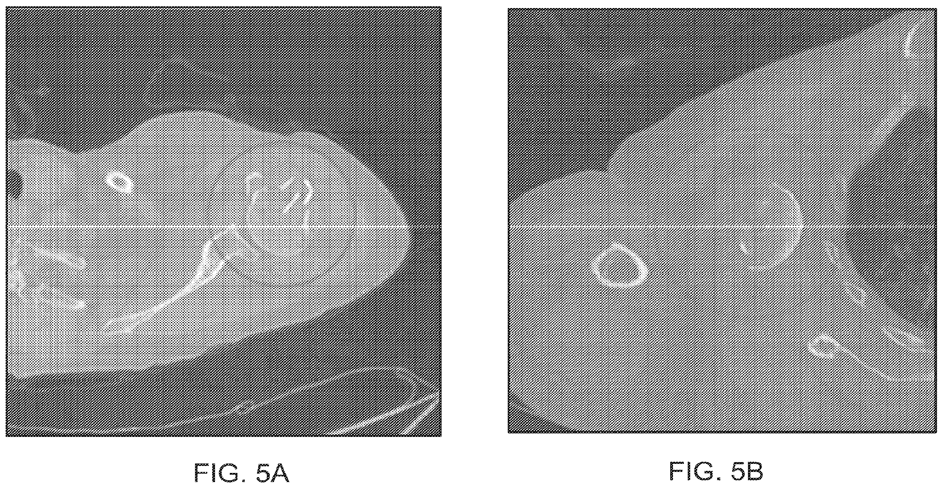

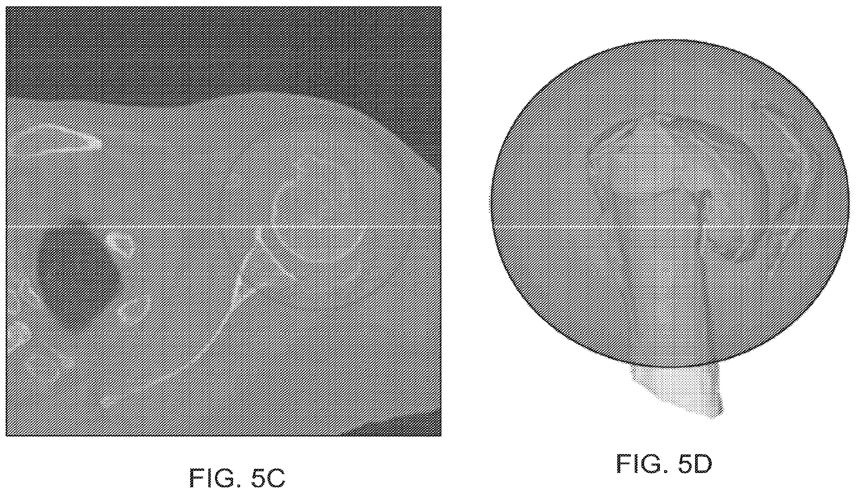

FIGS. 4A-4D show examples of different locations of a displaced broken humeral head. FIG. 4A shows an example of a humeral head close to original location. FIG. 4B shows an example of a humeral head far from original position. FIG. 4C shows an example axial view of a humeral head pushed lesser tuberosity. FIG. 4D shows an example 3D view of a humeral head pushed lesser tuberosity. FIGS. 5A-5D show the automatic detection of broken humeral head and the associated segmentation zone for the examples of FIGS. 4A-4D.

Processing circuitry 102 may be configured to perform automatic identification of the other broken fragment zone. Processing circuitry 102 may be configured to perform lesser and greater tuberosities detection and segmentation. The restoration of the anatomical position of greater tuberosity has a direct impact on surgery outcomes. Greater and lesser tuberosities detection is important because the greater and lesser tuberosities contain shoulder muscle insertions. Greater tuberosity is attached to supraspinatus, infraspinatus and teres minor muscles while lesser tuberosity is attached to subscapularis and teres major muscles. Due to this fact, broken tuberosities are often close to their original location.

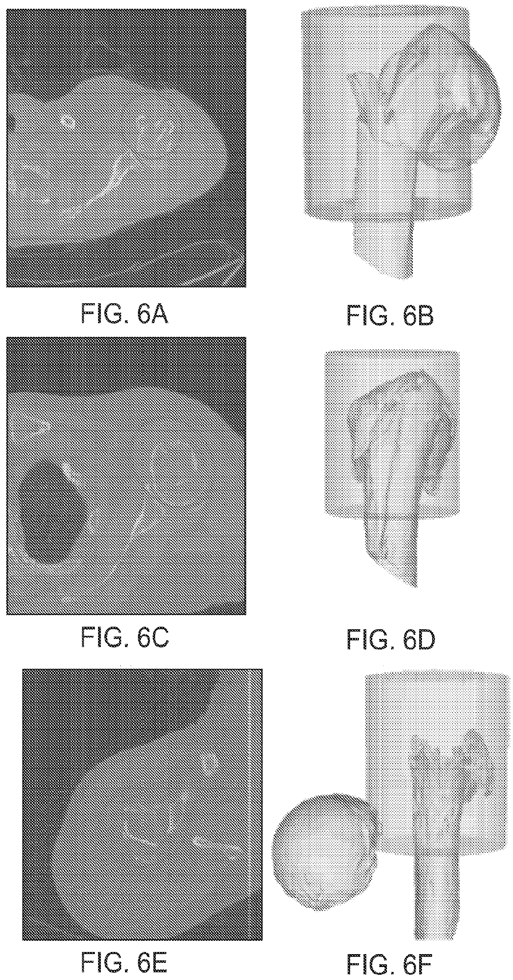

In order to determine a volume of interest, processing circuitry 102 may be configured to use the diaphysis centerline axis, calculated as described above. A cylinder is created around this axis and is used as a region of interest (ROI) for the broken tuberosities. FIGS. 6A-6F show examples of the identified broken fragment ROIs.

Processing circuitry 102 may be configured to optimize the Hessian parameters described above. As described above, two cost functions (vesselness functions) are proposed for analyzing local image structures. One is developed for vessel detection and the other one for bone enhancement. Both of these functions contain several parameters that control the sensitivity of the filter. In some examples, processing circuitry 102 may set $\alpha$ and $\beta$ to 0.5 and c to half the value of the maximum Hessian norm. In other examples, processing circuitry 102 may set to c to a range between 60 to 150 in order to regulate the noise suppression.

Another important parameter for the local structure analysis filter is the scale, which represents the anatomical structure size that should be detected (a cortical thickness for bone analysis and a vessel diameter for vessel segmentation). In some example, a scale in the range from smallest to thickest possible bone structure ($0.5 \leq s \leq 3.0$) may be used. In other example, such as for a segmentation of proximal humerus fracture, the sheetness measure may be evaluated for two scales 0.75 and 1.0, representing the average cortical thickness in the proximal part of the humerus.

A thresholding level applied to the output image after Hessian processing is another parameter for coping with image noise. Another parameter used during the segmentation process is a $\sigma$ of a Gaussian smoothing filter. Processing circuitry 102 may use $\sigma$ to smooth an input image before applying a local structure analysis filter. Higher values of $\sigma$ may significantly blur the bone edges and introduce a significant error, while small values might not be enough in case of important horizontal noise.

Thus, there are six different parameters (Gaussian smoothing $\sigma$, $\alpha$, $\beta$, c, Hessian scale s, Threshold) that may be adjusted in order to obtain the best possible segmentation of a proximal humerus fracture. Example determinations for these six different parameters are described in more detail elsewhere in this disclosure.

Processing circuitry 102 may be configured to determine a predicted humerus shaped based on a humerus statistical shape model. SSMs have become a widely-used tool and have been studied extensively for statistical analysis of 3D shapes, including for medical applications. Such models draw meaningful statistical conclusions about a class of shapes, and therefore, can be used as prior information for object segmentation, analysis and processing tasks. A typical application is model-based segmentation. This is normally used when lack of information exists and thus a priori information about the missing parts is provided by the model. Using SSMs, processing circuitry 102 may be configured to separate shape variations into groups, which form bases of a shape space.

In the orthopedic domain, SSMs have been frequently applied to several bones of the human body other than the humerus. According to the techniques of this disclosure, as part of determining a predicted humerus shaped based on a humerus statistical shape model, processing circuitry 102 may be configured to perform anatomical landmark detection, including humeral head detection. Processing circuitry 102 may be configured to detect the humeral head using the same techniques described above for automatic identification of a broken head zone. For example, processing circuitry 102 may construct an octree out of an input mesh, define a line with the vertex and a normal for each vertex on a mesh, and increase a voxel's internal counter by one when any line intersects a structure element (e.g., a voxel) of the octree. Using these techniques, processing circuitry 102 can detect the center of the humeral head as the octree structure element with the biggest number of intersections. Processing circuitry 102 may also record vertex IDs that belong to lines intersecting this structure element, which correspond to humeral head vertices.

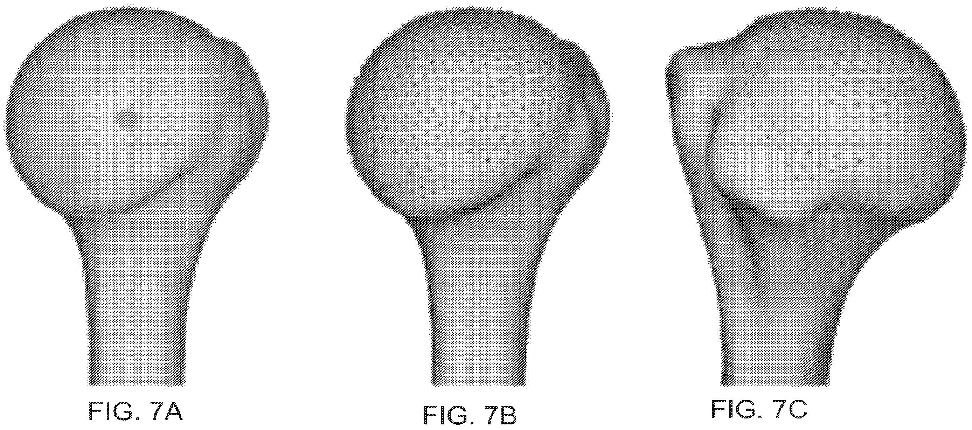

FIGS. 7A-7C show an example of a detected humeral head. Processing circuitry 102 detects the center of the humeral head. During humeral head vertices detection, however, some outliers may be detected. FIG. 7A shows an example of humeral head center detection. FIG. 7B shows an example of humeral head vertices detection. FIG. 7C shows an example of humeral head vertices detection.

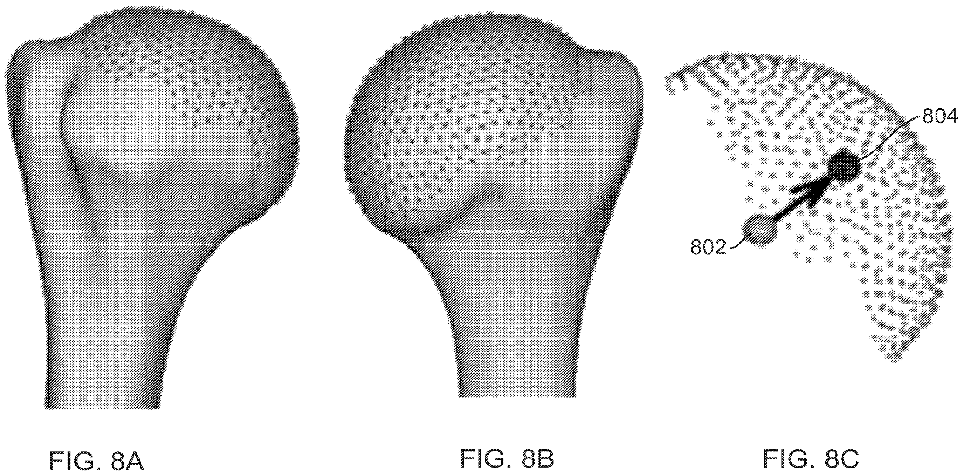

In order to remove these outliers, processing circuitry 102 may be configured to remove small disconnected detected regions (e.g., size less than 10 vertices), detect a direction from the humeral head center (FIG. 8C, point 802) to the barycenter of the detected vertices (FIG. 8C, point 804). Processing circuitry 102 may remove any vertex whose direction is opposite to the humeral head direction. Processing circuitry may also apply a morphological closing operation. FIGS. 8A-8C show an example of cleaned humeral head points.

Processing circuitry 102 may be configured to detect the humeral diaphysis axis by first computing an oriented bounding box (OBB). The maximal vector of this OBB may be the first approximation of the diaphysis axis. Processing circuitry 102 may then cut the humerus with planes perpendicular to the biggest OBB axis.

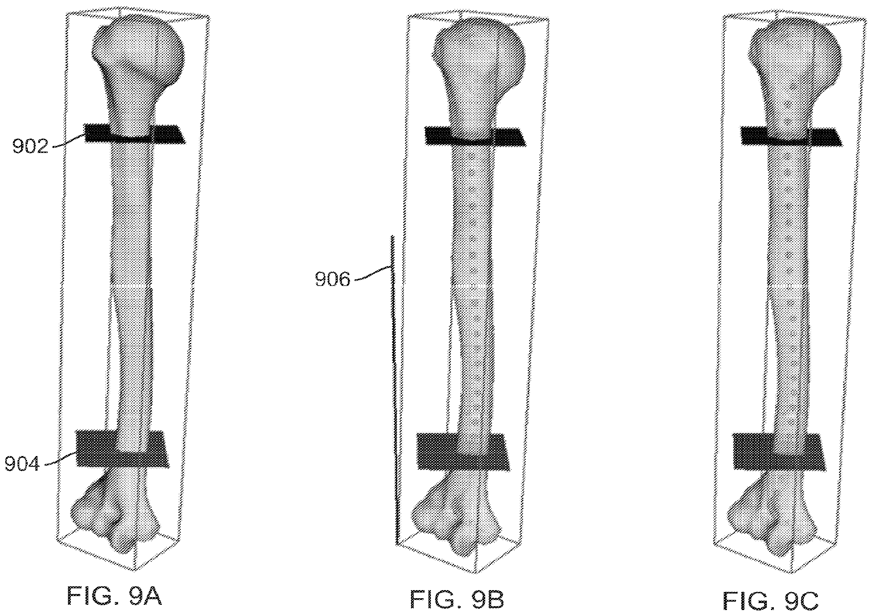

Processing circuitry 102 can compute start and end limit points using the following equations and as shown in FIGS. 9A and 9B, with plane 902 and 904.

$$\text{startPoint} = \text{OBBCorner} + 0.2 \times \text{OBBMaxVecto} \qquad (3.1)$$

$$\text{endPoint} = \text{OBBCorner} + 0.8 \times \text{OBBMaxVector} \qquad (3.2)$$

Where 0.2 and 0.8 represent 20 and 80 percent of the humerus length. These values were chosen based on the statistical mean shape, and such that the planes that pass at these levels never cross the humeral head and the elbow regions. Other values may also be used.

FIGS.9A-9C show an example of diaphysis points computing. FIG. 9A shows an example of humerus OBB and cut limits for diaphysis axis computing. FIG. 9B shows an example of points for axis computing and diaphysis axis. FIG. 9C shows an example of diaphysis banding points.

For each cut, processing circuitry 102 detects intersected points between the 3D mesh and cutting plane and computes a barycenter for these points. The diaphysis axis is computed based on line fitting to barycenters, as shown by line 906 in FIG. 9B.

Processing circuitry 102 may also be configured to compute diaphysis banding points as barycenters of intersections between perpendicular planes to the newly computed diaphysis axis and a 3D humerus mesh. For this process, processing circuitry 102 enlarges the start and end limits as follows:

$$startPoint = OBBCorner + 0.1 \times OBBMaxVector \qquad (3.3)$$

$$endPoint = OBBCorner + 0.9 \times OBBMaxVector \qquad (3.4)$$

where 0.1 and 0.9 represent 10 and 90 percent of the humerus length. These values were determined based on the statistical analysis, such as boundary locations that separate humerus into three parts: humeral head, diaphysis and elbow. Other values may also be used. FIG. 9C shows examples of the determined banding points, which may be used during an elastic registration in order to match humerus diaphyses.

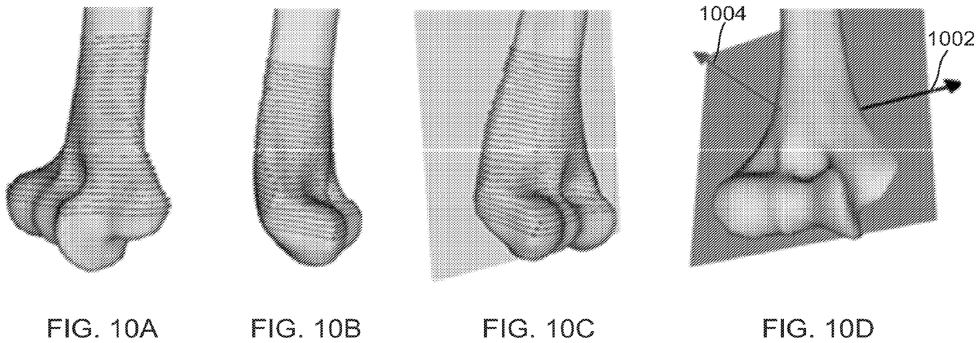

Processing circuitry 102 may also be configured to determine an elbow axis and orientation. Processing circuitry 102 detects an elbow plane that passes through the elbow medial and lateral column ribs. To detect points located on these ribs, processing circuitry 102 performs the following steps: (1) on the elbow level, cut the humerus 3D mesh with a plane perpendicular to the diaphysis axis ( black points on FIGS. 10A and 10B); for each cut, detect intersection points between the mesh and the intersection plane; order detected points to create a closed contour; for each closed contour, apply a discrete curve evolution algorithm to determine the two most significant points of the contour (the dots on FIGS. 10A and 10B).

FIGS. 10A-10D shows an example of diaphysis points computing. FIG. 10A shows an example of elbow column rib detection. FIG. 10B shows an example of elbow column rib detection. FIG. 10C shows an example of an elbow plane. FIG. 10D shows an example of an elbow axis (line 1002).

Processing circuitry 102 may be configured to compute the elbow plane as the best fit plane to the detected rib points, as shown in FIG. 10C. The elbow axis is computed as the vector product between diaphysis axis and the elbow plane normal. An example is shown in FIG. 10D, where the elbow plane normal is shown by line 1004 and the elbow axis by line 1002. The elbow axis points in the same direction as the humeral head. To guarantee a correct elbow axis direction, processing circuitry 102 may be configured to determine a scalar product between the elbow axis and the humeral head direction, as described above with respect to humeral head detection, and if the product is negative, then flip the elbow axis.

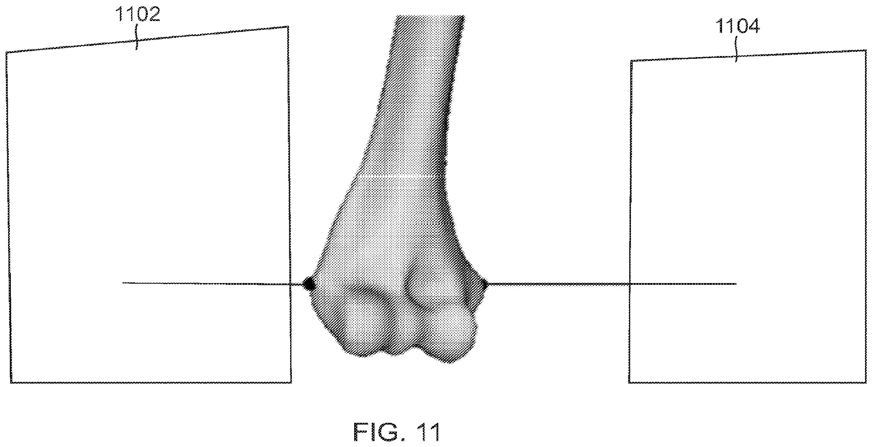

Processing circuitry 102 may be configured to detect an elbow correspondence point by determining the epicondiler axis based on the elbow's most lateral and medial points. To identify these points, processing circuitry 102 constructs two planes whose normals are equal to the elbow axis direction. Processing circuitry 102 obtains the origin points by translating humeral head center by, for example, 10 cm along the elbow axis direction (medial plane origin) and opposite direction (lateral origin), as shown by planes 1102 and 1104 in FIG. 11. Ten cm is a distance that is much large than an elbow size based on statistical analysis, which ensures that the constructed planes do not intersect the elbow.

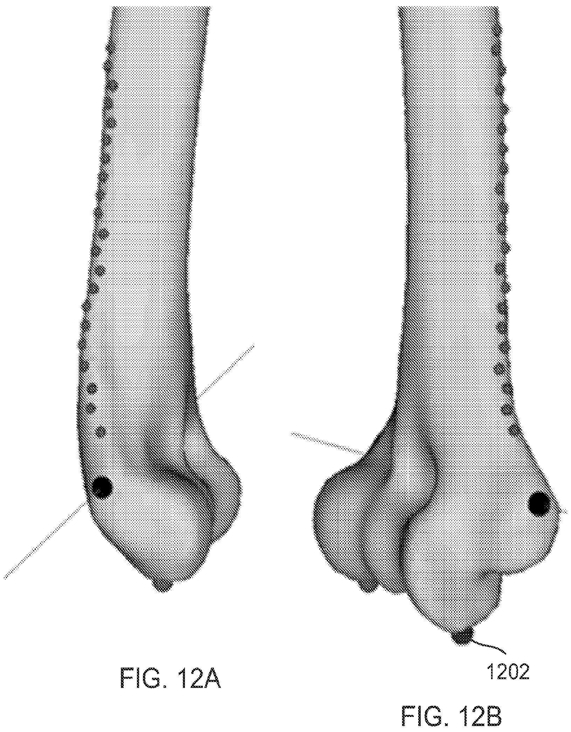

FIG. 11 illustrates most medial and lateral elbow points detection. In order to find the most medial elbow point, processing circuitry 102 finds the closest point to the medial plane 1104. In the same manner, using lateral plane 1102, processing circuitry 102 determines the most lateral point. The elbow is divided into two parts (medial and lateral) based on the medial point of the epicondilar axis, as shown in FIG. 12A.

FIGS. 12A and 12B show elbow landmarks. FIG. 12A shows a lateral view, and FIG. 12B shows a medial view.

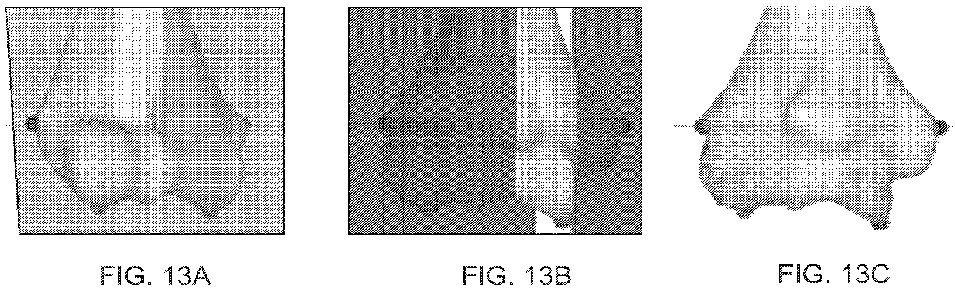

In the medial part, processing circuitry 102 determines the farthest elbow point from the elbow axis line that corresponds to the trochlea lowest point (point 1202 in FIG. 12B). Using this point, processing circuitry 102 determines a trochlea zone by defining two planes. The first plane is defined by a normal direction equal to the elbow axis and a trochlea point medially shifted by, for example, 3 mm along the elbow axis. Three (3) mm value is chosen based on the elbow anatomy, as medially the trochlea resembles a plane so that a small shift contains the trochlea part. The second plane is defined with the same normal and a trochlea point laterally shifted by, for example, 10 mm along the elbow axis. This time the distance is chosen experimentally with the aim of obtaining at least a half of the trochlea volume. An example of the trochlea zone is shown on figure 13B.

Processing circuitry 102 may also be configured to detect a trochlea center by applying the humeral head detection algorithm described above with respect to humeral head detection. This algorithm is a general method that is able to detect spherical or circular structures on 3D images. The trochlea center detected using this technique generally corresponds to an intuitive center that a human would choose. Processing circuitry 102 may be configured to use a similar approach for capitulum landmark detection. The obtained results are shown in FIG. 13C.

FIGS. 13A-13C illustrate trochlea and capitulum landmark detection. FIG. 13A shows an example of the elbow medial and lateral parts. FIG. 13B shows an example of trochlea detection. FIG. 13C shows an example of trochlea and capitulum internal landmarks.

Processing Circuitry 102 may also be configured to detect elbow column ribs points by applying the discrete curve evolution algorithm as previously described, but in this instance, starting to cut the humerus at the elbow axis line level. An example of the obtained elbow landmark points are shown in FIGS. 12A and 12B.

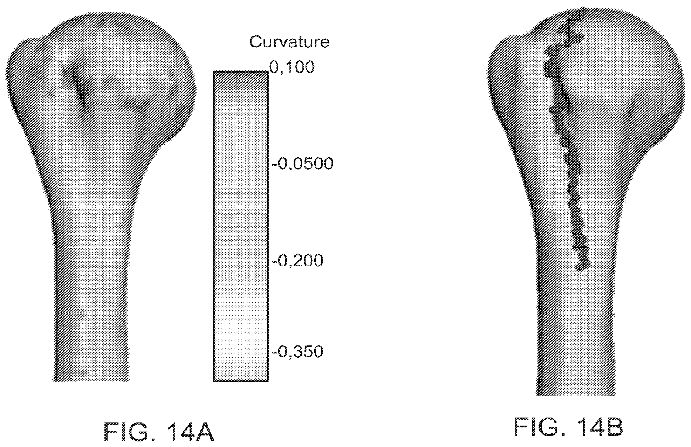

FIGS. 14A and 14B show an example of humerus bicipital groove detection. FIG. 14A shows bicipital groove minimal curvature, and FIG. 14B shows bicipital groove points. Processing circuitry 102 may be configured to perform bicipital groove detection by performing localization and detection that lasts until the upper end of the humeral head is reached. First, processing circuitry 102 computes a diaphysis starting point (Pstart) as the humeral head center point shifted by two head radii along diaphysis axis towards the elbow. At this level, the bicipital groove shape is concave towards the diaphysis center and can be characterized by low curvature value (e.g., FIG. 14A) and close distance to the diaphysis center. In order to localize the bicipital groove points, processing circuitry 102 computes an intersection between the diaphysis and a plane defined by the diaphysis axis and the Pstart point. For each intersection point a cost function is computed as follows:

$$Kgroovei = Di + 2 \times C \tag{3.5}$$

where D is a Euclidean distance from a point to the diaphysis axis, and C is the minimal curvature value at the analyzed point. Processing circuitry 102 determines the bicipital groove point as a point with the minimal cost function. Processing circuitry then continues the bicipital groove point detection by shifting the intersection plane upper along the diaphysis axis. For each intersection point, processing circuitry 102 computes the cost function as shown in equation 3.5 above and identifies a point with the minimal cost function value. At the humeral head level, this algorithm may fail due to several zones with low curvature. To cope with this issue, processing circuitry 102 may also be configured to use a tracking technique in order to approve the selected points. An example of detected bicipital groove points is shown in FIG. 14B.

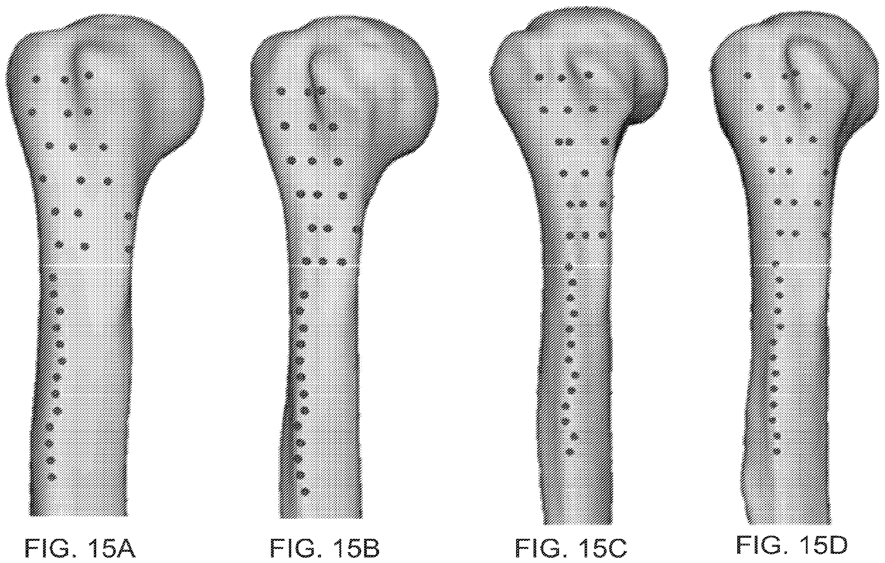

Processing circuitry 102 may use these points as references for the upper diaphysis landmark detection. Starting at the humeral head center level, processing circuitry 102 may compute an intersection between the humerus mesh and a plane perpendicular to the diaphysis axis. For each intersection point, processing circuitry 102 may apply a discrete curve evolution algorithm in order to detect the most significant points. Processing circuitry 102 may chose the two closest points to the previously computed bicipital groove point as upper diaphysis landmarks. Moving down the intersection plane along the diaphysis axis, processing circuitry 102 repeats the previous procedure in order to compute the upper diaphysis landmark. The algorithm may stop, for example, after reaching a distance equal to 2 humeral head diameters (D). An example of detected landmarks are shown in FIGS. 15A-15D for four different humeri.

Once the intersection plane is at a distance D, processing circuitry 102 may implement a difference algorithm. The concave ridge that descends from the lesser tuberosity vanishes approximately at this level (2 humeral head diameters from the humeral head center). To detect points on the ridge that descends from the greater tuberosity, processing circuitry 102 applies the discrete curve evolution algorithm and then uses a tracking algorithm which, with based on the previously detected landmarks, is able to track the points on the greater tuberosity ridge.

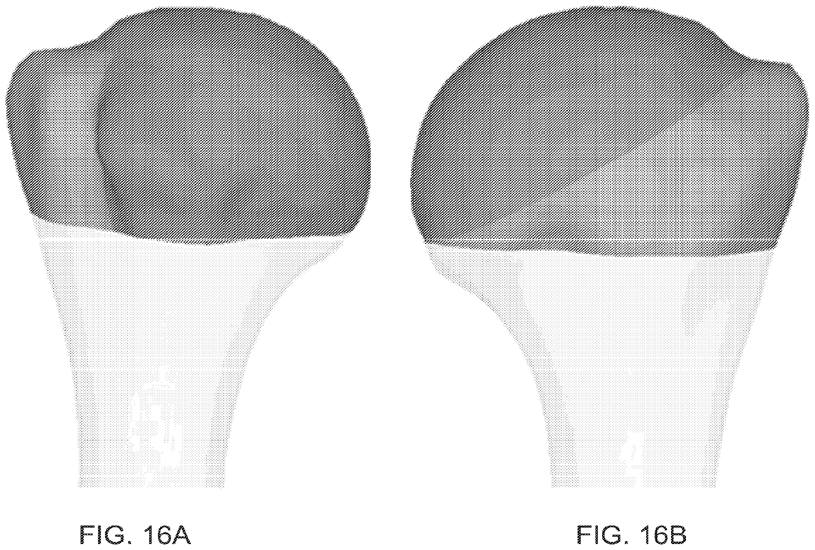

The humerus retroversion varies significantly across the population. In order to establish a correct correspondence between humeral heads of two humeri, processing circuitry 102 may be configured to divide a humerus into several specific zones in order to establish zone-to-zone correspondences. Processing circuitry 102 divides the proximal humerus into three zones: humeral head, greater- and lesser-tuberosities. To do so, processing circuitry defines two planes: (1) humeral head plane - a best fit plane (in a least square sense) to the previously detected humeral head points that passes by the articular center; and (2) tuberosity plane - a plane that is defined by a line fitted to the previously detected proximal bicipital groove points (in a least square sense) and the articular center. Processing circuitry 102 uses these planes to cut out the humeral head and to separate two tuberosities. An example of the obtained results is shown in FIGS. 16A and 16B, which show examples of upper humerus zones for correspondence establishment.

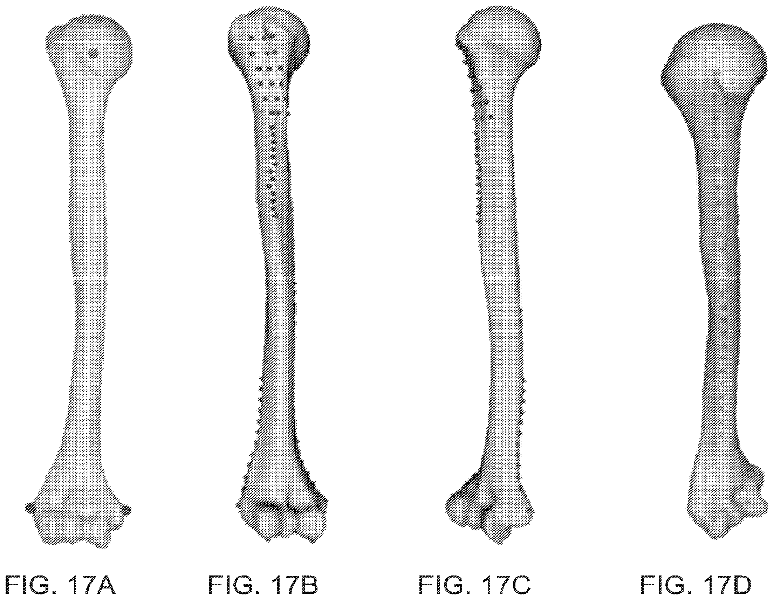

To find the correspondence between two surfaces, processing circuitry 102 may be configured to minimize the distance between them. The correspondence then is based on the minimal matching distance. To perform distance minimization between two meshes, processing circuitry 102 may be configured to first perform uniform scaling, which is a method that brings meshes to the same size by equalizing the longest vectors of the oriented bounding box. Processing circuitry 102 may secondly perform rigid registration - a method that globally minimizes the distance between two surfaces by translation and rotation. More specifically, 3 landmark points are automatically detected on each humerus. Two surfaces are aligned in a least squares sense by computing the best fit mapping between the two sets of landmarks. Processing circuitry may thirdly perform (3) non-Rigid registration, which is a technique for local distance minimization by surface deformation. For example, processing circuitry 102 may implement a hierarchical B-Splines registration technique that uses surface landmark points to locally approach two surfaces. Based on the techniques described above for anatomical landmark detection, as many as 85 landmarks can be automatically detected on the surface (see e.g., FIGS. 17B and 17C) and inside the surface (see e.g., FIG. 17D) of humerus. In addition to these points, processing circuitry 102 can establish zone-to-zone correspondences between the previously defined proximal humerus zones (humeral head, greater, and lesser tuberosities) that can be used to detect landmark points on these zones. This number of landmark points may be used for a sufficient and reliable surface deformation, but other numbers of landmarks may also be used.

FIGS. 17A-17D show examples of landmarks for rigid and non-rigid registrations. FIG. 17A shows an example of landmarks for rigid registration. FIG. 17B shows an example of surface landmarks for non-rigid registration. FIG. 17C shows an example of surface landmarks for non-rigid registration. FIG. 17D shows an example of non surface landmarks for non-rigid registration.

Once the B-Splines registration are applied and two meshes are as close as possible, processing circuitry 102 may use the minimum distance criteria to find point-to-point correspondences. At this stage, processing circuitry 102 is able to compute a mean shape and to apply Principal Component Analysis (PCA) algorithm to determine principal modes of variation.

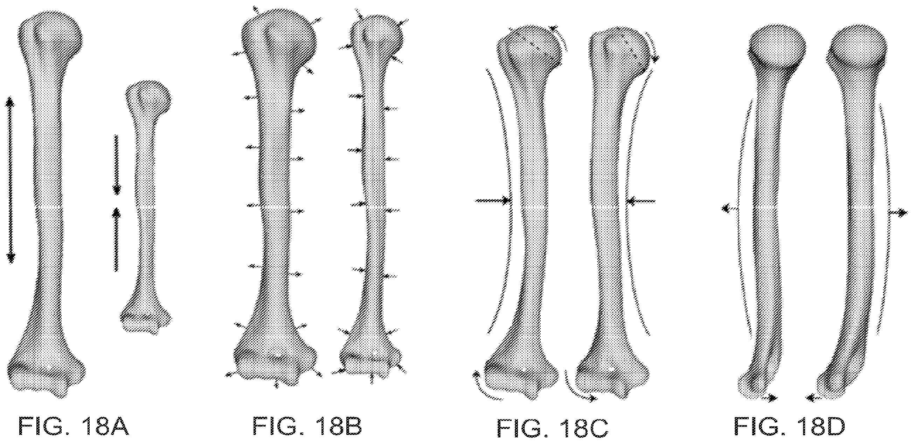

FIG. 18A shows a 1st principal mode of variation as a scale factor of the mean shape. The humerus length increases when the mode value is decreasing, and contrariwise the humeral length decreases when the mode value is increasing.

FIG. 18B shows an impact of a 2nd principal mode on the mean shape. This mode represents a variation that consists in a uniform expansion when the mode value is decreased and a uniform shrinking when the value increases.

FIG. 18C demonstrates an impact of a 3rd mode on the mean shape. It can be observed that this mode has a high impact onto the diaphysis curvature in the coronal plane and the humeral head varus/valgus. This third mode also produces a modified position of the elbow capitulum and the medial epicondyle.

FIG. 18D shows a variation of a 4th mode on the mean shape. It can be seen that modifying the mode value changes the diaphysis curvature in the sagittal plane. The 4th mode also has a slight influence on the humeral head and the elbow positions.

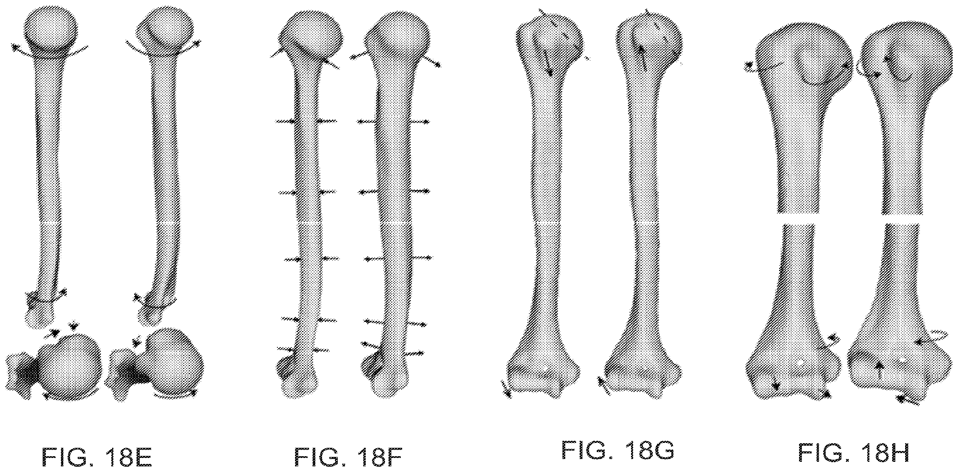

FIG. 18E shows an impact of a 5th mode on the mean shape. It can be observed that the mode controls a humeral head retroversion by turning the humeral head in one direction and the transepicondylar axis into the another one. The 5th mode cause the positions of the lesser and greater tuberosities to rotate in the same direction with the humeral head.

FIG. 18F presents a variation of a 6th mode on the mean shape. It can be observed that the decreasing the mode value only shrinks the humeral diaphysis and consequently increasing the mode value dilates the diaphysis. This mode is a bit similar to the 2nd mode; the difference lies in the intensity of influence on different bone zones. It can be seen that the current mode has a high influence on the diaphysis and a very light influence on the head and elbow, while the 2nd mode has a uniform impact on the entire humerus.

FIG. 18G demonstrates an influence of a 7th mode, where the principal variations consist in humeral head varus/valgus changes and tuberosities and capitulum displacements.

FIG. 18H demonstrates a variation of an 8th mode. It can be seen that this mode considerably changes the position and shape of the capitulum, as well as the position, shape and orientation of the humerus trochlea. In addition, this variation of the 8th mode has an influence on the positions of the lateral and medial epicondyles. The 8th mode further has a strong impact on the proximal humerus shape and more specifically on the both tuberosities. When the shape parameter decreases the distance between tuberosities increases and vice-versa. A change in the tuberosities' positions also impact the shape of the bicipital groove.

The above examples describe influences of the first 8 principal modes of variations on the mean shape. By minimizing the square distances between the correspondences, processing circuitry 102 can minimize the compactness of the SSM. But even with the minimized value of the compactness, the 8th mode has a great impact on the mean shape. Thus, in order to correctly represent all the humeri variations, processing circuitry 102 may be configured to use more than 8 modes.

To determine proximal humerus points/zones that are correlated with the humerus retroversion, processing circuitry 102 may be configured to: (1) create N humerus instances Sn based on the humerus SSM; (2) for each Sn, compute the diaphysis central axis ($A_{diaphysis}$) and the transepicondylar axis ($A_{transepicondylar}$) and retroversion angle ($theta_n$); (3) project each humerus 3D surface Sn point pi onto the $A_{diaphysis}$ axis; (4) for each projected point, determine a vector $V_i = p_i - p_{projected}$ I; and, (5) for each vector $V_i$, compute an angle $\alpha i = \angle(V_i, A_{transepicandylar})$.

After processing all the N generated humeri, processing circuitry 102 can determine the angle values $\alpha_i$ for each humerus and then apply a linear regression analysis to determine any potential correlation between the humerus retroversion angle and the angles $\alpha_i$ which are computed for all humerus vertices.

The obtained results reveal several zones on the proximal humerus that are strongly correlated with humerus retroversion. These zones that can be used as reliable landmarks include the bicipital groove, the lesser tuberosity, the edge of the intertubercular sulcus, and also the anterior face of the humeral proximal diaphysis.

Processing circuitry 102 may also be configured to determine articular surface diameter correlation. Based on regression analysis, processing circuitry 102 can determine the equation of the regression line as follows:

$$y = 0.825 \times x - 0.37 \qquad\qquad (3.6)$$

where x is the articular surface radius and y is the articular surface thickness.

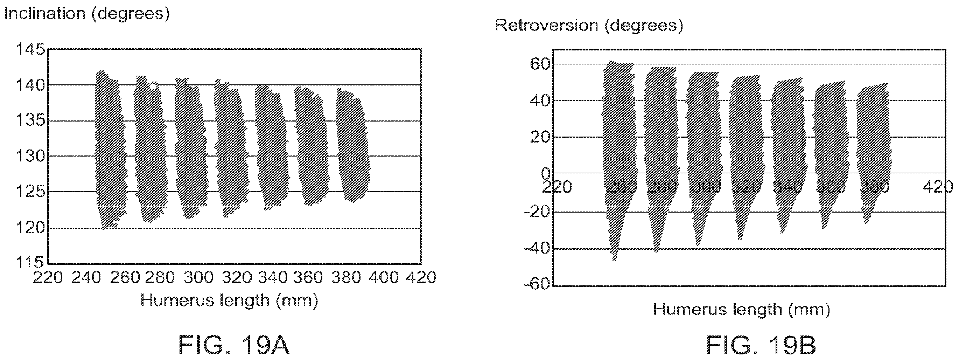

It has been determined that humerus length has a correlation to the retroversion/Inclination variations. FIGS. 19A and 19B show the humeral length impact on the retroversion and inclination variations, with small humeral length corresponding to higher retroversion and inclination variation and a longer humeral length value corresponding to decreasing retroversion and inclination variations. Accordingly, processing circuitry 102 may be configured to account for object scale with SSM.

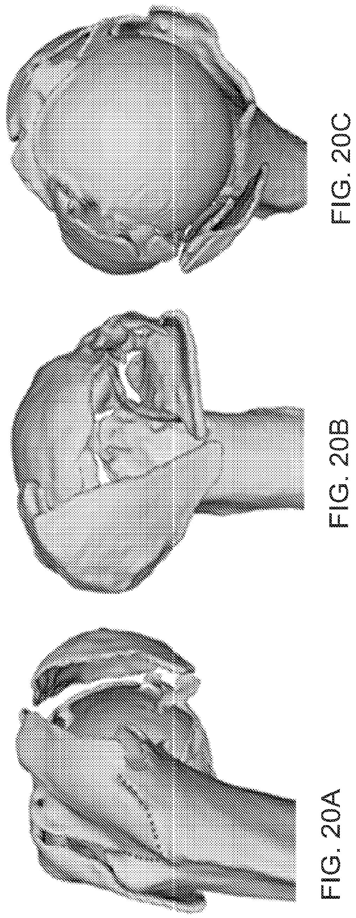

Utilizing the above-described tools, processing circuitry 102 may be configured to perform fracture fragment processing. For proximal humerus fractures, CT-Scan images often show some of the broken fragments as being fused together. There are generally three types of fragment fusion: (1) fusion of class 1 - a bone that is not broken but just bent (e.g., FIG. 20A); a fusion of class 2 - a physical displacement of a fractured fragment that hits into another fragment (e.g., FIG. 20B); a fusion of class 3 - an error during automatic segmentation caused by a narrow inter-bone spacing (e.g., FIG. 20C).

FIGS. 20A-20C illustrate the different processes that cause broken fragment fusion. FIG. 20A shows a fragment where a broken piece is bent compared to the diaphysis. FIG. 20B shows a fragment where two fractured parts moved until collision. FIG. 20C shows a fragment where fusion between broken fragments caused by segmentation error.

In order to be able to manipulate broken fragments and to restore the original patient anatomy, processing circuitry 102 may configured to separate all fused fragments. It has been discovered that any fracture line contains region(s) with a high absolute minimal curvature value. The minimal curvature significantly varies along the fracture lines and often happens to have regions with very low absolute curvature value. Furthermore, it has been observed that proximal humerus possesses a lot of intact bone regions with a moderate absolute minimal curvature value. These conditions make it difficult to separate fused fragments using thresholding alone. To solve the separation problem, processing circuitry 102 may be configured to perform step segmentation.

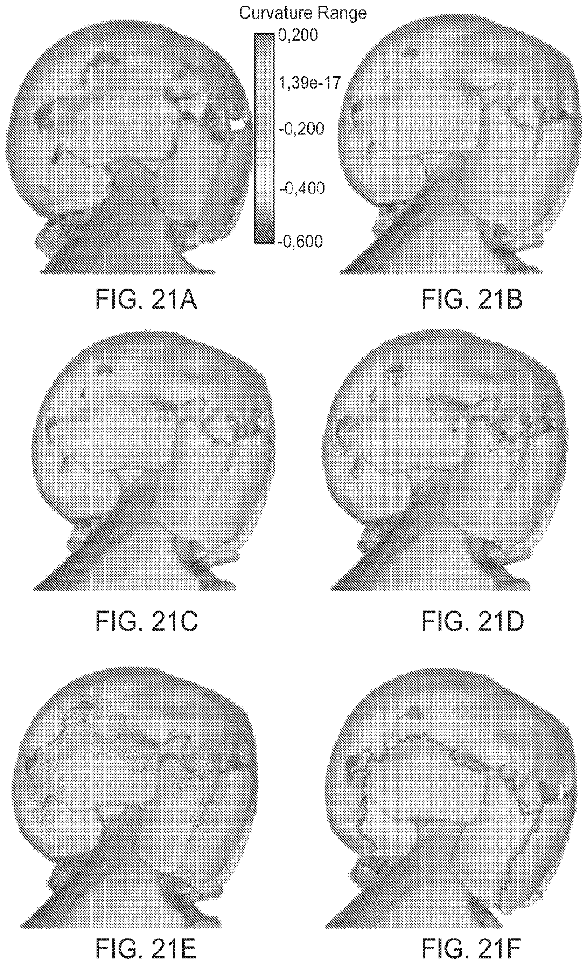

The idea behind the step segmentation algorithm is to gradually propagate a segmentation process. During each step, a segmentation process starts in a specific region of interest that guides the global segmentation. FIGS. 21A-21F illustrate the principle of the algorithm and show three steps of the proposed idea applied on a broken proximal humerus. FIG. 21A shows the minimal curvature. FIG. 21B shows the regions initialization by curvature thresholding. FIG. 21C shows next step propagation points. FIG. 21D shows, after the first propagation step, the next propagation points that are recomputed. FIG. 21E shows after the second propagation step, and FIG. 21F shows the skeleton of the obtained regions after four iterations.

The minimal surface curvature has been chosen as a fracture line descriptor (FIG. 21A). Along any fracture line, it is possible to find region(s) with a "high" absolute minimal curvature value. This value is problem-specific and must be chosen experimentally. A value of "0.3" can be considered as a "high" absolute minimal curvature value. Hence, to initialize the regions for the step segmentation algorithm, processing circuitry 102 may be configured to select all vertices with the minimal curvature value inferior to -0.3 (FIG. 21B).

The next step is the propagation source points detection. Processing circuitry 102 is configured to detect boundary contours for the previously defined regions and apply the discrete curve evolution algorithm (FIG. 21C). Then, processing circuitry 102 uses each source point as a center for a wave propagation based on a fast marching method. Processing circuitry 102 uses the absolute minimal curvature value as a speed function for the wave propagation which implies a faster propagation in regions with high curvature and consequently slower propagation in regions with low curvature value. A propagation distance is limited to a certain value that permits to avoid a propagation in wrong directions caused by image noise. The obtained result after the first propagation steps is shown in FIG. 21D.

Once the propagation process is stopped, processing circuitry 102 combines the obtained points with the initial regions to create an input for the next iteration. Thus, for each new region, processing circuitry 102 recomputes the boundary contours and source points (some, but not all, of which are labeled as points in FIG. 21D) and re-applies the Fast Marching Method for further propagation. This is an iterative algorithm where a number of iterations is a problem-defined value.

An example of propagated regions after two iterations is shown in FIG. 21E. For proximal humerus broken fragments separation, processing circuitry 102 may, for example, be configured to use four iterations. FIG. 21F demonstrates the obtained broken lines that are the skeletons of the final regions.

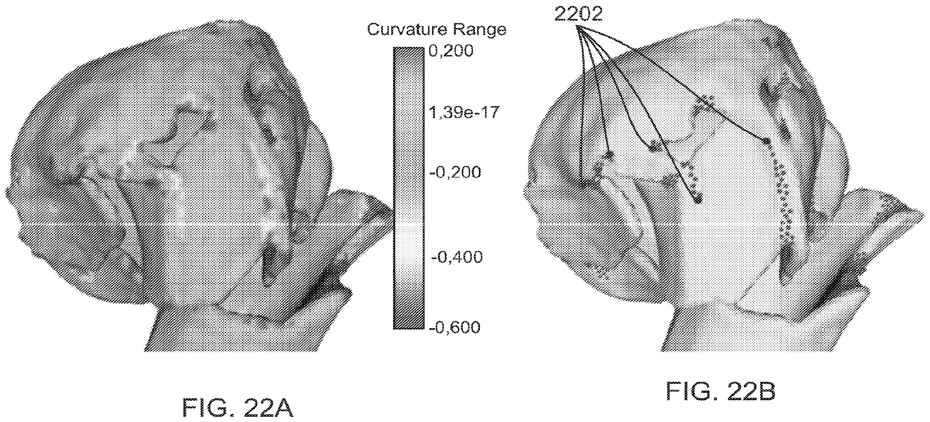

Processing circuitry 102 may be configured to perform step propagation landmark detection. One "key to success" of step segmentation algorithm is correct and robust step propagation landmark detection. In order to correctly detect these points, processing circuitry 102 may be configured to define which points are important for the propagation process. FIGS. 22A and 22B show 3D images of a proximal humerus fracture where a surgeon manually selected all vertices that belong to fracture lines. It can be observed that selected vertices form elongated lines that may form closed cycles.

As discussed above, the surface minimal curvature has been chosen as a fracture line descriptor (see e.g., FIG. 21A). Along any fracture line, it is possible to find region(s) with a "high" absolute minimal curvature value. The term "high" may be a problem-specific value that can be defined experimentally. Such regions follow the fracture lines and, therefore, take the elongated shape. In order to detect the rest of the fracture lines, processing circuitry 102 may be configured to propagate the waves from the salient (ending) points of the defined regions. FIG. 22A shows an example of curvature along a humeral head, with the top of the scale representing minimal curvature and the bottom representing higher curvature. Points 2202 in FIG. 22B correspond to examples of potentially optimal points for a propagation process for detecting fracture lines.

Processing circuitry 102 may also execute a discrete curve evolution (DCE) algorithm to compute the previously described points. A DCE algorithm serves to eliminate an objects' contour distortions while at the same time preserving the perceptual appearance at a level sufficient for object recognition. Essentially, this technique eliminates less important contour vertices (often vertices affected by noise) and keeps vertices that are significant for contour description. This technique is widely used for a shape similarity measure, skeleton pruning, and salient point detection of binary objects on a regular 2D grid.

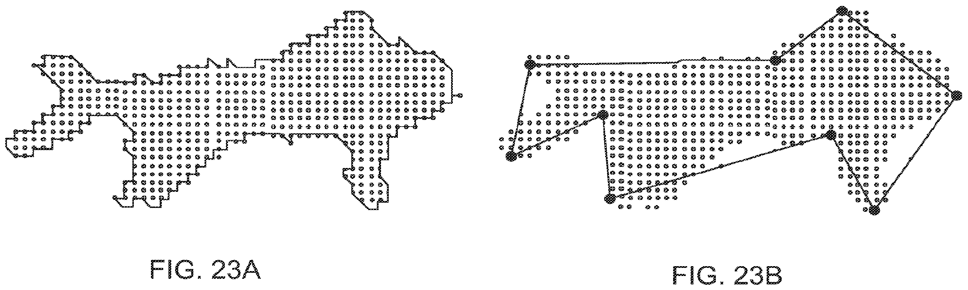

An application of the DCE algorithm on a simple 2D fish shape is shown in FIGS. 23A and 23B, where a shape contour is perturbed with noise. FIG. 23B demonstrates the obtained results where the contour's most significant points are shown in blue. After observing these results, it can be concluded that this algorithm can be used for detecting the step propagation points.

The basic idea of the DCE technique will now be introduced. For each evolution step, a contour point with the smallest relevance measure is removed, and two neighbor points become connected and form a contour's line segment. The relevance measure K (equation 4.1) can be determined based on Euclidean distances and angles as follows:

$$K(s1, s2) = \frac{\beta(s1, s2)\, l(s1)\, l(s2)}{l(s1) + l(s2)}$$

$$(4.1)$$

where $\beta$ is the turn unsigned angle at the common vertex of two contour's segments s1 and s2 and 1(.) is the length function, normalized with respect to the total length of a polygonal curve. The main property of this measure is that higher the value of K(s1; s2), the larger is the contribution of the arc s1 U s2 to the shape.

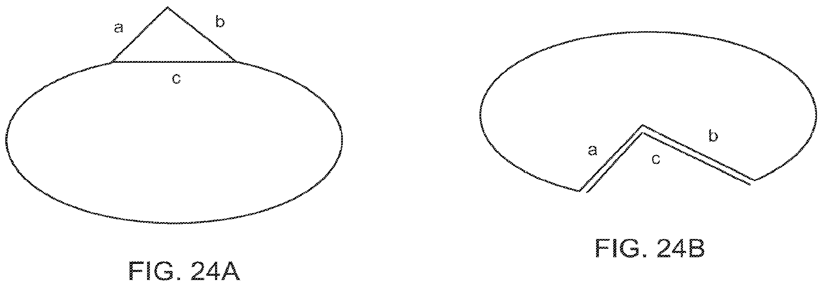

FIGS. 24A and 24B shows two types of contour topology. FIG. 24A shows a spike, and FIG. 24B shows a cavity. Processing circuitry 102 may also be configured to perform step propagation landmark detection. Processing circuitry 102 may be configured to implement a new cost function that can be easily computed on a triangulated surface and yields similar results on a regular 2D grid in comparison to the classical DCE. To apply a DCE algorithm to a contour formed by vertices on a triangulated surface, processing circuitry may be configured implement the same techniques as for contours on a regular 2D grid. The difference consists in a new relevance measure that can be computed on a 3D surface. The vertex relevance measure is directly proportional to the sum of segment lengths connected to the analyzed vertex and is inversely proportional to the distance between non-connected vertices of these segments.

$$K = \frac{a + b}{c}$$

$$(4.2)$$

where *a, b,* and *c* are shown in FIG. 24A.

To find a distance c, processing circuitry 102 may be configured to search for the shortest path between contour vertices only inside an object that defines the studied contour (e.g., FIG. 24A). When a vertex is found in a concave region, the distance c is equal to the sum of segments that form the cavity (e.g., FIG. 2413).

$$c = a + b$$

$$(4.3)$$

The last condition does not distinguish between "concave" vertices. Take for example, the following two different concave regions:

    1. *a* = 10 *cm, b* = 20 *cm* and *c* = 30*cm*
    2. *a* = 1 *cm, b* = 0.5 *cm* and *c* = 1.5 *cm*

The relevance measure (equation 4.2) may, for example, be 1 for both cases, but the segment in the first case is bigger, and thus more significant. In order to distinguish between various concave segments, processing circuitry 102 may be configured to determine a size coefficient:

$$C_{size} = \frac{a+b}{\sqrt[3]{c}}$$

    (4.4)

For the above example, the size coefficient equals to 4.48 for the first case and 1.22 for the second case. Thus, processing circuitry 102 can distinguish between such concave regions. This relevance measure may be applied to arbitrary triangulated mesh and is equal to the multiplication of the cost function in equation 4.2 by the size coefficient:

$$K_{final} = \frac{(a+b)^2}{c \times \sqrt[3]{c}}$$

    (4.5)

As previously described, fracture lines can be characterized with the minimal curvature. In order to separate the fused bone fragments, processing circuitry 102 may be configured to use the minimal curvature as a surface descriptor. The minimal curvature value significantly varies along the fracture lines, and this variation depends on an angle between fused fragments, DICOM image quality, and subsequent segmentation. For almost any fracture line, one or several zones with a low minimal curvature value are identifiable. The term "low minimal curvature" is application-dependent and may be defined for each specific problem. These "low curvature" zones form elongated shapes and follow the fracture line. Other humerus zones whose minimal curvature value varies in between the "low" value and 0 might belong to the fracture lines or belong to the bicipital groove or to be just a part of an intact bone surface.

Processing circuitry 102 may be configured to implement the following segmentation algorithm for step segmentation. This algorithm is an iterative process where during each iteration (step) a wave propagates in a specific direction that permits to segment zones with very weak intensities. The principal steps of this algorithm are as follows:

    1. Region Initialization:

- For each vertex $V_i$ on the mesh $M$, compute the minimal curvature value $C_i^{cmin}$ ;
- Create a set of vertices R:

$$IF\ (C_i^{cmin} < -0.3)$$
$$R_{init} \leftarrow V_i$$

    2. Region labeling:

- Separate non-connected vertex sets in $R_{init}$: $R_j \leftarrow R_{init}$, $j \in M$, $M$ is a number of the detected non-connected vertex sets;

    3. Boundary Vertex detection:

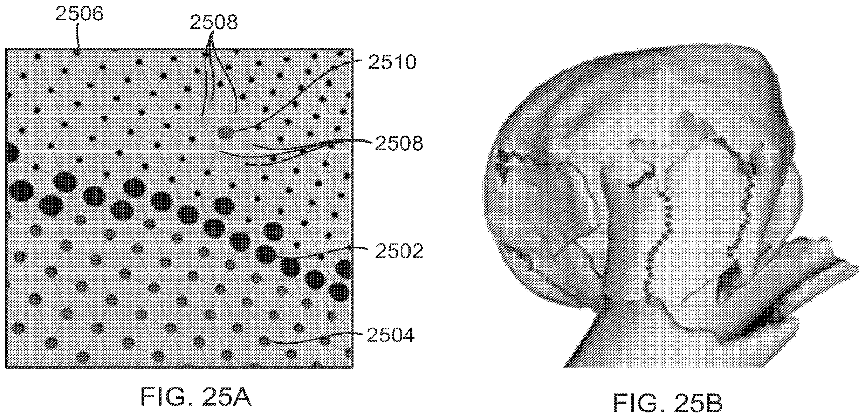

- For each Rj. : j ∈ M , detect boundary contour Bj (e.g., points 2502 in FIG. 25A);
- Boundary contour is an ordered list of topologicalely connected boundary vertices. A vertex is classified as a boundary vertex if in its neighborhood simultaneously presents vertices from the foreground (e.g., points 2504, FIG. 25A) and from the background (e.g., points 2506, FIG. 25A);
- the neighborhood of a vertex *v* is all the edge-connected vertices to v (e.g., vertices 2508 are the neighborhood of the vertex 2510 in FIG. 25A).

    4. Source point computing:

- For each detected boundary contour $B_j$, apply the DCE algorithm described above with respect to step propagation landmark detection in order to compute the step propagation points $P_k^j$, $k \in K_j$, $j \in M$, $K_j$ is the number of detected source points for a contour $B_j$;

5. Surface speed computing:

- For each vertex Vi define a speed function as an absolute minimal curvature value.

6. Propagation:

- For each $p_k^j$ source point, apply a Fast Marching Method. The defined surface speed (step 5) implies a faster wave propagation in the regions with the higher curvature value and as a consequence slower propagation into the regions with low curvature.

- The propagation is limited in terms of geodesic distance. If the farthest contour wave reaches the limit distance - the propagation is stopped. This distance is a problem-specific value.

- To choose the best limit for a wave propagation distance, a range from 3mm to 10mm with a step of 1mm was tested. Any distance smaller than 3mm may not permit the algorithm to advance in a specific direction (direction that is determined with the surface speed function). Any propagation distance higher than 10mm is likely to propagate in the noise regions. For every test, the RMS distance between the manually selected fracture lines and the liens detected by the step segmentation algorithm were computed. The distance limit of 5mm produced the best outcomes.

7. Stop criterion:

- When the limit distance is reached for all source points $P_k^j$ → add the propagated points to the point set in step 2.

- Go to step 2.

By iterating from step 2 to step 7 the segmentation process moves forward. The iteration number is an important parameter and may be application dependent. Increasing the number of iterations typically increases the number of detected regions, but these regions do not necessarily belong to fracture lines. A large number of iterations might detect a lot of noisy regions which are not desirable for the segmentation rest-tits.

The number of iterations may be a problem-specific value. For the broken fragments separation problem, processing circuitry 102 may be configured to perform 3 iterations to achieve desired results, but other numbers of iterations may also be used.

Processing circuitry 102 may also be configured to perform fracture line detection. Processing circuitry 102 may compute the fracture lines for a fractured proximal humerus by applying a skeleton extraction to the regions detected using step segmentation as described above. The problem of skeleton extraction for sets of vertices on arbitrary triangulated 3D mesh is not a trivial task. One solution is a method where a sequence of mathematical morphology operations are applied to a set of vertices on a tri-angulated surface. The described technique is efficient and simple to implement but has several drawbacks that may lead to disconnected skeleton lines. To overcome this issue, processing circuitry 102 may be configured to include an additional definition of vertex categories to improve the thinning process by integrating a priority between vertex classes. This method has been tested on different categories of meshes (homogeneous and heterogeneous) and vertex set configurations. The obtained results illustrate the high accuracy and the efficiency of the approach.

As has been explained above, for proximal humerus fractures, CT-Scan images often show some of the broken fragments as being fused together. To address this challenge, processing circuitry 102 may be configured to characterize fracture lines based on a minimal curvature. However, the minimal curvature significantly varies along the fracture lines and often happens to observe regions with low absolute curvature value along the fracture lines. Moreover, proximal humerus possesses lot of intact bone regions with a moderate absolute minimal curvature value. Thus, processing circuitry 102 may be configured to implement the step segmentation algorithm described above. The principle of the step segmentation algorithm is a gradual propagation process. During each iteration a segmentation process starts in a specific region of interests that guides the global segmentation/propagation. Such regions of interest are called "source points" and are computed using the discrete curve evolution algorithm.

Using the tools introduced above, processing circuitry 102 may be configured to determine procedures for fracture reduction. Fracture reduction is a surgical procedure to restore a fracture or dislocation to the correct alignment. In this context, the term "reduction" does not necessarily imply any sort of removal or quantitative decrease but rather implies a restoration, or "bringing back to normal." In bone fractures, the fragments lose their alignment in term of displacement and/or angulation. For the fractured bone to heal without any deformity the bony fragments must be realigned to their normal anatomical position. Orthopedic surgery attempts to restore the normal anatomy or function of the fractured bone by reduction of the displacement.

There are several techniques for proximal humerus fracture management, including non-operative treatment, closed reduction, open reduction and internal fixation, shoulder hemiarthroplasty, and reverse total shoulder arthroplasty. For all these techniques, except the non-operative treatment, the original anatomy (before the fracture) is important information for a surgeon to have in order to obtain satisfactory outcomes.

Techniques for reducing proximal humerus fracture and finding the original anatomy include manual broken fragment reduction, haptic-based approach for proximal fracture reduction, broken fragments reduction based on the intact contralateral proximal humerus shape, and broken fragments reduction based on the statistical anatomical atlas model.

Processing circuitry 102 may be configured to perform humerus SSM fitting onto the broken diaphysis fragment. The objective of coarse registration is to align the available broken fragments up to the level where the similarity of their borders can be captured, which in turn permits a fine registration based on an ICP algorithm. Processing circuitry 102 may be configured to perform coarse proximal humerus fragment registration by using a humerus SSM, where the SSM acts as a template for the broken fragment placement. However, as described above, the humerus shape has a significant variation across the population, such that a simple mean shape cannot be used as a reliable template. Processing circuitry 102 may be configured to implement a step algorithm, where for each step SSM incorporates additional information about the fracture. Such improves the SSM shape approximation to patient's native proximal humerus shape.

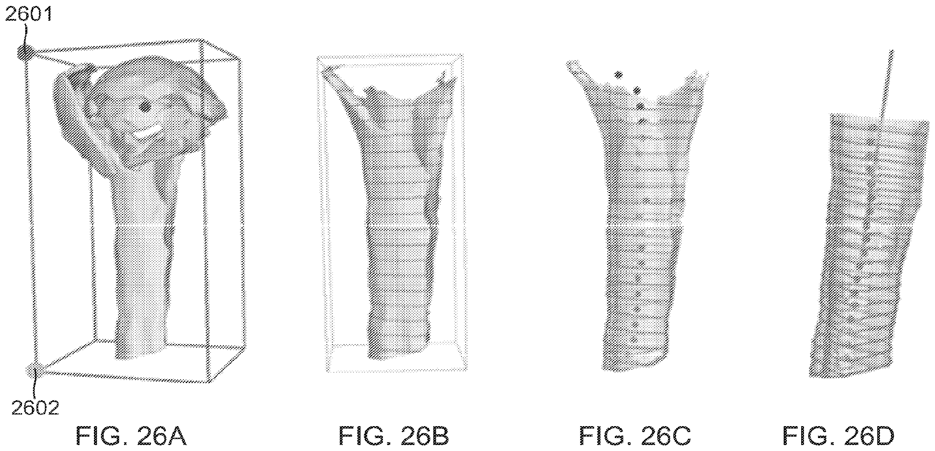

Processing circuitry 102 may be configured to diaphysis detection and cleaning. FIGS. 26A-26D show a process for diaphysis detection and characterization. FIG. 26A shows an example of a diaphysis oriented bounding box (OBB). FIG. 26B shows an example of diaphysis broken part removal. FIG. 26C shows an example of a diaphysis central axis point. FIG. 26D shows an example of a diaphysis without broken part central axis computing.

Processing circuitry 102 begins the reduction process by determining the diaphysis fragment among the broken fragments. To do so, processing circuitry 102 computes an oriented bounding box (OBB) for the entire broken humerus and determines the closest and the farthest corner to the humerus rotation center based on the Euclidean distance (points 2601 and 2602 in FIG. 26A). By iterating over the detected fragments, processing circuitry 102 determines the diaphysis as a fragment which has the closest vertex to an OBB farthest point.

Processing circuitry 102 is also configured to perform diaphysis characterization. Processing circuitry 102 characterizes the diaphysis using the central axis which always points toward the humeral head. As will be described in more detail below, a correct computation of this vector requires a diaphysis portion without the fracture contours. Hence, a procedure for a robust broken diaphysis central axis computing consists of two steps: (1) fracture contour removal and (2) diaphysis central axis computation.

Processing circuitry 102 may be configured to perform fractured contour removal, during which the fractured diaphysis part is cut off. Very often, a complex proximal humerus fracture occurs on a metaphyseal level. To detect the metaphyseal level, a method that detects the difference of diaphysis radii may be used. To do so, processing circuitry 102 computes the OBB around the broken diaphysis fragment and cuts this diaphysis mesh by planes perpendicular to the longest OBB's vector. Processing circuitry 102 starts by the farthest OBB point and moves in the direction of the closest point, (see e.g., FIG. 26B). For each cut, processing circuitry 102 determines a radius of a fitted circle and compares that radius to the previous cut. If the radius value has been increased by 10% or more, processing circuitry 102 stops the cutting process as the metaphyseal level has been achieved.

Processing circuitry 102 performs diaphysis central axis computing in two steps. First, processing circuitry 102 estimates an initial central axis by cutting the diaphysis without broken edges by planes perpendicular to the longest OBB vector axis. For each cut, processing circuitry 102 computes a gravity center for intersection points between a cutting plane and diaphysis mesh. Processing circuitry uses the obtained gravity centers to fit a line in least mean square sense that is the first diaphysis central axis estimations (FIG. 26D).

Processing circuitry 102 computes a final central axis by reapplying the previous procedure, but this time processing circuitry 102 cuts the diaphysis by planes that are perpendicular to the previously estimated central axis.

To summarize, the broken diaphysis is cut by planes perpendicular to a longest OBB axis, and for each cut, a gravity center is computed. To determine the central axis, processing circuitry 102 fits a line into the detected gravity centers in the least square sense. As can be seen in FIG. 26C, central points at the fracture contour level deviate from the central axis, which is why processing circuitry 102 may be configured to remove diaphysis surface portions higher than metaphysis level.

Processing circuitry 102 may be configured to perform internal surface removal. For a correct broken fragment reconstruction, during the CT-Scan segmentation, processing circuitry 102 may be configured to detect both external and internal surfaces of the cortical bone. For broken fragment reduction and original humerus shape prediction, processing circuitry 102 may only need the external cortical shape. In order to detect the internal cortical bone, each diaphysis triangle is tested for its normal orientation. This test processes in three steps: (1) the triangle gravity center is projected onto the diaphysis central axis; (2) the direction to the diaphysis central axis is determined as a vector from the triangle gravity center to its projection; and (3) if the dot product of triangle normal with the direction to the central axis is positive, the triangle is marked as a part of the internal cortical surface. To extract only the external cortical shape, all triangles marked as internal surface may be removed.

Processing circuitry 102 may be configured to perform coarse fitting. As discussed above, humerus length and diaphysis diameter have significant variation across the population but can still be modeled using a humerus SSM. Each humerus variation (length, retroversion, inclination ...) can be controlled by a specific principal mode of variation. Accordingly, processing circuitry 102 can initialize the shape of the SSM by adapting the proximal diaphysis radius to the radius of the broken diaphysis fragment.

Processing circuitry 102 can: (1) determine a proximal diaphysis (without metaphysis) extraction as described in the previous section; (2) determine an extracted surface cutting by planes perpendicular to diaphysis central axis; (3) for each cut, fit a circle into the plane-surface intersection points; and (4) determine a proximal diaphysis radius as the mean value of fit radii.

The first and the second modes of variation have a significant impact on the proximal diaphysis radius. Processing circuitry 102 may be configured to apply a nonlinear simplex optimization that minimizes the difference between humerus SSM diaphysis radius and broken diaphysis radius by varying the first two principal modes of variation. Such optimization estimates the original shape diaphysis radius which is important for further pose refinement. This is the first basic approximation of the original bone shape. As humerus SSM diaphysis radius is roughly equal to the broken one, processing circuitry 102 can approximately detect the metaphysis zones on the same levels for both diaphyses. Processing circuitry 102 uses a point on the diaphysis central axis at the metaphyseal level and the axis itself to compute a rigid transformation between the broken diaphysis and humerus SSM. The resulting transformation enables initializing the pose of the fractured diaphysis relatively to the humerus SSM.

Processing circuitry 102 can be configured to perform translation and rotation of broken diaphysis along and around the central axis. Based on the previously described initialization, processing circuitry 102 obtains a broken diaphysis position on humerus SSM. This position is a first approximation of the real position and therefore needs to be improved. There are two parameters that can be adjusted in order to determine a correct broken diaphysis position: vertical translation along the central axis and rotation around the central axis.

The previously described initialization is an accurate technique to determine the height of the broken diaphysis. However, this initialization may introduce an error, and thus existing techniques may benefit from improved determination of the broken diaphysis height. This disclosure introduces an algorithm to potentially improve the height determination for a broken diaphysis. The algorithm includes translating up and down the broken diaphysis fragment along its central axis. For each translation, processing circuitry 102 computes the RMS distance between the diaphysis fragment and humerus SSM. Processing circuitry 102 searches a translation which minimizes the RMS distance.

After the initial alignment and the vertical translation, processing circuitry 102 obtains a very precise vertical positioning of the broken diaphysis but does not know anything about its orientation around the central axis. Often, diaphysis is modeled as a cylinder that makes impossible to find a correct orientation. Fortunately, proximal diaphysis contains the bicipital groove and two ridges that descend from the greater and lesser tuberosities. Such a shape deformation permits processing circuitry 102 to determine a correct broken diaphysis orientation relative to humerus SSM. The algorithm is based on a broken diaphysis rotation around its central axis. For each turn, the RMS distance between broken diaphysis and SSM is computed. The best orientation corresponds to the minimal RMS distance.

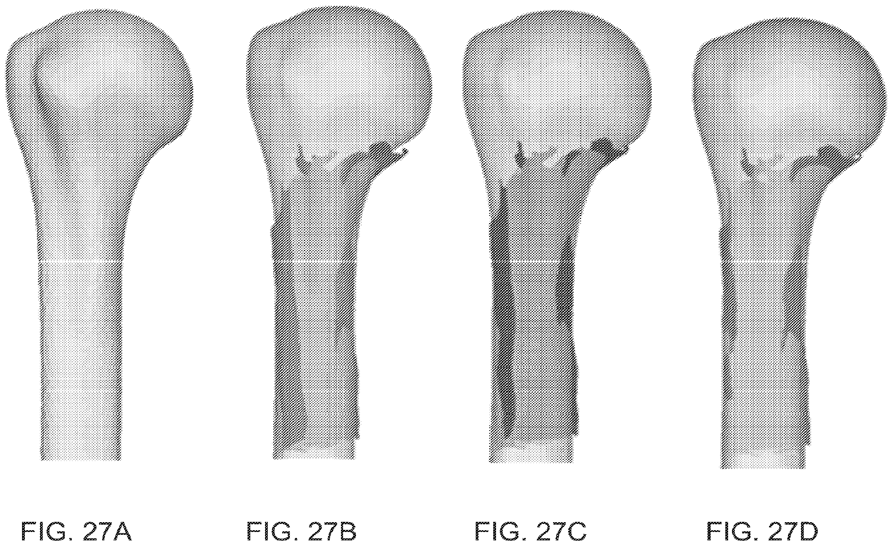

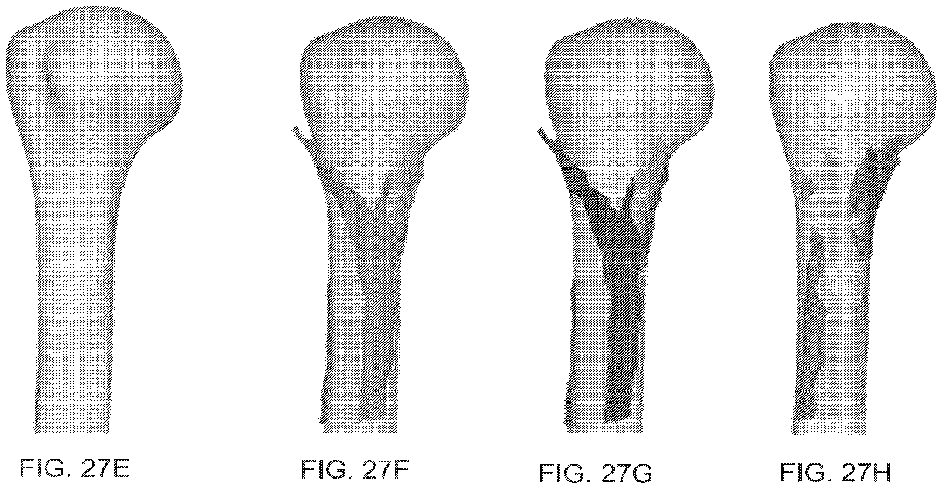

FIGS. 27A-27D represent a first example of initial alignment of the broken diaphysis with humerus SSM, and FIGS. 27-27H represent a second example of initial alignment of the broken diaphysis with humerus SSM. FIGS. 27A-27D show a patient with a proximal diaphysis radius smaller than the diaphysis radius of the SSM. FIG. 27F demonstrates the output of the simplex optimization, where the SSM size has been decreased. Initial translation and orientation were computed very accurately. Hence, translation and rotation optimizations adjusted the initial position only by 2mm and 3 degrees respectively.

FIGS. 27E-27H show a patient that has a similar diaphysis radius with the humerus SSM. The simplex optimization does not almost change the size of the humerus. Initial vertical alignment was accurately estimated and the vertical adjustment procedure did not change the height of the broken diaphysis. However, FIG. 27G shows the wrong broken diaphysis orientation. The optimization process rotated the broken diaphysis by 68 degrees and on FIG. 27H shows a correctly oriented broken diaphysis.

Processing circuitry 102 is configured to fit as good as possible of a humerus SSM onto the available portion of the broken diaphysis. Processing circuitry 102 is configured to perform shape optimization. More specifically, processing circuitry 102 applies the simplex minimization algorithm that seeks to find a combination of principal modes of variation that minimizes the RMS distance between the broken diaphysis and SSM.

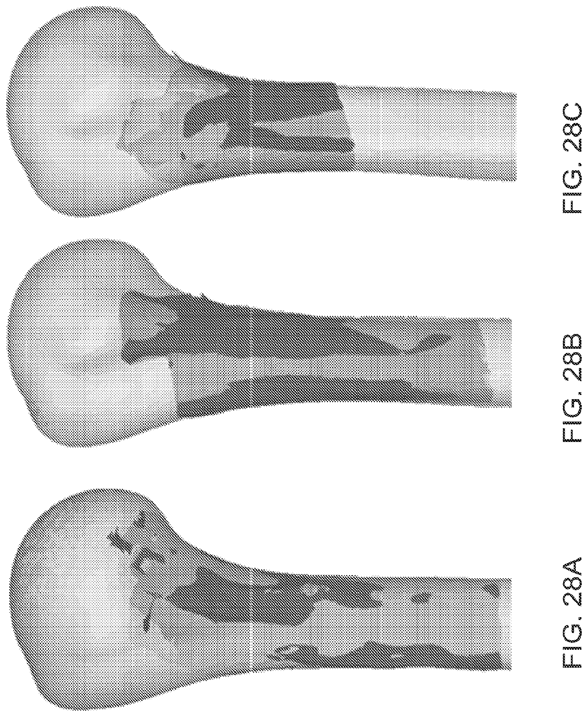

FIGS. 28A-28C show examples of broken humerus shape estimation based on diaphysis shape. FIGS. 28A-28C show an application of the previously described algorithm on three different patients. Visual observation of the obtained results demonstrates that the estimated model shapes are very close to the available diaphysis portions. The obtained results also show that the algorithm with the same accuracy can process diaphyses of different lengths. Examples of humerus shape estimation with a long portion of available diaphysis shown in FIG. 28A and 28B. FIG. 28C shows an example of a predicted humerus shape based on a short diaphysis portion.

The humeral head possesses one anatomical and one geometric peculiarity that enables and/or necessitates the creation of a specific algorithm for its reduction. First, the humeral head is the only fragment that is not attached to any muscle. This anatomical peculiarity enables humerus head displacement in any directions and over long distances in case of proximal humerus fracture. Second, the humeral head surface can be accurately approximated with a sphere shape. This geometric peculiarity enables processing circuitry 102 to compute the humeral head center, and register that humeral head center to the humerus SSM head center.

Using the geometric properties of the spherical humeral head, processing circuitry 102 may implement the following algorithm to register the broken humeral head to the humerus SSM atlas.

- Broken humeral head detection:

  - Determine broken humeral head center $C_{broken}$ as the voxel intersected by the largest number of surface normals as described above;
  - Determine the humeral head among the broken fragments Fj, j $\in$ M, M

    • number of detected broken fragments;

    • For each fragment Fj fit a sphere in the least square sense and store the sphere center Cj;
    • For each Cj compute the Euclidean distance Dj = dist(Cj, Cbroken);

  • The minimal distance Dmin = minjEM {Dj} corresponds to the broken humeral head fragment FBrokenHead ← Fj, j is the index that corresponds to the distance Dmin. CBrokenHead corresponds to the detected broken head center;

- Humerus SSM head center detection:

  - Manually selected humeral head vertex IDs on the mean shape enable their locations on the humerus SSM for any principal modes configuration to be known;
  • Sphere fitting to these points permits the humerus SSM head center Chssm to be computed.

- Broken humeral head centers alignment:

  • Define a transformation $T_{BrokenHead}$ as a translation vector $V_{tr} = C_{hssm}\ C_{BrokenHead}$;
  • Apply the transformation $T_{BrokenHead}$ to the broken head fragment: $F_{BrokenHeadAtHSSM} \leftarrow Tr(F_{Broken\ Head}.\ T_{Broken\text{-}Head})$;

- Broken humeral head reduction:

  - After the previous head centers alignment the orientation of the broken humeral head might be incorrect. To find the correct orientation, processing circuitry 102 applies the simplex optimization algorithm. By the mean of broken humeral head rotations around its rotational center, the optimization process minimizes the RMS distance between broken humeral head and the humerus SSM surface.

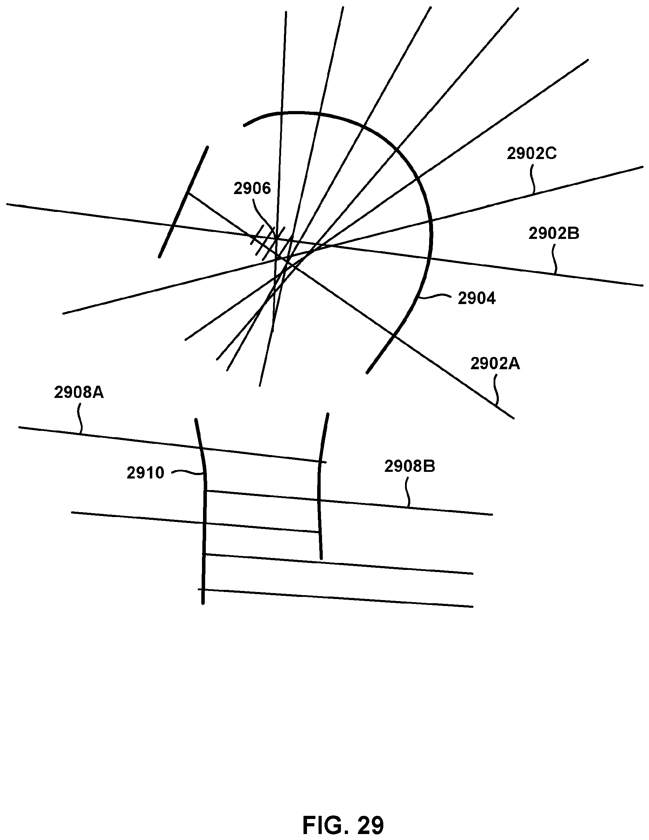

Examples of the humeral head reduction are shown in FIGS. 28A-28D and 29A-29D. The broken humeral head fragment fits perfectly into the predicted humerus shape. The obtained visual results demonstrate a correct behavior of the described broken head reduction algorithm.

Proximal humerus fracture is placed as the fourth most common fracture in the world (after hip, spine and radius fractures) occurring in osteoporotic bone. It accounts approximately 8% of all fractures transpire in the world and is associated with an important burden cost. To treat a fractured shoulder a surgeon needs to restore its shape and (or) functionality. Such operation is very complicated and cannot guaranty a successful outcome. The re-hospitalizations often occur after proximal humerus fractures and the cost to manage these re-hospitalizations is typically higher than primary hospitalization. The main reason for the unsatisfactory outcomes is the unknown native humerus shape caused by the displaced fragments.

A summary of the above-described techniques will now be provided. Processing circuitry 102 performs a CT-Scan segmentation. The core of the proposed segmentation is the multi-scale Hessian based bone enhancement filter. This filter is the most adapted solution in case of proximal humerus fracture, however it has several drawbacks such as rapidity, excessive memory usage and application-specific parameters dependency.

To overcome the two first problems, this disclosure describes techniques for automatically determining the locations of the broken humeral head, the diaphysis and the remaining fragments. The ROIs are created around the located fragments and the segmentation process is carried out only inside the defined ROI.

In order to determine the filter's application-specific parameters set, a large number of possible sets may be tested. For each test the obtained results are compared to the ground truth data. Then, the binary classification is used to determine the best parameters set. The described procedure is applied on seven patients. The best parameter set is the one that simultaneously maximizes the segmentation results for all patients.

To validate the proposed segmentation algorithm a set of 20 CT-Scan exams with proximal humerus fracture was used. The RMS distance between automatically segmented broken surfaces and the ground truth surfaces was chose to quantify the segmentation error. The obtained mean RMS error for 20 patients was less than 1mm. These results prove the high accuracy of the proposed segmentation algorithm.

Processing circuitry 102 performs fracture fragment processing. In cases of proximal humerus fractures, CT-Scan images often show some of the broken fragments as being fused together. In order to manipulate broken fragments and to restore the original patient anatomy, all fused fragments must be separated. In the literature, very few attempts have been undertaken to resolve the above problem and all of the described methods are either manual or semi-automatic. Fused fragments separation is not a trivial task. To be able to process all possible fusion types, this disclosure describes a new segmentation algorithm, referred to as step segmentation. The idea behind this algorithm is a gradual propagation of the segmentation process. During each step a specific region of interest is used as a start point for a wave propagation. Such a "surgical-strike" propagation permits to guide the global segmentation process towards the desired regions.

The fragments separation process operates on the broken bone surface. To identify the "region of interest" for the guided propagation, processing circuitry 102 performs a "Discrete Curve Evolution on Arbitrary Triangulated 3D Mesh" algorithm, as described in Sergii Poltaretskyi, Jean Chaoui and Chafiaa Hamitouche-Djabou. Discrete Curve Evolution on Arbitrary Triangulated 3D Mesh. In Elena Barcucci, Andrea Frosini and Simone Rinaldi, editeurs, Discrete Geometry for Computer Imagery, numéro 8668 de Lecture Notes in Computer Science, pages 410-421. Springer International Publishing, September 2014. These methods enable identification of the salient vertices for any contour defined on a 3D mesh. The above-described techniques operate on an arbitrary triangulated mesh. However, the same idea can be applied to 2D segmentation problems.

Processing circuitry 102 performs humerus statistical shape modeling. Statistical shape model can be considered as a priori information about a modeled shape. These models are widely-used in medical applications for the purpose of segmentation, shape prediction, morphometric analysis, etc. In the orthopedic domain, SSMs have been frequently applied on several human bones; however, there is no full-scale work about the humerus SSM and its application.

This disclosure describes an automatic algorithm to detect the correspondences between two humeri. These correspondences allow processing circuitry 102 to compute the SSM in virtue of principal component analysis. An automatic approach plays an important role for the accuracy improvement due to the exclusion of the error introduced by a human expert during manual correspondence establishment.

Using the SSM, processing circuitry 102 performs virtual fragment reduction. The previously described techniques serve as tools to achieve broken proximal humerus shape estimation/prediction.

Two major problems need to be resolved in order to succeed in the shape reconstruction. The identified issues are the weak bone intensity caused by the osteoporosis and the comminution effect which often occurs during the proximal humerus fracture. The first problem adversely affects the segmentation quality so that not all bone surfaces might be detected. The second problem makes impossible the use the fracture contours for the reduction procedure.

In view of these two problems, this disclosure describes techniques for broken shape prediction based on humerus SSM. The diaphysis surface can be used as a reliable information for the retroversion, inclination, and humerus HH estimation. In order to estimate these parameters, processing circuitry 102 performs a fitting-based procedure. This procedure adapts the humerus SSM model so that it fits into the available diaphysis fragment. The resulting shape is used as the native humerus prediction.

An example application for fracture reduction, using techniques introduced above, will now be described. Device 100 obtains image data of a joint that includes at least a portion of a humerus and a portion of a scapula. The image data may be 3D image data that includes a plurality of voxels. Device 100 segments the image data to identify portions of the image data that correspond to cortical bone. Device 100 may, for example, use any of the various segmentation techniques discussed above to identify, in the 3D image data, voxels that correspond to cortical bone. The segmentation may result in the identification, in the image data, of multiple fragments for the humerus, as well as identification of cortical bone corresponding to the scapula. Each fragment in the image data generally corresponds to a group of mostly contiguous cortical bone voxels that are partially or completely separated from other groups of mostly contiguous cortical bone voxels.

Depending on the type and severity of fracture, the segmented image data may include several fragments, such as a diaphysis fragment, a humeral head fragment, a lesser tuberosity fragment, and/or a greater tuberosity fragment. Some of these portions of the humerus may still be together, i.e., not broken, in the 3D image data. For example, in many fractures, the humeral head will stay together with the lesser tuberosity. It is also possible that some parts of the humerus may be broken into multiple fragments. For example, in some fractures, the greater tuberosity can be broken into two, three, four, or sometimes even more fragments.

Device 100 may create a 3D surface model that includes a 3D surface mesh for the scapula and each fragment identified in the segmented image data. Device 100 may, for example, perform a decimation algorithm on the segmented image data to determine 3D meshes for each identified fragment. Each 3D surface mesh may be represented by a plurality of triangles, each having three vertices, with the vertices being locations in the 3D model space. Device 100 can generate the 3D surface model at the same scale as the 3D image data, such that voxels map to vertices, and vice versa. Unlike a voxel, a vertex may not have any sort of associated color information, such as luminance or chrominance values, RGB values, monochrome values, or the like.

Device 100 can be configured to identify, in the 3D surface model, a portion of a 3D mesh that corresponds to a humeral head. In a fractured humerus, the humeral head can move a relatively large distance in a number of different directions, compared to, for example, the movements of the lesser and greater tuberosities which are typically limited due to those parts of the humerus being attached to muscle. To identify the humeral head, device 100 may, for example, use the sphere detection algorithm described above to identify inward pointing normal vectors on the surfaces of the 3D meshes. Device 100 may determine a normal vector for each vertex as being the average of the normal vectors for all triangles that share that vertex. Device 100 can then determine a point that is intersected by the most normal vectors. As the humeral head is approximately spherical, the most intersected point represents an approximate center for the approximate sphere that corresponds to the humeral head.

FIG. 29 shows an example of a 2D slice of 3D meshes for a fractured humerus. As can be seen in FIG. 29, vectors 2902A, 2902B, 2902C, and other vectors not labeled in FIG. 29 are all normal vectors from the surface of fragment 2904. Based on vectors 2902A-2902C all intersecting at or near point 2906, device 100 can determine that 3D mesh 2904 is approximately spherical, and thus corresponds to a humeral head. By contrast, vectors 2908A, 2908B, and other vectors (not labeled in FIG. 29) are all normal vectors from the surface of 3D mesh 2910. As vectors 2908A, 2908B, and the other vectors do not have a common intersection point, device 100 can determine that 3D mesh 2910 does not correspond to the humeral head. Although the example of FIG. 29 is shown in 2D for purposes of explanation, device 100 can be configured to determine normal vectors and identify point 2906 in a 3D space.

Device 100 may also detect a 3D mesh that corresponds to the diaphysis of the humerus. Device 100 may, for example, identify a vertex in the 3D model that is farthest away from the humeral head. This vertex typically corresponds to a distal end of the diaphysis, i.e., towards the elbow. In a shoulder trauma, fragments do not move to this portion of a patient's arm due to the presence of muscle, so this farthest vertex can be assumed to correspond to the diaphysis. In this context, device 100 may be configured to determine the vertex that is farthest away from the humeral head using the approximate spherical center identified above as a reference point. That is, device 100 can identify the vertex farthest away from the spherical center (e.g., point 2906 in FIG. 29) as belonging to the 3D mesh that corresponds to the diaphysis of the patient.

After identifying the 3D mesh that corresponds to the diaphysis, device 100 may be configured to remove the diaphysis inner surface from the 3D mesh that corresponds to the diaphysis. That is, for the 3D mesh that is determined to correspond to the diaphysis, device 100 may be configured to identify and remove vertices, and the triangles associated with those vertices, that correspond to the inner surface of the diaphysis.

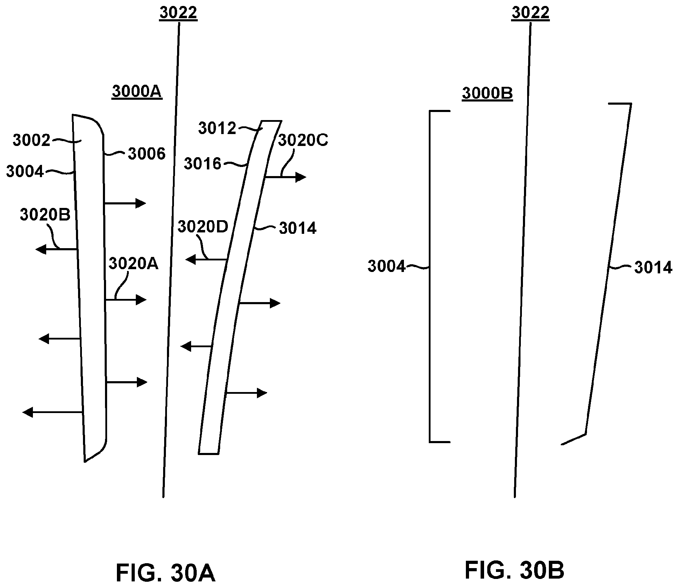

FIG. 30A shows a 2D slice of an example 3D mesh 3000A, which corresponds to a portion of a diaphysis. 3D mesh 3000A has left surface 3002 and right surface 3012. Left surface 3002 corresponds to cortical bone on the left side of the 2D slice of the diaphysis of 3D mesh 3000A and has outer surface 3004 and inner surface 3006. 3D mesh 3000A also has right surface 3002. Right surface 3002 corresponds to cortical bone on the right side of the 2D slice of the diaphysis of 3D mesh 3000A and has outer surface 3014 and inner surface 3016. Device 100 can determine central axis 3022 by orienting a bounding box around the identified diaphysis fragment, with the longer axis of the bounding box corresponding to the diaphyseal axis, i.e., central axis 3022. Device 100 can orient the bounding box by, for example, moving the bounding box around the diaphysis fragment to determine an orientation where the diaphysis fragment is completely within the box.

Device 100 can also be configured to calculate normal vectors for the vertices of 3D mesh 3000A, including all vertices of outer surfaces 3004 and 3014 and inner surfaces 3006 and 3016. Vectors 3020A-3020D are examples of such normal vectors. Other examples of normal vectors are also shown in FIG. 30A but are not labeled. Device 100 can then classify vertices with normal vectors pointing towards central axis 3022 as corresponding to an inner surface of the diaphysis bone wall and vertices with normal vectors pointing away from central axis 3022 as corresponding to an outer surface of the diaphysis bone wall. Thus, device 100 can identify, and remove, triangles corresponding to inner surfaces 3006 and 3016 by identifying vertices with normal vectors that point towards central axis 3022 and removing all the triangles that are connected, or associated, with these vertices.

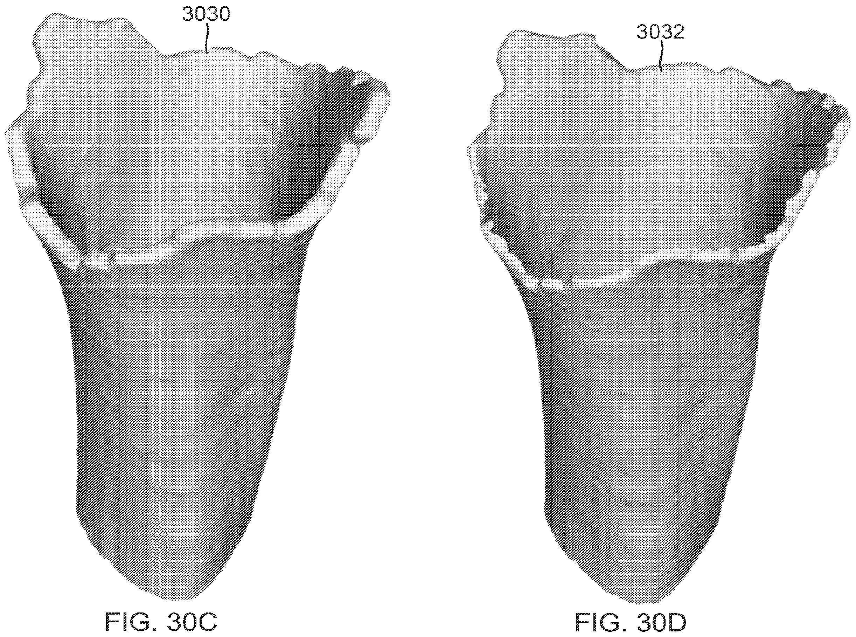

FIG. 30B shows a 2D slice of an example of 3D mesh 3000B, which corresponds to the same portion of a diaphysis as 3D mesh 3000A, but in 3D mesh 3000B, infer surfaces 3002 and 3016 have been removed. Similarly, FIG. 30C shows an example 3D mesh 3030, which corresponds to a humeral bone wall with all vertices corresponding to both the inner and outer bone wall, while FIG. 30D shows an example of 3D mesh 3032, which corresponds to the exterior surface of a humeral bone wall without any vertices not corresponding to the exterior surface of the humeral bone.

Device 100 may similarly remove the inner surface from the 3D meshes of the other fragments. For the humeral head fragment, for example, device 100 may identify inner surfaces by determining the vertices that have normal vectors that point towards a center point of the humeral head, such as point 2906 in FIG. 29, and remove from the 3D mesh triangles that include those vertices.

Based on a shape of the exterior surface of the 3D mesh corresponding to the diaphyseal bone wall, device 100 can determine a predicted humerus for a patient based on statistical shape modeling using the techniques described above. That is, device 100 generates another 3D mesh that corresponds to the predicted humerus.

In some implementations, device 100 may also be configured to remove, from the 3D model, 3D meshes that correspond to small fragments. That is, device 100 may remove from the 3D model, any 3D mesh that device 100 determines to have a surface area smaller than 200 mm$^2$, or smaller than some other such threshold. Surgeons commonly remove, rather than reattach, small fragments. Thus, for purposes of pre-operative planning and intraoperative guidance, a surgeon may desire to not see small fragments.

Because a diaphysis is frequently displaced during a proximal humerus fracture, a surgeon will likely need to move the diaphysis to repair the fracture. Thus, the location of a diaphysis in the obtained image data is likely not indicative of where the diaphysis was before the fracture. Device 100 may be configured to register the predicted humerus and the diaphysis to an estimated pre-fracture location using a known reference, such as the glenoid. That is, device 100 may determine a location for the 3D mesh of the predicted humerus based on a location of the 3D mesh for the glenoid. For instance, device 100 may be configured to identify the scapula in the 3D model based on factors such as location, size, and shape, and in the 3D mesh of the scapula, identify the glenoid cavity. Device 100 may then register the predicted humerus to the glenoid cavity. As one example, device 100 may register the predicted humerus such that a distance between the humeral head surface and glenoid surface is 3mm, and the angle between the diaphyseal axis and a scapula vertical axis is 30 degrees. Other distances and angles may also be used. Device 100 may additionally or alternatively be configured to register the predicted humerus to the scapula based on input from a user such as a surgeon.

Device 100 may be configured to mark all 3D meshes of fragments that do not correspond to the humeral head or diaphysis as corresponding to unknown fragments. Device 100 may then perform an initial projection and alignment of the 3D meshes for the unknown fragments to the humeral head portion of the 3D mesh for the predicted humerus. To project and align the fragments, device 100 may determine a sphere in the 3D model space that is centered at the center point of the humeral head of the predicted humerus. Device 100 may determine the sphere as having a slightly larger radius, e.g., 10% larger, than the radius of the humeral head of the predicted humerus.

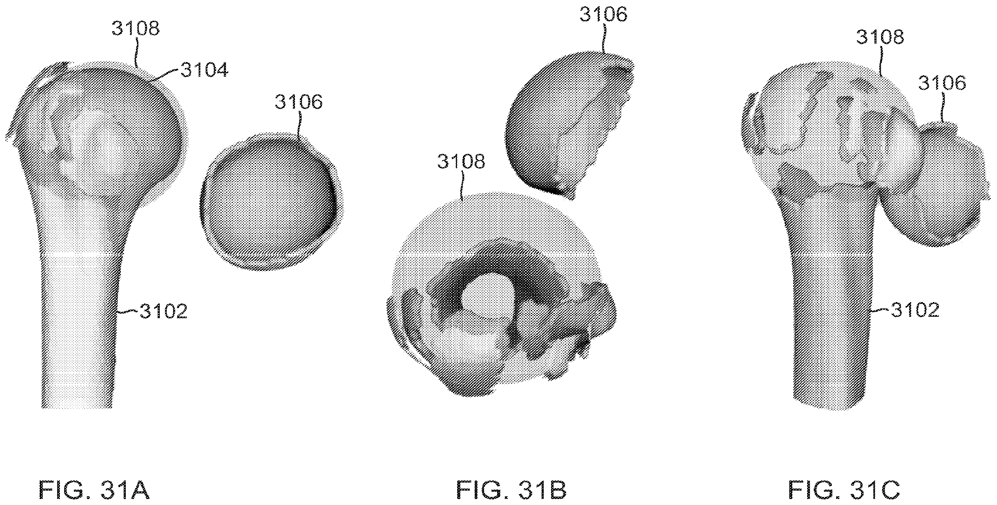

FIG. 31A shows an example of a 3D mesh for a predicted humerus 3102 with humeral head 3104. FIG. 31A also shows 3D mesh 3106, which corresponds to a humeral head fragment. FIG. 31A also shows several unknown fragments. Sphere 3108 is a sphere with a slightly larger radius than the radius of humeral head 3104. The center of sphere 3108 corresponds to the approximate center of humeral head 3104. FIGS. 31B and 31C show the same fragments and sphere as FIG. 31A but from different perspectives. By aligning the fragments to a sphere with a slightly larger radius than the predicted humeral head, device 100 may separate the fragments, e.g., to reduce touching or overlapping.

To project a 3D mesh of a fragment to a sphere (e.g., sphere 3108), device 100 may, for example, compute a center of gravity for the 3D mesh of a fragment. The center of gravity may, for example, be a mean vertex of all vertices of the fragment. Thus, device 100 can compute the mean vertex as the sum of all vertex coordinates divided by the number of vertices. Device 100 may then project the center of gravity for the 3D mesh of the fragment to the sphere. To project the center of gravity for the 3D mesh to the sphere, device 100 can determine a line that through the center of gravity for the 3D mesh of the fragment and through the center of the sphere. Device 100 can determine an intersection point where this line intersects the sphere. Device 100 can then determine a translation vector from the center of gravity of the fragment to the intersection point and apply that translation vector to all vertices of the 3D mesh for the fragment. The translation vector thus causes the fragment to be moved in the 3D model space, but without altering the size or shape of the fragment.

Device 100 can then align the 3D mesh for the fragment by determining an orientation that minimizes the distances between the vertices of the 3D mesh of the fragment and the vertices of the surface of the sphere. Device 100 can determine an estimated alignment by rotating the 3D mesh for the fragment around the center of gravity for the fragment, which corresponds to intersection point with the sphere after device 100 applies the translation vector to the 3D mesh for the fragment.

After device 100 has projected and aligned the unknown fragments with the sphere, device 100 can then move the 3D meshes for the unknown fragments until there is no intersection with the 3D meshes of other fragments. To do so, device 100 can compute a global center of gravity, e.g., a mean vertex of all vertices for all unknown fragments. Device 100 may, for example, then move the fragments away from the global gravity center in an iterative manner, such as 1mm per iteration, until there is no intersection. Device 100 may, for example, for each fragment, determine a vector that runs from the global gravity center through the center of gravity for an individual fragment and move the individual fragment along that vector. In some example, device 100 may, for each iteration, move only the fragment farthest from the global gravity center, and then compute a new global gravity center for each iteration.

Device 100 may then perform an approximate registration of the 3D mesh for the humeral head fragment to the humeral head of the predicted humerus. Device 100 may, for example, translate the 3D mesh for the broken humeral head by computing a translation vector, which is a vector from the determined center point for the 3D mesh of the detected broken humeral head to the center of predicted humeral head. That is, device 100 may be configured to compute a vector from the center of the 3D mesh for the humeral head fragment to the center of predicted humeral head, and then, using the computed vector, modify all vertices of the 3D mesh for the humeral head fragment in order to move the entire 3D mesh for the humeral head fragment.

Device 100 may then use a non-linear minimization in order to find an initial orientation for the 3D mesh of the humeral head fragment relative to the humeral head of the predicted humerus. To do so, device 100 may construct a set of three angles with associated axis (1,0,0; 0,1,0; 0,0,1). Rotating around these axes represents any possible rotation in 3D space. Device 100 may then rotate the 3D mesh of the humeral head fragment in the defined space to determine a minimum distance between vertices of the 3D mesh for the humeral head fragment and vertices of the humeral head portion of the 3D mesh for the predicted humerus. Device 100 may, for example, determine a sum of distances between vertices on the 3D mesh of the broken humeral head fragment and vertices on the 3D mesh of the predicted humeral head. For each vertex on the 3D mesh of the humeral head fragment, device 100 may determine a distance to a closest vertex on the 3D mesh of the predicted humeral head. Device 100 may rotate the 3D mesh of the humeral head fragment around the center to determine an orientation that minimizes this sum of distances. This minimization can serve as an initial estimation and need not be perfect.

Device 100 can be configured to move the 3D meshes for the undefined fragments back towards a new gravity center in order to reduce the fracture. After the movement and orientation described above, device 100 may compute a new global gravity center for the 3D meshes of the unknown fragments and iteratively move the 3D meshes for the unknown fragments towards the new global gravity center until the 3D meshes for the unknown fragments begin to touch or intersect. Device 100 may, for example, for each 3D mesh of an unidentified fragment, determine a vector that runs from the new global gravity center through the center of gravity for an individual 3D mesh of an unidentified fragment and move the 3D mesh for the individual fragment along that vector. For each iteration, device 100 may move a 3D mesh of an unidentified fragment a small amount, such as 1 mm or some other such distance. As with the orientation of the 3D mesh for the humeral head fragment to the humeral head of the predicted humerus, this minimization can serve as a satisfactory initial estimation for the locations of the 3D meshes of the unknown fragments and need not be perfect.

Device 100 may then compute allowed regions for the 3D meshes of the unknown fragments. The allowed regions represent the possible locations for the 3D meshes of the unknown fragments and can correspond to regions on the 3D mesh of the predicted humerus that do not overlap with the 3D meshes of the humeral head fragment and the diaphysis fragment. Device 100 can determine the allowed regions by subtracting the 3D meshes for the humeral head fragment and diaphysis fragment from the 3D mesh for the predicted humerus, with the remaining regions being the allowed regions.

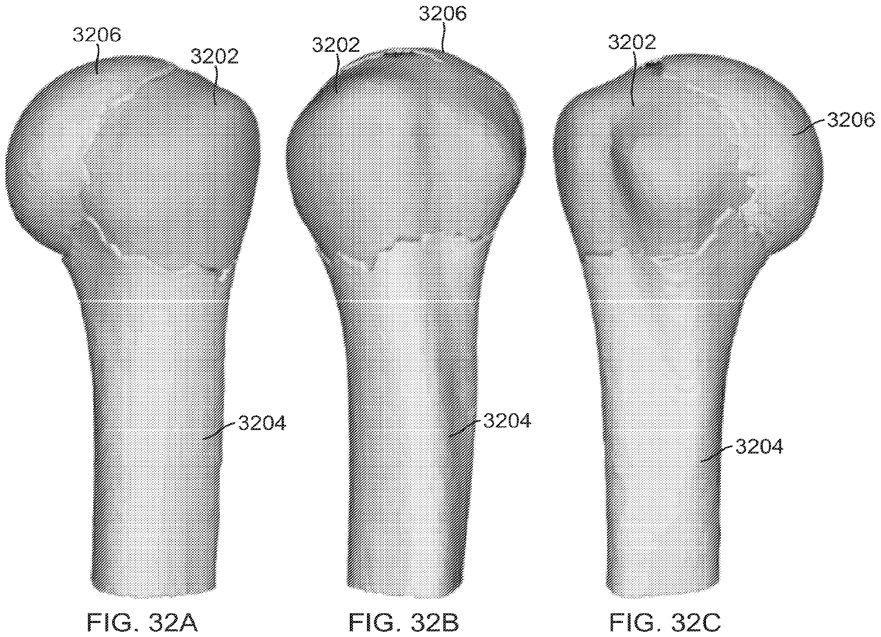

FIGS. 32A-32C show allowed region 3202 from three difference perspectives. Allowed region 3202 corresponds to a portion of the predicted humerus that does not overlap with diaphysis fragment 3204 or humeral head fragment 3206.

Device 100 can then project the 3D meshes of the undefined fragments onto the allowed regions. Device 100 may, for example, implement an ICP algorithm, or other type of mathematical optimization algorithm, to perform the projection. Device 100 may, for example, perform a non-linear position optimization on the undefined fragments to adjust the position of undefined fragments, both relative to other fragments and relative to the allowed region. To do so, for each undefined fragment, device 100 may be configured to define a set of 6 parameters (x, y, z translations and z, y, z rotations) and 3 axis (x(1,0,0), y(0,1,0), z(0,0,1)). The translations and rotations may be rigid, meaning the application of the translations and rotations to 3D meshes may change the orientation or location of the 3D meshes but do not affect a shape or size of the 3D meshes.

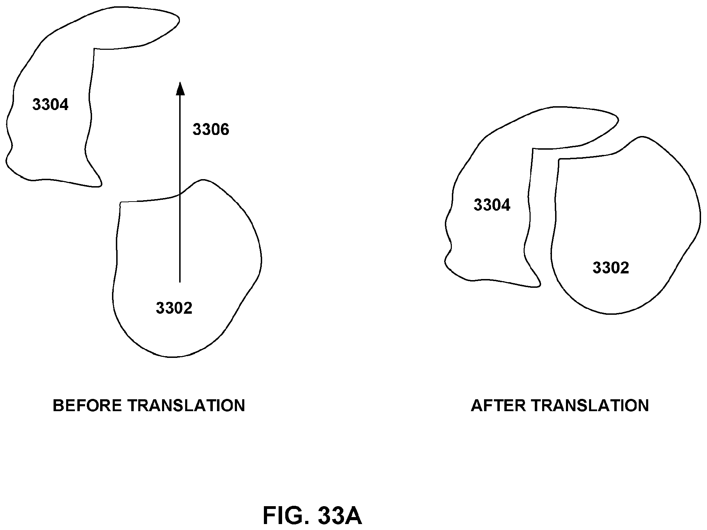

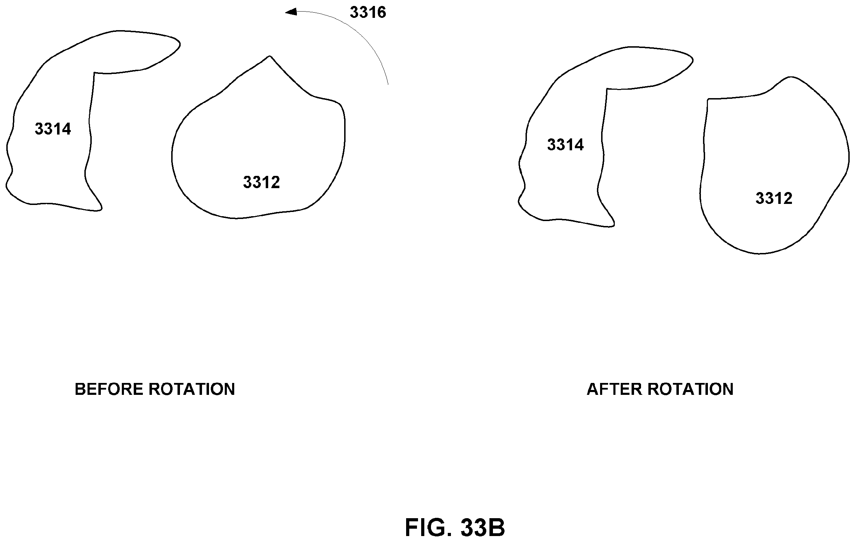

For every iteration of the minimization algorithm, device 100 can apply a transformation from a defined set of transformations to the 3D meshes of each fragment to align the contours of the 3D meshes. The defined set of transformations may include translations and rotations that transform the fragments. FIG. 33A shows an example of a translation that moves fragment 3302 relative to fragment 3304. In the example of FIG. 33A, device 100 applies a translation vector (represented by arrow 3306) to fragment 3302, which after the translation, moves fragment 3302 relative to fragment 3304. FIG. 33B shows an example of a rotation that moves fragment 3312 relative to fragment 3314. In the example of FIG. 33B, device 100 applies a rotation vector (represented by arrow 3316) to fragment 3312, which after the translation, moves fragment 3312 relative to fragment 3314.

Device 100 may then perform a translation on the aligned fragments to move the fragments towards the global gravity center of all the unknown fragments. Device 100 may be configured to calculate the minimization function as a product of two terms - (1) a distance between fragments and (2) a distance from allowed region border and fragment borders.

Device 100 may then refine the orientation of the 3D mesh for the humeral head fragment. Once approximate pre-fracture locations have been determined for the 3D meshes of unknown fragments, device 100 can refine the orientation of the 3D mesh for the humeral head fragment by identifying the humeral head contour and fitting a plane to this contour. Device 100 may, for example, identify the humeral head contour by identifying boundary vertices, e.g., those vertices of edges in the mesh for the humeral head fragment that are only connected to one triangle. Device 100 may rotate the humeral head around a normal defined by the plane. The plane may, for example, define a normal for humeral head pose rotation. Device 100 may, for example, rotate the humeral head from -180 to 180 degrees with a step of 4 degrees, or with some other such step. Device 100 can compute a distance from the humeral head contour to the undefined fragments and diaphysis for every iteration and then select the orientation with the minimal distance.

Device 100 may then perform global fragments adjustment to adjust the position of all fragments relative to each other. To do so, for each fragment (except the diaphysis), device 100 can define a set of 6 parameters (x, y, z translations and x, y, z rotations) and 3 axis (x(1,0,0), y(0,1,0), z(0,0,1)). For every iteration of the minimization algorithm, device 100 can apply a transformation from the defined set to each fragment. For each iteration, device 100 can compute a distance value between all fragments. The distance value may, for example, be a sum of shortest distances between fragment contour vertices. Using the minimization, device 100 can find a global optimal position for all fragments. In performing these calculations, device 100 may use the diaphysis for computing fragment distances without transforming the diaphysis because the diaphysis is the reference for all the fragments based on the positioning described above.

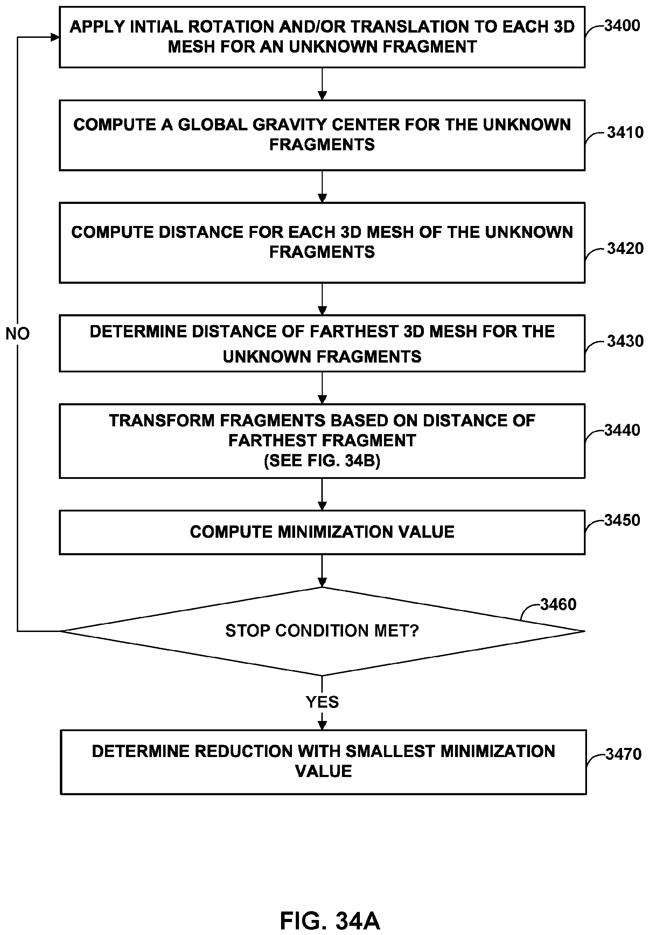

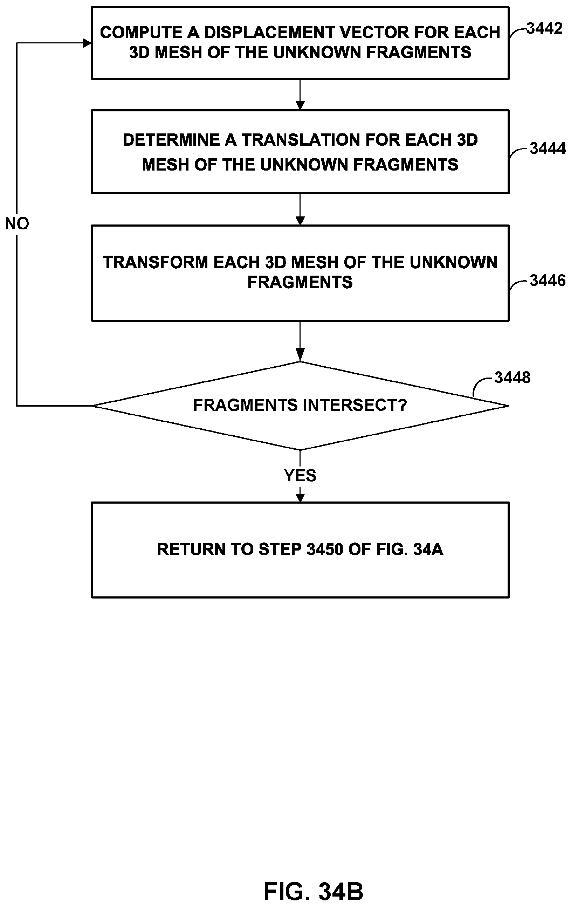

FIGS. 34A and 34B show an example of a minimization algorithm for performing the global fragment adjustment introduced above. After performing the initial positioning of the fragments described above, device 100 may perform the minimization algorithm of FIGS. 34A and 34B to determine final locations for the 3D meshes of the unknown fragments.

Device 100 applies initial rotations and/or translations to each 3D mesh of the unknown fragments to adjust the locations of the 3D meshes of the unknown fragments (3400). Device 100 may, for example, select the rotations and translations from a defined set of available rotations and translations. The defined set may limit the universe of possible available rotations and translation in order to restrict the rotation and/or distance which a 3D mesh may move. Device 100 then computes a global gravity center for the 3D meshes of the unknown fragments (3410).

Device 100 computes distances for each 3D mesh of the unknown fragments (3420). The distance for a fragment can be represented by Fragment_d_gc_i, with "i" representing a fragment ID. The distance may, for example, be a distance between a gravity center of the fragment and the global gravity center or some other such distance. Device 100 determines a distance of a farthest 3D mesh for the unknown fragments (3430). That is, device 100 determines the fragment that has the largest distance. This largest distance can be referred to as Max_d.

Device 100 then transforms the unknown fragments based on the distance of the farthest fragment. FIG. 34B shows an example process for transforming the fragments based on the value of Max_d. For each 3D mesh of the unknown fragments, device 100 computes a displacement vector (3442). The displacement vector is referred to herein as Displacement_i for fragment i and can have a set magnitude such as 1mm. Device 100, for example, may determine Displacement_1 as a vector from the center of gravity of fragment i to the global gravity center for all unknown fragments. Device 100 then determines a translation for each 3D mesh of the unknown fragments (3444). Device 100 may, for example, determine the translation as (Fragment_d_gc_i / Max_d ) * Displacment_i. Thus, for the farthest fragment, Fragment_d_gc_i equals Max_d, so the farthest fragment is moved a full increment of the magnitude of Displacement_i, and the other unknown fragments are moved a fraction of the increment. The fraction corresponds to (Fragment_d_gc_i / Max_d ). Device 100 then transforms each 3D mesh of the unknown fragments using the translation determined for that particular fragment (3446).

After transforming the unknown fragments, device 100 determines if any of the 3D meshes for the unknown fragments intersect (3448). If the 3D meshes for the unknown fragments do not intersect (3448, NO), then device 100 repeats steps 3442, 3444, and 3446 until the 3D meshes for the unknown fragments do intersect.

Once the unknown fragments intersect (3448, YES), device 100 computes a minimization value (FIG. 34A, 3450). Device 100 may, for example, determine the minimization value based on one or both of a total distance between fragments and a total distance between the unknown fragments and the outer contours of the allowed region. To compute the total distance between fragments, device 100 may, for example, for each vertex on a boundary contour of each fragment, identify the closest vertex on a boundary edge of another fragment and compute the distance between these two vertices. Device 100 may determine the total distance between fragments as the sum of the distances between each boundary contour vertex of a fragment and a nearest boundary contour vertex of a different fragment.

Device 100 may also compute a between the unknown fragments and the outer contours of the allowed region. To do so, device 100 may, for each vertex on a boundary contour of the allowed region, identify a closest vertex on any unknown fragment boundary contour, and determine a distance between the allowed region vertex and closest unknown fragment vertex. Device 100 may determine the total distance between the unknown fragments and the outer contours of the allowed region as the sum of the distances between the allowed region vertices and closest unknown fragment vertices.

Device 100 may, for example, determine the minimization value as the sum or product of the total distance between fragments and the total distance between the unknown fragments and the outer contours of the allowed region. In some examples, device 100 may weight these two factors such that one influences the minimization value more than the other. In some examples, a percentage of the allowed region covered by unknown fragments may also be used for determining the minimization value, for example, instead of the total distance between the unknown fragments and the outer contours of the allowed region. After computing the minimization value, device 100 determines if a stop condition has been met (3460). If the stop condition has not been met (3460, NO), then device 100 repeats steps 3400, 3410, 3420, 3430, 3440, and 3450 until the stop condition has been met. For each iteration of steps 3400, 3410, 3420, 3430, 3440, and 3450, device 100 applies different initial rotations and/or translations to each of the unknown fragments. Once the stop condition is met (3460, YES), device 100 determines the reduction with the smallest minimization value (3470), because this reduction can be considered to be a good reconstruction of the unknown fragments.

The stop condition above may, for example, be a number of iterations or an amount of time. In some implementations, the stop condition may alternatively or additionally be based on achieving a minimization value that is below a threshold

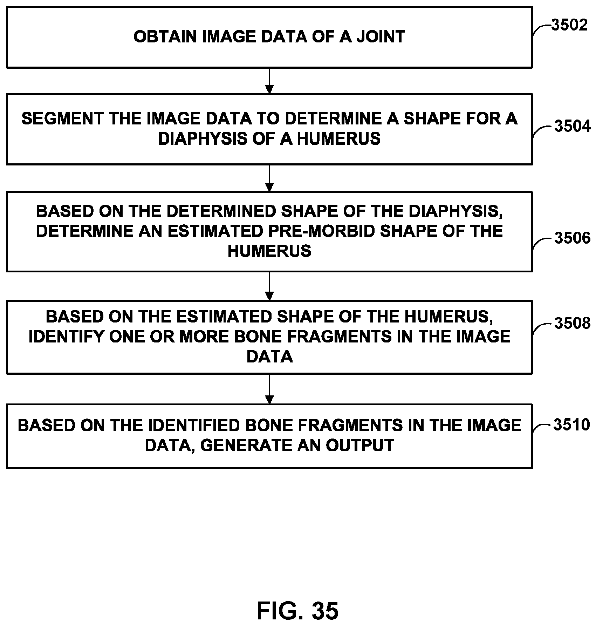

FIG. 35 is a flowchart illustrating an example method of operation of a computing device in accordance with one or more example techniques described in this disclosure. The techniques of FIG. 35 will be described with respect to device 100 of FIG. 1 but are not limited to any particular type of computing device.

Device 100 obtains image data of a jjoint (3502). The image data may, for example, be CT scans, a 3D model developed from CT scans, or any other type of image data described herein. Device 100 segments the image data to determine a shape for a diaphysis of a humerus (3504). Device 100 may, for example, perform the segmentation to determine the shape for the diaphysis in response to determining that the joint includes a fracture. Device 100 may determine that the joint includes a fracture based on an analysis of the image data or based on a user input from a user of device 100, such as a surgeon.

Based on the determined shape of the diaphysis, device 100 determines an estimated pre-morbid shape of the humerus (3506). Device 100 may, for example, determine the estimated pre-morbid shape of the humerus using the SSM techniques described above. In most cases of fracture, image data of a patient's non-damaged humerus is not available. Thus, device 100 may be configured to determine the estimated pre-morbid shape of the humerus as described above. In instances where image data of a patient's non-damaged humerus is available, however, device 100 may utilize the image data of the patient's non-damaged humerus in lieu of the estimated pre-morbid shape of the humerus.

Based on the estimated shape of the humerus, device 100 identifies one or more bone fragments in the image data (3508). Device 100 may, for example, perform the various techniques described above to identify the one or more bone fragments. For instance, device 100 may be configured to identify a region of interest based on the estimated pre-morbid shape of the humerus and perform segmentation in the region of interest to identify fragments in the region of interest. In some examples, device 100 may also refine the estimated shape of the humerus after identifying the one or more bone fragments. As part of identifying the one or more bone fragments, device 100 may also be configured to compare the image data of the joint to an image of the estimated pre-morbid shape of the humerus, and based on the comparison, identify fracture locations in the image data of the joint. Device 100 may be configured to determine a shape of the one or more bone fragments in the image data, and based on the shape of the one or more bone fragments and the identified fracture locations, determine predicted locations that correspond to the one or more bone fragments.

Based on the identified bone fragments in the image data, device 100 generates an output (3510). The output may, for example, be a classification of a fracture determined for the joint (e.g., determined for the humerus) and/or an identification of a surgical procedure for fixing the joint. The classification may, for example, be an AO classification, a Neer classification, or some other type of classification. The surgical recommendation may, for example, include an identification of a procedure for fixing the joint. The procedure may, for example, be an indication that no operation is recommended, a minimally invasive operation, such as installation of a nail or plate, is recommended, or installation of an implant is recommended.

In some examples, device 100 may be configured to determine a number of fragments present in the image data and output a surgical recommendation based on the determined number of fragments. As one example, in response to identifying four or more fragments, device 100 may be configured to generate a surgical recommendation indicating that a surgical intervention, such as a joint replacement operation, is needed for the patient. In another example, in response to detecting only one or two fragments, device 100 may be configured to generate a surgical recommendation indicating that a non-surgical treatment, such as a nail or a plate, is recommended for the patient.

In some examples, device 100 may be configured to determine a shape of the one or more bone fragments in the image data, and based on the shape of the one or more bone fragments, identify locations on the estimated pre-morbid shape of the humerus that correspond to the one or more bone fragments. By detecting a shape of a fragment, device 100 may, for example, determine to what part of the patient anatomy the fragment corresponds. For example, if the shape of the fragment is spherical, then the fragment may correspond to a humeral head. After all the fragments are segmented, device 100 can determine to what part of the patient anatomy the fragments correspond, such as detecting fragments from the greater and lesser tuberosities, the humeral head, the bicipital groove, or other parts of patient anatomy.

In some examples, device 100 may be configured to determine a displacement and/or an angle of displacement of the one or more bone fragments in the image data. Based on the displacement and/or the angle of displacement of the one or more bone fragments presents in the image data, device 100 may be configured to output a surgical recommendation or a classification of a fracture determined for the joint. Based on the displacement and/or the angle of displacement of a fragment presents in the image data, device 100 may be configured to classify the fragment. For example, if a fragment has more than 45 degrees of rotations and/or a displacement of 10mm or more, then processing circuitry 102 may classify a fragment as being displaced. Based on a number of displaced fragments and other criteria, device 100 may be configured to output the surgical recommendation or the classification of a fracture determined for the joint.

The output generated by device 100 may also be an output image, a term which is intended to include a still image, a series of images, video, an animation, or any other sort of graphical output. Device 100 may generate the output image for display on display 110, visualization device 116, or any other such display.

The output image may, for example, be an output image of the patient's joint. The output image may include visual representations of the patient's damaged humerus, at least a portion of the estimated pre-morbid shape of the humerus, and at least one bone fragment of the one or more bone fragments. Device 100 may, for example, superimpose an image of the estimated pre-morbid shape of the humerus and an image of the patient's damaged humerus to identify portions of the patient's humerus that have been damaged by the fracture. For example, for undamaged portions of the patient's humerus, the image of the estimated pre-morbid shape of the humerus and the image of the patient's actual humerus should closely match in shape and other characteristics. For damaged portions of the patient's humerus, the image of the estimated pre-morbid shape of the humerus may deviate from the image of the patient's actual humerus.

Device 100 may be configured, for example, to present areas of the estimated pre-morbid shape of the humerus that do not match the image of the patient's actual humerus using one or more identifying features. The identifying feature(s) may include outlining, shading, coloring, highlighting, etc. Similarly, device 100 may be configured, for example, to present areas of the estimated pre-morbid shape of the humerus that do match the image of the patient's actual humerus and present portions of the image of the patient's actual humerus that do not match the estimated pre-morbid shape of the humerus in manners that contrast with one another. Thus, device 100 may be configured to generate an output image where undamaged portions of a patient's humerus, damaged portions of a patient's humerus, and predicted portions of a patient's humerus are uniquely identified. In this context, the predicted portions of the patient's humerus generally show what those portions of the patient's humerus would look like if not damaged.

Device 100 may also be configured to generate an output image that includes a visual representation of at least one bone fragment of the one or more bone fragments. Device 100 may also add an annotation to the output image that identifies a portion of the visual representation of the estimated pre-morbid shape of the humerus that corresponds to a bone fragment of the one or more bone fragments. Device 100 may, for example, use outlining, shading, coloring, highlighting, or some other technique to identify where a fragment is to be moved as part of fixing the damaged humerus. Additionally or alternatively, device 100 may also add other annotations, such as arrows or textual directions identifying techniques for moving the fragment as part of fixing the damaged humerus.

Device 100 may be configured to generate output images for display via display 110, for example, as part of a surgery planning phase. The output images generated by device 100 as part of the surgery planning phase may enable a surgeon to better estimate the scope and duration of an operation, determine the tools needed to perform the operation, determine the techniques to be performed during the operation, and make other such surgical decisions. Device 100 may be configured to output to a surgeon, during a surgery planning phase prior to an actual surgery, an output image that includes some or all of the estimated pre-morbid shape of the humerus and/or image data corresponding to a patient's actual, damaged humerus. The output image may show, from the image data of the patient's damaged humerus, one or more fragments that can be identified using the techniques described above. The output image may also show a portion of the estimated pre-morbid shape of the humerus or the patient's non-damaged humerus. The estimated pre-morbid shape of the humerus or the patient's non-damaged humerus may be annotated to show locations corresponding to fragments. That is, the estimated pre-morbid shape of the humerus or the patient's non-damaged humerus may be annotated to show a surgeon where fragments are to be moved during an operation.

If a patient is to undergo a joint replacement surgery, then during the surgery planning phase, processing circuitry 102 may be configured to output an output image that includes a visual representation of a humeral head implant corresponding to a humeral head implant to be installed for the patient. The humeral head implant may, for example, be shown as being installed to the diaphysis corresponding to a patient's actual, damaged humerus. The output image may also show, from the image data of the patient's damaged humerus, one or more fragments that can be identified using the techniques described above. The output image with the humeral head implant and the estimated pre-morbid shape of the humerus or the patient's non-damaged humerus may be annotated to show locations corresponding to fragments. The annotations may be used to advise a surgeon as to where the fragments should be positioned during the implant surgery.

Device 100 may also be configured to generate output images for display via visualization device 116, for example, during an operation. In some instances, the surgery planning phase described above may occur days or weeks before an actual operation is performed. In other instances such as major traumas, the surgery planning phase may occur immediately before or concurrently with an actual surgical operation. Device 100 may be configured to generate the same output images described above for the surgery planning phase during an actual operation.

Device 100 may produce a series of images for guiding a surgeon on how to install an implant and how to move fragments to repair a damaged humerus. For example, based on the predicted humerus, device 100 may determine the humeral height, humeral head size, and medullary canal size. With this information, device 100 may be able to suggest, to the surgeon, the most appropriate implant size. Moreover, device 100 also can identify one or more characteristic points on the broken diaphysis line (e.g., a point that a surgeon can easily find during a surgical intervention). From this point, device 100 can compute the height at which the implant should be positioned in order to respect the predicted pre-morbid shape. During the surgery, a surgeon can find this point, measure the height, and correctly install the implant. In some examples, device 100 may also be configured to display, via visualization device 116 for instance, information that may guide the surgeon's installation of an implant component such as the humeral head. For example, device 100 may generate markings or annotations that show the surgeon how to position the implant with the correct height, orientation, etc.

After installation of the humeral head, device 100 may output images showing the humeral head implant. Device 100 may also annotate the images to guide the surgeon as to where various fragments should be positioned relative to the humeral head implant.

As part of generating the output images described above, device 100 may be configured to align the image data of the joint to an image of the estimated pre-morbid shape of the humerus and generate a composite image that shows a portion of the image data of the joint and a portion of the image of the estimated pre-morbid shape of the humerus. The composite image may also show the one or more bone fragments, a visual representation of a humeral head implant, and/or annotations identifying a position to move one or more of the bone fragments.

While the techniques been disclosed with respect to a limited number of examples, those skilled in the art, having the benefit of this disclosure, will appreciate numerous modifications and variations there from. For instance, it is contemplated that any reasonable combination of the described examples may be performed. It is intended that the appended claims cover such modifications and variations as fall within the scope of the invention.

It is to be recognized that depending on the example, certain acts or events of any of the techniques described herein can be performed in a different sequence, may be added, merged, or left out altogether (e.g., not all described acts or events are necessary for the practice of the techniques). Moreover, in certain examples, acts or events may be performed concurrently, e.g., through multi-threaded processing, interrupt processing, or multiple processors, rather than sequentially.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transitory media, but are instead directed to non-transitory, tangible storage media. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Operations described in this disclosure may be performed by one or more processors, which may be implemented as fixed-function processing circuits, programmable circuits, or combinations thereof, such as one or more digital signal processors (DSPs), general purpose microprocessors, application specific integrated circuits (ASICs), field programmable gate arrays (FPGAs), or other equivalent integrated or discrete logic circuitry. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute instructions specified by software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutatile. Accordingly, the terms "processor" and "processing circuitry," as used herein may refer to any of the foregoing structures or any other structure suitable for implementation of the techniques described herein.

**Claims**

1. A method comprising:

   obtaining image data of a joint that comprises at least a portion of a humerus;
   segmenting the image data to identify portions of the image data that correspond to cortical bone;
   generating a three-dimensional (3D) model based on the portions of the image data that correspond to cortical bone, wherein the 3D model comprises one or more 3D meshes corresponding to surfaces of the portions of the image data that correspond to cortical bone;
   identifying, in the one or more 3D meshes, a portion of a 3D mesh (3000A, 3000B) that corresponds to a diaphysis;
   determining an estimated pre-morbid shape of the humerus based on a shape of the portion of the 3D mesh that corresponds to the diaphysis; and
   generating an output based on the estimated pre-morbid shape of the humerus.

2. The method of claim 1, further comprising:
   identifying, in the one or more 3D meshes, a portion of a 3D mesh (2904) that corresponds to a humeral head (3104), wherein identifying the portion of the 3D mesh that corresponds to the humeral head comprises:

   determining normal vectors for vertices of the one or more 3D meshes;
   determining a most common point of intersection for the normal vectors for the vertices of the one or more 3D meshes; and
   identifying vertices with normal vectors intersecting the most common point of intersection as being vertices that belong to the portion of the 3D mesh that corresponds to the humeral head.

3. The method of claim 1, further comprising:
   identifying, in the one or more 3D meshes, a portion of a 3D mesh (2904) that corresponds to a humeral head (3104), wherein identifying the portion of the 3D mesh that corresponds to the diaphysis comprises determining in the one or more 3D meshes, a vertex that is farthest from the portion of the 3D mesh that corresponds to the humeral head.

4. The method of claim 1, further comprising:

   identifying, in the one or more 3D meshes, a portion of a 3D mesh (2904) that corresponds to a humeral head (3104);
   identifying, in the 3D model, 3D meshes corresponding to unknown fragments, wherein the 3D meshes corresponding to the unknown fragments comprises 3D meshes that are not the 3D mesh that corresponds to the diaphysis or the 3D mesh that corresponds to the humeral head;
   determining an allowed region (3202) for the 3D meshes corresponding to the unknown fragments based on the estimated pre-morbid shape of the humerus, the 3D mesh that corresponds to the diaphysis, and the 3D mesh that corresponds to the humeral head.

5. The method of claim 4, further comprising:
   determining, in the allowed region, locations for the 3D meshes corresponding to the unknown fragments.

6. The method of claim 4, further comprising:

determining a minimization value based on one or more of distances between a boundary of the allowed region and the 3D meshes corresponding to the unknown fragments, a percentage of the allowed region covered by the 3D meshes corresponding to the unknown fragments, distances between respective 3D meshes corresponding to the unknown fragments.

7. The method of claim 4, further comprising:

performing multiple transformations on the 3D meshes corresponding to the unknown fragments;
determining, for each of the multiple transformations, a minimization value based on one or more of a percentage of the allowed regions covered by the 3D meshes corresponding to the unknown fragments, distances between a boundary of the allowed region and the 3D meshes corresponding to the unknown fragments, or distances between respective 3D meshes corresponding to the unknown fragments; and
selecting a fragment reduction based on the minimization values for the multiple transformations.

8. The method of claim 1, further comprising:
determining a number of unknown fragments present in the image data, wherein the unknown fragments are represented by 3D meshes that are not the 3D mesh that corresponds to the diaphysis or a 3D mesh that corresponds to a humeral head.

9. The method of claim 1, wherein generating the output comprises:

aligning the image data of the joint to an image of the estimated pre-morbid shape of the humerus;
generating a composite image that shows a portion of the image data of the joint and a portion of the image of the estimated pre-morbid shape of the humerus, wherein the composite image further shows a visual representation of one or more unknown fragments, wherein the one or more unknown fragments correspond to 3D meshes that are not the 3D mesh that corresponds to the diaphysis or a 3D mesh that corresponds to a humeral head.

10. The method of claim 1, wherein generating the output comprises:

aligning the image data of the joint to an image of the estimated pre-morbid shape of the humerus;
generating a composite image that shows a portion of the image data of the joint and a portion of the image of the estimated pre-morbid shape of the humerus, wherein the composite image further shows an annotation identifying a position to move one or more unknown fragments, wherein the one or more unknown fragments correspond to 3D meshes that are not the 3D mesh that corresponds to the diaphysis or a 3D mesh that corresponds to a humeral head.

11. The method of claim 1, further comprising:

determining an angle of displacement of one or more unknown fragments from the 3D model, wherein the one or more unknown fragments are represented by 3D meshes that are not the 3D mesh that corresponds to the diaphysis or a 3D mesh that corresponds to a humeral head, wherein determining the angle of displacement of the one or more unknown fragments from the 3D model comprises determining an amount of rotation needed for a fragment reduction; and
determining a surgical recommendation based on the angle of displacement of the one or more unknown fragments, wherein the output comprises the surgical recommendation.

12. The method of claim 1, further comprising:

determining a displacement of one or more unknown fragments from the 3D model, wherein the one or more unknown fragments are represented by 3D meshes that are not the 3D mesh that corresponds to the diaphysis or a 3D mesh that corresponds to a humeral head, wherein determining the displacement of the one or more unknown fragments from the 3D model comprises determining an amount of translation needed for a fragment reduction; and
determining a surgical recommendation based on the displacement of the one or more unknown fragments, wherein the output comprises the surgical recommendation.

13. The method of claim 12, wherein the surgical recommendation comprises a classification of a fracture determined for the joint.

**14.** A computing system comprising:

means for storing image data of a joint that comprises at least a portion of a humerus; and

means for performing the methods of any of claims 1-13.

**15.** A computer-readable storage medium having instructions stored thereon that, when executed, configure a computing system to perform the methods of any of claims 1-13.

## Patentansprüche

**1.** Verfahren, umfassend:

Erhalten von Bilddaten eines Gelenks, das mindestens einen Abschnitt eines Oberarmknochens umfasst;
Segmentieren der Bilddaten zum Identifizieren von Abschnitten der Bilddaten, die dem kortikalen Knochen entsprechen;
Erzeugen eines dreidimensionalen Modells (3D-Modells) basierend auf den Abschnitten der Bilddaten, die dem kortikalen Knochen entsprechen, wobei das 3D-Modell ein oder mehrere 3D-Netze umfasst, die den Oberflächen der Abschnitte der Bilddaten entsprechen, die dem kortikalen Knochen entsprechen;
Identifizieren eines Abschnitts eines 3D-Netzes (3000A, 3000B) in dem einen oder den mehreren 3D-Netzen, der einer Diaphyse entspricht;
Bestimmen einer geschätzten prämorbiden Form des Humerus basierend auf einer Form des Abschnitts des 3D-Netzes, der der Diaphyse entspricht; und
Erzeugen einer Ausgabe basierend auf der geschätzten Form des Oberarmknochens vor der Erkrankung.

**2.** Verfahren nach Anspruch 1, ferner umfassend:
Identifizieren eines Abschnitts eines 3D-Netzes (2904) in dem einen oder den mehreren 3D-Netzen, der einem Oberarmkopf (3104) entspricht, wobei das Identifizieren des Abschnitts des 3D-Netzes, der dem Oberarmkopf entspricht, umfasst:

Bestimmen von Normalenvektoren für Scheitelpunkte des einen oder der mehreren 3D-Netze;
Bestimmen eines häufigsten Schnittpunkts für die Normalenvektoren der Scheitelpunkte des einen oder der mehreren 3D-Netze; und
Identifizieren von Scheitelpunkten mit Normalenvektoren, die den häufigsten Schnittpunkt schneiden, als Scheitelpunkte, die zu dem Abschnitt des 3D-Netzes gehören, der dem Oberarmkopf entspricht.

**3.** Verfahren nach Anspruch 1, ferner umfassend:
Identifizieren eines Abschnitts eines 3D-Netzes (2904) in dem einen oder den mehreren 3D-Netzen, der einem Oberarmkopf (3104) entspricht, wobei das Identifizieren des Abschnitts des 3D-Netzes, der der Diaphyse entspricht, das Bestimmen eines Scheitelpunkts in dem einen oder den mehreren 3D-Netzen umfasst, der am weitesten von dem Abschnitt des 3D-Netzes entfernt ist, der dem Oberarmkopf entspricht.

**4.** Verfahren nach Anspruch 1, ferner umfassend:

Identifizieren eines Abschnitts eines 3D-Netzes (2904) in dem einen oder den mehreren 3D-Netzen, der einem Oberarmkopf (3104) entspricht;
Identifizieren von 3D-Netzen in dem 3D-Modell, die unbekannten Fragmenten entsprechen, wobei die 3D-Netze, die den unbekannten Fragmenten entsprechen, 3D-Netze umfassen, die nicht das 3D-Netz sind, das der Diaphyse entspricht, oder das 3D-Netz, das dem Oberarmkopf entspricht;
Bestimmen eines zulässigen Bereichs (3202) für die 3D-Netze, die den unbekannten Fragmenten entsprechen, basierend auf der geschätzten Form des Oberarmkopfes vor der Erkrankung, des 3D-Netzes, das der Diaphyse entspricht, und des 3D-Netzes, das dem Oberarmkopf entspricht.

**5.** Verfahren nach Anspruch 4, ferner umfassend:
Bestimmen der Positionen der 3D-Netze, die den unbekannten Fragmenten entsprechen, in dem zulässigen Bereich.

**6.** Verfahren nach Anspruch 4, ferner umfassend:

Bestimmen eines Minimierungswerts basierend auf einem oder mehreren von Entfernungen zwischen einer Grenze des zulässigen Bereichs und den 3D-Netzen, die den unbekannten Fragmenten entsprechen, einem Prozentsatz des zulässigen Bereichs, der von den 3D-Netzen abgedeckt wird, die den unbekannten Fragmenten entsprechen, Entfernungen zwischen jeweiligen 3D-Netzen, die den unbekannten Fragmenten entsprechen.

7. Verfahren nach Anspruch 4, ferner umfassend:

Durchführen multipler Transformationen an den 3D-Netzen, die den unbekannten Fragmenten entsprechen;
Bestimmen eines Minimierungswerts für jede der multiplen Transformationen basierend auf einem oder mehreren von einem Prozentsatz der zulässigen Bereiche, die von den 3D-Netzen abgedeckt werden, die den unbekannten Fragmenten entsprechen, Abständen zwischen einer Grenze des zulässigen Bereichs und den 3D-Netzen, die den unbekannten Fragmenten entsprechen, oder Abständen zwischen jeweiligen 3D-Netzen, die den unbekannten Fragmenten entsprechen; und
Auswählen einer Fragmentreduktion basierend auf den Minimierungswerten für die multiplen Transformationen.

8. Verfahren nach Anspruch 1, ferner umfassend:
Bestimmen einer Anzahl unbekannter Fragmente, die in den Bilddaten vorhanden sind, wobei die unbekannten Fragmente durch 3D-Netze dargestellt werden, bei denen es sich nicht um das 3D-Netz handelt, das der Diaphyse entspricht, oder um ein 3D-Netz, das einem Oberarmkopf entspricht.

9. Verfahren nach Anspruch 1, wobei das Erzeugen der Ausgabe umfasst:

Ausrichten der Bilddaten des Gelenks mit einem Bild der geschätzten Form des Oberarmknochens vor der Erkrankung;
Erzeugen eines zusammengesetzten Bildes, das einen Abschnitt der Bilddaten des Gelenks und einen Abschnitt des Bildes der geschätzten Form des Oberarmknochens vor der Erkrankung zeigt, wobei das zusammengesetzte Bild ferner eine visuelle Darstellung eines oder mehrerer unbekannter Fragmente zeigt, wobei das eine oder die mehreren unbekannten Fragmente 3D-Netzen entsprechen, bei denen es sich nicht um das 3D-Netz handelt, das der Diaphyse entspricht, oder um ein 3D-Netz, das einem Oberarmkopf entspricht.

10. Verfahren nach Anspruch 1, wobei das Erzeugen der Ausgabe umfasst:

Ausrichten der Bilddaten des Gelenks mit einem Bild der geschätzten Form des Oberarmknochens vor der Erkrankung;
Erzeugen eines zusammengesetzten Bildes, das einen Abschnitt der Bilddaten des Gelenks und einen Abschnitt des Bildes der geschätzten Form des Oberarmknochens vor der Erkrankung zeigt, wobei das zusammengesetzte Bild ferner eine Anmerkung zeigt, die eine Position zum Bewegen eines oder mehrerer unbekannter Fragmente identifiziert, wobei das eine oder die mehreren unbekannten Fragmente 3D-Netzen entsprechen, bei denen es sich nicht um das 3D-Netz handelt, das der Diaphyse entspricht, oder um ein 3D-Netz, das einem Oberarmkopf entspricht.

11. Verfahren nach Anspruch 1, ferner umfassend:

Bestimmen eines Verschiebungswinkels eines oder mehrerer unbekannter Fragmente aus dem 3D-Modell, wobei das eine oder die mehreren unbekannten Fragmente durch 3D-Netze dargestellt werden, bei denen es sich nicht um das 3D-Netz handelt, das der Diaphyse entspricht, oder um ein 3D-Netz, das einem Oberarmkopf entspricht, wobei das Bestimmen des Verschiebungswinkels des einen oder der mehreren unbekannten Fragmente aus dem 3D-Modell das Bestimmen eines für eine Fragmentreduktion erforderlichen Drehungsbetrags umfasst; und
Bestimmen einer chirurgischen Empfehlung basierend auf dem Verschiebungswinkel des einen oder der mehreren unbekannten Fragmente, wobei die Ausgabe die chirurgische Empfehlung umfasst.

12. Verfahren nach Anspruch 1, ferner umfassend:

Bestimmen einer Verschiebung eines oder mehrerer unbekannter Fragmente aus dem 3D-Modell, wobei das eine oder die mehreren unbekannten Fragmente durch 3D-Netze dargestellt werden, bei denen es sich nicht um das 3D-Netz handelt, das der Diaphyse entspricht, oder um ein 3D-Netz, das einem Oberarmkopf entspricht, wobei das Bestimmen der Verschiebung des einen oder der mehreren unbekannten Fragmente aus dem 3D-Modell das Bestimmen eines für eine Fragmentreduktion erforderlichen Translationsbetrags umfasst; und Bestimmen einer chirurgischen Empfehlung basierend auf der Verschiebung des einen oder der mehreren unbekannten Fragmente, wobei die Ausgabe die chirurgische Empfehlung umfasst.

13. Verfahren nach Anspruch 12, wobei die chirurgische Empfehlung eine Klassifizierung einer für das Gelenk bestimmten Fraktur umfasst.

14. Rechensystem, umfassend:

Mittel zum Speichern von Bilddaten eines Gelenks, das mindestens einen Abschnitt eines Oberarmknochens umfasst; und
Mittel zum Durchführen der Verfahren nach einem der Ansprüche 1 bis 13.

15. Computerlesbares Speichermedium, das Anweisungen darauf gespeichert aufweist, die, wenn sie ausgeführt werden, ein Rechensystem zum Durchführen der Verfahren nach einem der Ansprüche 1 bis 13 konfigurieren.

**Revendications**

1. Procédé comprenant :

l'obtention d'une donnée d'image d'un joint qui comprend au moins une partie d'un humérus ;
la segmentation de la donnée d'image pour identifier des parties de la donnée d'image qui correspondent à un os cortical ;
la production d'un modèle tridimensionnel (3D) sur la base des parties de la donnée d'image qui correspondent à un os cortical, dans lequel le modèle 3D comprend une ou plusieurs mailles 3D correspondant à des surfaces des parties de la donnée d'image qui correspondent à un os cortical ;
l'identification, dans les une ou plusieurs mailles 3D, d'une partie d'une maille 3D (3000A, 3000B) qui correspond à une diaphyse ;
la détermination d'une forme prémorbide estimée de l'humérus sur la base d'une forme de la partie de la maille 3D qui correspond à la diaphyse ; et
la production d'une sortie sur la base de la forme prémorbide estimée de l'humérus.

2. Procédé selon la revendication 1, comprenant en outre :
l'identification, dans les une ou plusieurs mailles 3D, d'une partie d'une maille 3D (2904) qui correspond à une tête humérale (3104), dans lequel l'identification de la partie de la maille 3D qui correspond à la tête humérale comprend :

la détermination de vecteurs normaux pour des sommets des une ou plusieurs mailles 3D ;
la détermination d'un point le plus commun d'intersection pour les vecteurs normaux pour les sommets des une ou plusieurs mailles 3D ; et
l'identification de sommets avec des vecteurs normaux croisant le point le plus commun d'intersection en tant que sommets qui appartiennent à la partie de la maille 3D qui correspond à la tête humérale.

3. Procédé selon la revendication 1, comprenant en outre :
l'identification, dans les une ou plusieurs mailles 3D, d'une partie d'une maille 3D (2904) qui correspond à une tête humérale (3104), dans lequel l'identification de la partie de la maille 3D qui correspond à la diaphyse comprend la détermination dans les une ou plusieurs mailles 3D, d'un sommet qui est le plus éloigné depuis la partie de la maille 3D qui correspond à la tête humérale.

4. Procédé selon la revendication 1, comprenant en outre :

l'identification, dans les une ou plusieurs mailles 3D, d'une partie d'une maille 3D (2904) qui correspond à une tête humérale (3104) ;
l'identification, dans le modèle 3D, de mailles 3D correspondant à des fragments inconnus, dans lequel les mailles 3D correspondant aux fragments inconnus comprend des mailles 3D qui ne sont pas la maille 3D qui correspond à la diaphyse ou la maille 3D qui correspond à la tête humérale ;
la détermination d'une région autorisée (3202) pour les mailles 3D correspondant aux fragments inconnus sur la base de la forme prémorbide estimée de l'humérus, de la maille 3D qui correspond à la diaphyse et de la maille 3D qui correspond à la tête humérale.

5. Procédé selon la revendication 4, comprenant en outre :
la détermination, dans la région autorisée, d'emplacements pour les mailles 3D correspondant aux fragments inconnus.

6. Procédé selon la revendication 4, comprenant en outre :
la détermination d'une valeur de minimisation sur la base d'un ou plusieurs parmi des distances entre une frontière de la région autorisée et les mailles 3D correspondant aux fragments inconnus, un pourcentage de la région autorisée recouverte par les mailles 3D correspondant aux fragments inconnus, des distances entre des mailles 3D respectives correspondant aux fragments inconnus.

7. Procédé selon la revendication 4, comprenant en outre :

la réalisation de transformations multiples sur les mailles 3D correspondant aux fragments inconnus ;
la détermination, pour chacune des transformations multiples, d'une valeur de minimisation sur la base d'un ou plusieurs parmi un pourcentage des régions autorisées recouvertes par les mailles 3D correspondant aux fragments inconnus, des distances entre une frontière de la région autorisée et les mailles 3D correspondant aux fragments inconnus ou des distances entre des mailles 3D respectives correspondant aux fragments inconnus ; et
la sélection d'une réduction de fragment sur la base des valeurs de minimisation pour les transformations multiples.

8. Procédé selon la revendication 1, comprenant en outre :
la détermination d'un certain nombre de fragments inconnus présents dans la donnée d'image, dans lequel les fragments inconnus sont représentés par des mailles 3D qui ne sont pas la maille 3D qui correspond à la diaphyse ou une maille 3D qui correspond à une tête humérale.

9. Procédé selon la revendication 1, dans lequel la production de la sortie comprend :

l'alignement de la donnée d'image du joint avec une image de la forme prémorbide estimée de l'humérus ;
la production d'une image composite qui montre une partie de la donnée d'image du joint et une partie de l'image de la forme prémorbide estimée de l'humérus, dans lequel l'image composite montre en outre une représentation visuelle d'un ou plusieurs fragments inconnus, dans lequel les un ou plusieurs fragments inconnus correspondent à des mailles 3D qui ne sont pas la maille 3D qui correspond à la diaphyse ou une maille 3D qui correspond à une tête humérale.

10. Procédé selon la revendication 1, dans lequel la production de la sortie comprend :

l'alignement de la donnée d'image du joint avec une image de la forme prémorbide estimée de l'humérus ;
la production d'une image composite qui montre une partie de la donnée d'image du joint et une partie de l'image de la forme prémorbide estimée de l'humérus, dans lequel l'image composite montre en outre une annotation identifiant une position pour déplacer un ou plusieurs fragments inconnus, dans lequel les un ou plusieurs fragments inconnus correspondent à des mailles 3D qui ne sont pas la maille 3D qui correspond à la diaphyse ou une maille 3D qui correspond à une tête humérale.

11. Procédé selon la revendication 1, comprenant en outre :

la détermination d'un angle de déplacement d'un ou plusieurs fragments inconnus depuis le modèle 3D, dans lequel les un ou plusieurs fragments inconnus sont représentés par des mailles 3D qui ne sont pas la maille 3D qui correspond à la diaphyse ou une maille 3D qui correspond à une tête humérale, dans lequel la détermination de l'angle de déplacement des un ou plusieurs fragments inconnus depuis le modèle 3D comprend la détermination d'une quantité de rotation nécessaire pour une réduction de fragment ; et
la détermination d'une recommandation chirurgicale sur la base de l'angle de déplacement des un ou plusieurs fragments inconnus, dans lequel la sortie comprend la recommandation chirurgicale.

12. Procédé selon la revendication 1, comprenant en outre :

la détermination d'un déplacement d'un ou plusieurs fragments inconnus depuis le modèle 3D, dans lequel les un ou plusieurs fragments inconnus sont représentés par des mailles 3D qui ne sont pas la maille 3D qui correspond à la diaphyse ou une maille 3D qui correspond à une tête humérale, dans lequel la détermination du déplacement des un ou plusieurs fragments inconnus depuis le modèle 3D comprend la détermination d'une quantité de translation nécessaire pour une réduction de fragment ; et

la détermination d'une recommandation chirurgicale sur la base du déplacement des un ou plusieurs fragments inconnus, dans lequel la sortie comprend la recommandation chirurgicale.

13. Procédé selon la revendication 12, dans lequel la recommandation chirurgicale comprend une classification d'une fracture déterminée pour le joint.

14. Système informatique comprenant :

un moyen pour stocker une donnée d'image d'un joint qui comprend au moins une partie d'un humérus ; et
un moyen pour réaliser les procédés selon l'une quelconque des revendications 1-13.

15. Support de stockage lisible par ordinateur présentant des instructions stockées sur celui-ci qui, lorsqu'elles sont exécutées, configurent un système informatique pour réaliser les procédés selon l'une quelconque des revendications 1-13.

FIG. 1

FIG. 2

FIG. 3A

FIG. 3B

FIG. 3C

EP 4 065 023 B1

FIG. 4B

FIG. 4A

FIG. 4D

FIG. 4C

FIG. 5A

FIG. 5B

FIG. 5D

FIG. 5C

FIG. 6A

FIG. 6B

FIG. 6C

FIG. 6D

FIG. 6E

FIG. 6F

FIG. 7A

FIG. 7B

FIG. 7C

FIG. 8A

FIG. 8B

FIG. 8C

FIG. 9A          FIG. 9B          FIG. 9C

FIG. 10A

FIG. 10B

FIG. 10C

FIG. 10D

FIG. 11

FIG. 12A

FIG. 12B

1202

FIG. 13A

FIG. 13B

FIG. 13C

FIG. 14B

FIG. 14A

FIG. 15A  FIG. 15B  FIG. 15C  FIG. 15D

FIG. 16B

FIG. 16A

FIG. 17A

FIG. 17B

FIG. 17C

FIG. 17D

FIG. 18A       FIG. 18B       FIG. 18C       FIG. 18D

EP 4 065 023 B1

FIG. 18E  FIG. 18F  FIG. 18G  FIG. 18H

EP 4 065 023 B1

FIG. 19A

FIG. 19B

60

FIG. 20A

FIG. 20B

FIG. 20C

FIG. 21A

FIG. 21B

FIG. 21C

FIG. 21D

FIG. 21E

FIG. 21F

FIG. 22A

FIG. 22B

FIG. 23B

FIG. 23A

FIG. 24B

FIG. 24A

FIG. 25B

FIG. 25A

FIG. 26A

FIG. 26B

FIG. 26C

FIG. 26D

FIG. 27A          FIG. 27B          FIG. 27C          FIG. 27D

FIG. 27E  FIG. 27F  FIG. 27G  FIG. 27H

FIG. 28A  FIG. 28B  FIG. 28C

**FIG. 29**

**FIG. 30A**

**FIG. 30B**

FIG. 30C

FIG. 30D

FIG. 31A          FIG. 31B          FIG. 31C

FIG. 32A

FIG. 32B

FIG. 32C

BEFORE TRANSLATION

AFTER TRANSLATION

FIG. 33A

FIG. 33B

APPLY INTIAL ROTATION AND/OR TRANSLATION TO EACH 3D MESH FOR AN UNKNOWN FRAGMENT ⟋—3400

COMPUTE A GLOBAL GRAVITY CENTER FOR THE UNKNOWN FRAGMENTS ⟋—3410

COMPUTE DISTANCE FOR EACH 3D MESH OF THE UNKNOWN FRAGMENTS ⟋—3420

DETERMINE DISTANCE OF FARTHEST 3D MESH FOR THE UNKNOWN FRAGMENTS ⟋—3430

TRANSFORM FRAGMENTS BASED ON DISTANCE OF FARTHEST FRAGMENT
(SEE FIG. 34B) ⟋—3440

COMPUTE MINIMIZATION VALUE ⟋—3450

NO

STOP CONDITION MET? ⟋—3460

YES

DETERMINE REDUCTION WITH SMALLEST MINIMIZATION VALUE ⟋—3470

FIG. 34A

COMPUTE A DISPLACEMENT VECTOR FOR EACH 3D MESH OF THE UNKNOWN FRAGMENTS ⌐3442

DETERMINE A TRANSLATION FOR EACH 3D MESH OF THE UNKNOWN FRAGMENTS ⌐3444

TRANSFORM EACH 3D MESH OF THE UNKNOWN FRAGMENTS ⌐3446

FRAGMENTS INTERSECT? ⌐3448

NO

YES

RETURN TO STEP 3450 OF FIG. 34A

FIG. 34B

OBTAIN IMAGE DATA OF A JOINT — 3502

SEGMENT THE IMAGE DATA TO DETERMINE A SHAPE FOR A DIAPHYSIS OF A HUMERUS — 3504

BASED ON THE DETERMINED SHAPE OF THE DIAPHYSIS, DETERMINE AN ESTIMATED PRE-MORBID SHAPE OF THE HUMERUS — 3506

BASED ON THE ESTIMATED SHAPE OF THE HUMERUS, IDENTIFY ONE OR MORE BONE FRAGMENTS IN THE IMAGE DATA — 3508

BASED ON THE IDENTIFIED BONE FRAGMENTS IN THE IMAGE DATA, GENERATE AN OUTPUT — 3510

## FIG. 35

## REFERENCES CITED IN THE DESCRIPTION

**Patent documents cited in the description**

- US 62940810 B
- US 63049497 B
- WO 2014145267 A
- US 8971606 B

**Non-patent literature cited in the description**

- Computer Science. Springer International Publishing, September 2014, 410-421